(19) Europäisches Patentamt
European Patent Office
Office européen des brevets

(11) Publication number: **0 009 419 B1**

(12) # EUROPEAN PATENT SPECIFICATION

(45) Date of publication of patent specification: **02.01.86**

(21) Application number: **79302014.0**

(22) Date of filing: **26.09.79**

(51) Int. Cl.⁴: **A 01 N 47/36,** C 07 D 239/47, C 07 D 239/48, C 07 D 251/16, C 07 D 251/46, C 07 D 251/22, C 07 D 409/12, C 07 D 405/12

(54) **Agricultural sulfonamides, and preparation and use thereof.**

(30) Priority: **27.09.78 US 946176**
**07.08.79 US 63208**

(43) Date of publication of application:
**02.04.80 Bulletin 80/07**

(45) Publication of the grant of the patent:
**02.01.86 Bulletin 86/01**

(84) Designated Contracting States:
**AT BE CH DE FR GB IT LU NL SE**

(56) References cited:
**EP-A-0 001 514**
**DE-A-2 715 786**

(73) Proprietor: **E.I. DU PONT DE NEMOURS AND COMPANY**
**1007 Market Street**
**Wilmington Delaware 19898 (US)**

(72) Inventor: **Levitt, George**
**3218 Romilly Road Cardiff**
**Wilmington Delaware 19810 (US)**

(74) Representative: **Hildyard, Edward Martin et al**
**Frank B. Dehn & Co. Imperial House**
**15-19 Kingsway**
**London WC2B 6UZ (GB)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 009 419 B1

Courier Press, Leamington Spa, England.

**Description**

*Background of the Invention*

This invention relates to novel N-(heterocyclicaminocarbonyl)arylsulfonamides and N-(heterocyclic-aminocarbonyl)thienylsulfonamides in which the heterocyclic radical is disubstituted. The compounds of this invention and their agriculturally suitable salts, are useful and their agricultural chemicals, e.g., plant growth regulants and herbicides.

Our United States Patent Specification No. 4,127,405 discloses, *inter alia,* compounds of the following formula as herbicides:

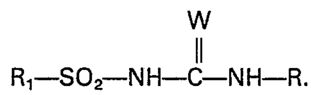

In the former R is a radical having the formula:

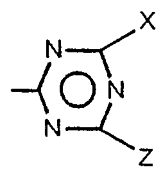

and in the latter, a radical having the formula:

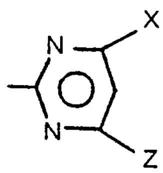

In each of those applications, $R_1$ may be

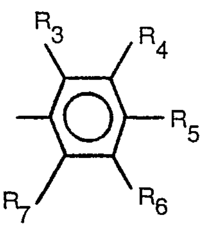

or

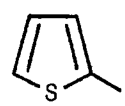

;

$R_3$ may be chlorine, bromine, fluorine, nitro, methyl, methoxy, trifluoromethyl or

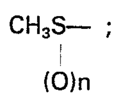

n is 0, 1 or 2;

$R_4$, $R_5$, $R_6$ and $R_7$ may each be hydrogen, fluorine, chlorine, bromine, or methyl; moreover in each application, each of $R_5$, $R_6$ and $R_7$ may be methoxy. In each application, X may be alkoxy of 1—3 carbon atoms, trifluoromethyl, $CH_3S$— or $CH_3OCH_2$—;

Z is methyl or methoxy; and

W is oxygen or sulfur.

Our European Patent Application No. 78300468.2 (publication No. 0001514) discloses, *inter alia,* compounds having the following formula as herbicides:

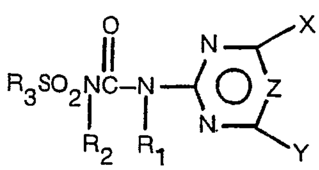

wherein

each of $R_1$ and $R_2$ may be hydrogen or methyl;

$R_3$ is

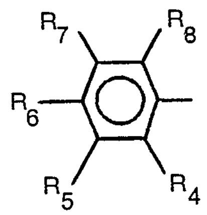 or 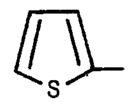 ;

X may be alkoxy of one to three carbons, $CF_3$, $CH_3S-$, $CH_3OCH_2-$ or $CH_3OCH_2CH_2O-$;
Y is $CH_3$ or $OCH_3$; and
Z is CH or N.

Our European Patent Application 79300982.0 (publication No. 0007687) disclosed, *inter alia,* compounds of the following formula as herbicides:

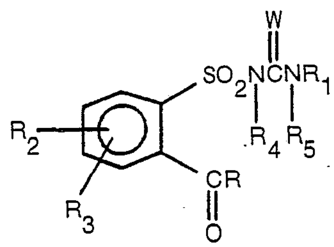

wherein

$-\underset{\underset{O}{\|}}{C}R$ is an acid, salt, ester or amide radical;

$R_1$ may be

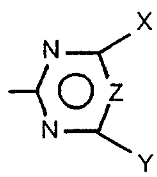 ;

$R_2$ may be hydrogen, chlorine, bromine, fluorine, nitro, methyl, methoxy or methylthio; $R_3$ is hydrogen, chlorine, bromine or methyl;
$R_4$ and $R_5$ are independently hydrogen or methyl;
W is oxygen or sulfur;
X may be methyl or methoxy;
Y may be $-OCH_2CF_3$, $-O(CH_2)_2OCH_3$, $-O(CH_2)_2OC_2H_5$, $-O(CH_2)_3OCH_3$, $-O(CH_2)_3OC_2H_5$, $-OCH_2CO_2CH_3$, $-OCH_2CO_2C_2H_5$,

$-O\underset{\underset{CH_3}{|}}{C}HCO_2CH_3$, $-O\underset{\underset{CH_3}{|}}{C}HCO_2C_2H_5$,

$-O(CH_2)_2CO_2CH_3$, $-O(CH_2)_2CO_2C_2H_5$; $-S(CH_2)_2OCH_3$, $-S(CH_2)_2OC_2H_5$, $-SCH_2CO_2CH_3$, $-SCH_2CO_2C_2H_5$,

$-S\underset{\underset{CH_3}{|}}{C}HCO_2CH_3$, $-S\underset{\underset{CH_3}{|}}{C}HCO_2C_2H_5$,

$-S(CH_2)_2CO_2CH_3$, or $-S(CH_2)_2CO_2C_2H_5$; and Z is CH or N.

The presence of undesired vegetation causes substantial damage to useful crops, especially agricultural products that satisfy man's basic food and fiber needs, such as cotton, rice, corn, wheat, and the like. The current population explosion and concomitant world food and fiber shortage demand improvements in the efficiency of producing these crops. Preventing or minimizing loss of a portion of such valuable crops by killing, or inhibiting the growth of undesired vegetation is one way of improving this efficiency. A wide variety of materials useful for killing or inhibiting (controlling) the growth of undesired vegetation is available; such materials are commonly referred to as herbicides. However, the need still exists for effective herbicides.

*Summary of the Invention*

According to this invention, there are provided novel compounds of Formula I and their agriculturally suitable salts, suitable agricultural compositions containing them and methods of using them as plant growth regulants, pre-emergence and post-emergence herbicides, both general and selective:

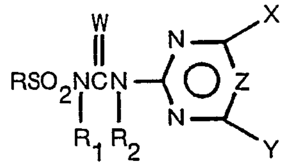

(I)

wherein

R is

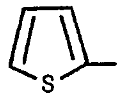

or

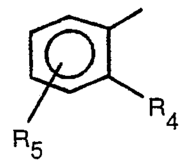

$R_1$ and $R_2$ are independently H or $CH_3$;

$R_4$ is Cl, Br, F, $NO_2$, $CH_3$, $OCH_3$, $CF_3$ or $R_3S(O)_n$, where $R_3$ is $C_1$—$C_6$ alkyl or $C_3$—$C_4$ alkenyl;

$R_5$ is H, Cl, Br, F, $CH_3$ or —$OCH_3$;

n is 0, 1 or 2;

X is $CH_3$ or —$OCH_3$;

Y is $C_2$—$C_4$ alkyl substituted with $CH_3O$, $C_1$—$C_4$ alkyl substituted with $C_2H_5O$; $CH_2F$; $CH_2Cl$; $CH_2Br$; SCN; $N_3$; $A(CH_2)_mA_1R_7$, where m is 2 or 3; $R_7$ is $C_1$—$C_3$ alkyl; A is O or S; and $A_1$ is O or $S(O)_n$;

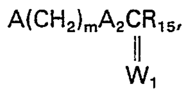

where $A_2$ and $W_1$ are independently O or S, and $R_{15}$ is $C_1$—$C_3$ alkyl, optionally substituted with 1—3 F, Cl or Br, or $R_{15}$ is $N(CH_3)OCH_3$ or $NR_8R_9$;

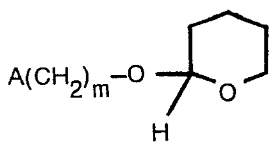

;

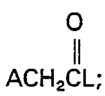

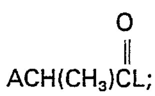

or

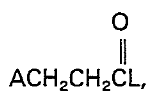

where L is $N(CH_3)OCH_3$ or $NR_8R_9$, where $R_8$ and $R_9$ are independently H, $CH_3$ or $C_2H_5$, or L is $OR_{10}$ where $R_{10}$ is H or $C_1$—$C_4$ alkyl; $NR_{11}R_{12}$ where $R_{11}$ is H or $CH_3$ and $R_{12}$ is $C_3$—$C_4$ alkenyl, $C_2$—$C_3$ alkyl substituted with $OCH_3$ or $OC_2H_5$, cyclopropyl or $C_1$—$C_2$ alkyl substituted with CN, $CO_2CH_3$ or $CO_2C_2H_5$; $OR_{13}$ where $R_{13}$ is $C_4$—$C_6$ alkyl, $C_3$—$C_4$ cyanoalkyl, $C_3$—$C_4$ alkenyl, $C_3$—$C_4$ alkynyl,

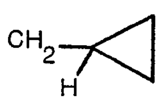

,

$C_2$ alkyl substituted with an atom of F, Cl or Br, $C_4$ alkyl substituted with 1—3 atoms of F, Cl or Br or $C_2$—$C_4$ alkyl substituted with 3 atoms of F; and

Z is CH or N;

provided that when either or both of $R_1$ and $R_2$ is $CH_3$, Y cannot be $OCH_2CH_2OCH_3$.

Preferred for reasons of biological activity, or ease of synthesis, or both, are those compounds of Formula I wherein

Y is SCN; $N_3$; $A(CH_2)_mA_1R_7$, where m is 2 or 3 $R_7$ is $C_1$—$C_3$ alkyl and $A_1$ is O or $S(O)_n$;

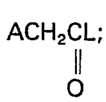

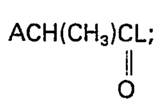

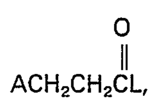

where A is O or S, and L is $N(CH_3)OCH_3$ or $NR_8R_9$, where $R_8$ and $R_9$ are independently H, $CH_3$ or $C_2H_5$, or L is $OR_{10}$ where $R_{10}$ is $C_1$—$C_4$ alkyl; $NR_{11}R_{12}$ where $R_{11}$ is H or $CH_3$ and $R_{12}$ is $C_3$—$C_4$ alkenyl, $C_2$—$C_3$ alkyl substituted with $OCH_3$ or $OC_2H_5$, or $C_1$—$C_2$ alkyl substituted with CN, $CO_2CH_3$ or $CO_2C_2H_5$; $OR_{13}$ where $R_{13}$ is $C_3$—$C_4$ cyanoalkyl, $C_3$—$C_4$ alkenyl, $C_3$—$C_4$ alkynyl,

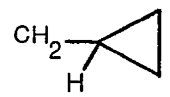 ,

$C_2$ alkyl substituted with an atom of F, Cl or Br, $C_4$ alkyl substituted with 1—3 atoms of F, Cl or Br or $C_2$—$C_4$ alkyl substituted with 3 atoms of F.

More preferred in order of increasing activity and/or increasingly favorable cost are:

(1) Compounds of the preferred scope wherein $R_1$ and $R_2$ are H and W is O;

(2) Compounds of the more preferred (1) wherein Y is $AR_{16}$ where $R_{16}$ is $CH_2CF_3$, $CH_2CH_2OR_{17}$, $CH(CH_3)CO_2R_{17}$, $(CH_2)_3OR_{17}$ or $CH_2CH_2CO_2R_{17}$, where A is O or S and $R_{17}$ is $CH_3$, —$C_2H_5$ or $i$-$C_3H_7$;

(3) Compounds of the more preferred (2) wherein A is O and $R_{16}$ is other than $CH_2CH_2CO_2R_{17}$;

(4) Compounds of more preferred (3) wherein R is

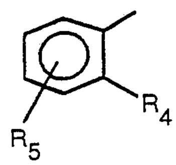 ;

(5) Compounds of more preferred (4) wherein $R_5$ is H or Cl;

(6) Compounds of more preferred (5) wherein $R_4$ is Cl or $NO_2$; and

(7) Compounds of more preferred (6) wherein $R_5$ is H.

Specifically preferred for their outstanding herbicidal activity, or their very highly favorable ease of synthesis, or both:

2-[[(6-Methyl-2-)[2-nitrophenylsulfonylamino)carbonylamino]pyrimidin-4-yloxy]acetic acid, methyl ester;

2-Chloro-[[6-methyl-4-(2,2,2-trifluoroethoxy)-1,3,5-triazin-2-yl]aminocarbonyl]benzenesulfonamide;

N-[[4-Methoxy-6-(2,2,2-trifluoroethoxy)-1,3,5-triazin-2-yl)aminocarbonyl]-2-nitrobenzenesulfonamide;

2-[[2-[(2-Chlorophenylsulfonylamino)carbonylamino]-6-methylpyrimidin-4-yloxy]]propanoic acid, methyl ester; and

2-[(6-Methyl-2-[(2-nitrophenyl)sulfonylaminocarbonylamino]-4-pyrimidinyloxy)]propanoic acid, methyl ester.

Synthesis

As shown in Equation 1, the compounds of Formula I can be prepared by reacting an appropriately substituted sulfonyl isocyanate or isothiocyanate of Formula II with an appropriate 2-aminopyrimidine or 2-amino-1,3,5-triazine of Formula III; R, W, X, Y and Z being as previously defined.

EQUATION 1

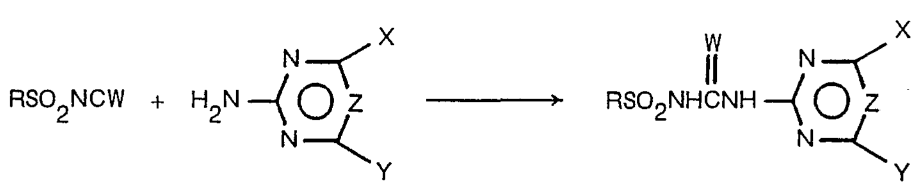

(II) (III) (I)

The reaction of Equation 1 is best carried out in inert aprotic organic solvents such as methylene chloride, tetrahydrofuran or acetonitrile, at ambient pressure and temperature. The mode of addition is not critical; however, it is often convenient to add the sulfonyl isocyanate or isothiocyanate II to a stirred suspension of heterocyclic amine III. Since such isocyanates and isothiocyanates usually are liquids, their addition can be easily controlled. The reaction is generally exothermic. In some cases, the desired product is insoluble in the warm reaction medium and crystallizes from it in pure form. Products soluble in the reaction medium are isolated by evaporation of the solvent, trituration of the solid residue with solvents such as 1-chlorobutane or ethyl ether, and filtration.

As shown in Equation 2, the compounds of Formula I, wherein $R_1$ is H and $R_2$ is $CH_3$, can be prepared by reacting an appropriately substituted sulfonyl isocyanate or isothiocyanate of Formula II with an appropriate 2-methylaminopyrimidine or 2-methylamino-1,3,5-triazine of Formula III; R, $R_2$, W, X, Y and Z being as previously defined.

EQUATION 2

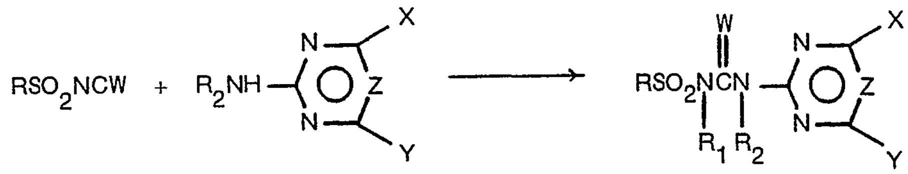

(II) (III) (I)

The reaction is carried out as is described for that of Equation 1.

Compounds of Formula I wherein $R_1$ is methyl, can be prepared by methylation of the salts of compounds of Formula I wherein $R_1$ is H, as shown in Equation 3; R, $R_2$, $R_1$, W, X, Y and Z being as previously defined and M is a metal cation and Q an anion, such as halide or methyl sulfate.

EQUATION 3

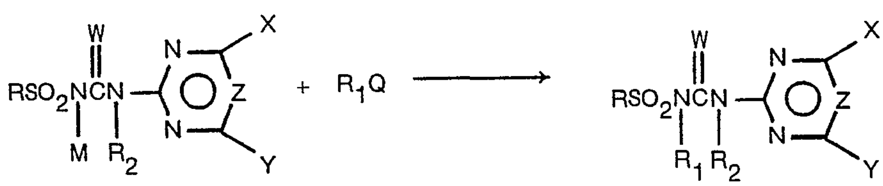

(I)

The reaction of Equation 3 is best carried out in aprotic organic solvents such as tetrahydrofuran, dimethylformamide, or dimethylacetamide, at ambient pressure and temperature. Alkylating agents such as dimethyl sulfate, or methyl iodide can be employed. The desired product can be isolated by pouring the reaction mixture into water and filtering off the precipitated solid.

Alternatively, compounds of Formula I wherein $R_1$ is methyl, can be prepared by the reaction of an appropriately substituted sulfonyl-N-methylcarbamyl chloride or sulfonyl-N-methylcarbamylthioic chloride of Formula IV with an appropriate 2-aminopyrimidine or 2-amino-1,3,5-triazine of Formula III; as shown in Equation 4; $R_2$, $R_1$, R, X, Y, W and Z being as previously defined.

EQUATION 4

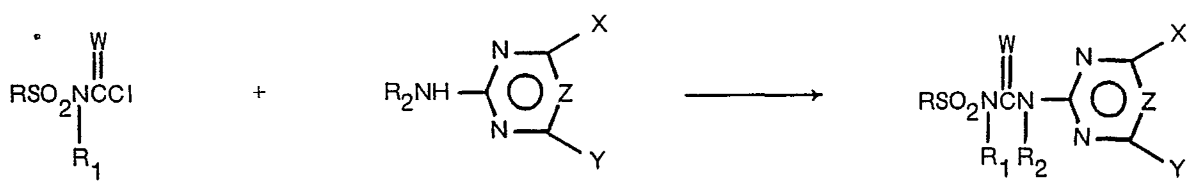

(IV) (III) (I)

The preparation of ureas from amines and carbamyl chlorides or carbamylthioic chlorides is well known to the art. The reaction can be carried out by adding equivalent amounts of the chloride IV and amine III to an inert organic solvent, such as tetrahydrofuran, xylene, or methylene chloride, in the presence of acid acceptor, such as triethylamine, pyridine, or sodium carbonate employing temperatures from 20°—130°. Soluble products can be isolated by filtering off precipitated salts and concentration of the filtrate. Insoluble products can be filtered off and washes free of salts with water.

The intermediate chlorides IV can be prepared by phosgenation or thiophosgenation of N-methylsulfonamide salts. The sulfonamide salt is added to an excess of phosgene or thiophosgene in an inert organic solvent, such as tetrahydrofuran, toluene, or xylene, whereupon the chloride IV can be isolated or reacted *in situ* with the amine III after removal of the excess phosgene or thiophosgene.

The synthesis of heterocyclic amine derivatives has been reviewed in "The Chemistry of Heterocyclic Compounds," a series published by Interscience Publ. New York and London. Aminopyrimidines are described by D. J. Brown in "The Pyrimidines," Vol. XVI of the above series.

2-Amino-1,3,5-triazines can be synthesized according to methods described by E. M. Smolin and L. Rapoport in "*s*-Triazines and Derivatives," Vol. XIII of the same series. 2-Amino-1,3,5-triazines are also conveniently prepared by the methods of K. R. Huffman and F. C. Schaefer in J. Org. Chem. *28,* 1812—1815 and 1816—1821 (1963).

The preparation of agriculturally suitable salts of the compounds of Formula I, as well as starting materials and intermediates for said compounds may be by the method disclosed in our U.S. Patent Specification No. 4,127,405 to which the reader is referred for more information.

The compounds of this invention and their preparation are further illustrated by the following examples wherein temperatures are given in degrees centigrade.

## Example 1

N-[[4-(2-methoxyethoxy)-6-methylpyrimidin-2-yl]aminocarbonyl]-2-chlorobenzenesulfonamide

To a dry stirred solution of 18.3 parts of 2-amino-4-(2-methoxyethoxy)-6-methylpyrimidine in 300 parts of methylene chloride at ambient temperature and pressure was added 22 parts of 2-chlorobenzenesulfonylisocyanate. The mixture was stirred for 2 hours and then the methylene chloride was removed under reduced pressure. The resulting solid was triturated with 1-chlorobutane and filtered to yield 35 parts of N-[[4-(2-methoxyethoxy)-6-methylpyrimidine-2-yl]aminocarbonyl]-2-chlorobenzenesulfonamide, m.p. 130°—140°C. The product showed characteristic absorption bands in the infrared spectrum.

By using the procedure of Example 1 with an equivalent amount of appropriate 2-aminopyrimidines or 2-amino-1,3,5-triazines and appropriately substituted benzenesulfonyl isocyanates or isothiocyanates, the compounds of Table 1 can be prepared.

TABLE 1-A

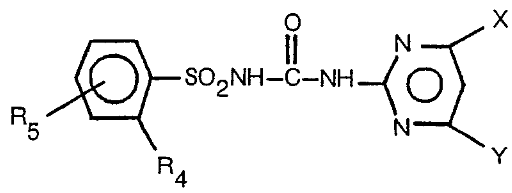

| $R_4$ | $R_5$ | X | Y | m.p. °C |
|---|---|---|---|---|
| Cl | H | $CH_3$ | $OCH_2CH_2OC_2H_5$ | |
| Cl | H | $CH_3$ | $OCH_2CF_3$ | 160—163 |
| Cl | H | $CH_3$ | $OCH_2CO_2CH_3$ | 157—160 |
| Cl | H | $CH_3$ | $OCH_2CO_2C_2H_5$ | |
| Cl | H | $CH_3$ | $OCHCO_2CH_3$<br>\|<br>$CH_3$ | 178—191 |
| Cl | H | $CH_3$ | $O—CHCO_2C_2H_5$<br>\|<br>$CH_3$ | 114—122 |
| Cl | H | $CH_3$ | $O—CH_2CH_2CO_2CH_3$ | |
| Cl | H | $CH_3$ | $OCH_2CH_2CO_2C_2H_5$ | |
| Cl | H | $CH_3$ | $OCH_2CH_2CH_2OCH_3$ | 103—115 |
| Br | H | $CH_3$ | $OCH_2CH_2OCH_3$ | |
| Br | H | $CH_3$ | $OCH_2CF_3$ | |
| Br | H | $CH_3$ | $OCH_2CO_2CH_3$ | |
| F | H | $CH_3$ | $OCH_2CH_2OC_2H_5$ | |

TABLE 1-A

| $R_4$ | $R_5$ | X | Y | m.p. °C |
|---|---|---|---|---|
| F | H | $CH_3$ | $OCH_2CF_3$ | |
| F | H | $CH_3$ | $OCH_2CO_2CH_3$ | |
| $NO_2$ | H | $CH_3$ | $OCH_2CH_2CH_2OCH_3$ | |
| $NO_2$ | H | $CH_3$ | $OCH_2CH_2OCH_3$ | 95—98 |
| $NO_2$ | H | $CH_3$ | $OCH_2CH_2OC_2H_5$ | |
| $NO_2$ | H | $CH_3$ | $OCH_2CF_3$ | 192—194 |
| $NO_2$ | H | $CH_3$ | $OCH_2CO_2CH_3$ | 155—165 |
| $NO_2$ | H | $CH_3$ | $OCH_2CO_2C_2H_5$ | |
| $NO_2$ | H | $CH_3$ | $OCH_2CO_2CH_3$<br>\|<br>$CH_3$ | 175—179 |
| $NO_2$ | H | $CH_3$ | $O—CHCO_2C_2H_5$<br>\|<br>$CH_3$ | |
| $NO_2$ | H | $CH_3$ | $O—CH_2CH_2CO_2CH_5$ | |
| $NO_2$ | H | $CH_3$ | $OCH_2CH_2CO_2C_2H_5$ | |
| $NO_2$ | H | $CH_3$ | $OCH_2CH_2CH_2OC_2H_5$ | |
| $CH_3$ | H | $CH_3$ | $OCH_2CH_2OCH_3$ | |
| $CH_3$ | H | $CH_3$ | $OCH_2CF_3$ | |
| $CH_3$ | H | $CH_3$ | $OCH_2CO_2C_2H_5$ | |
| $CH_3$ | H | $CH_3$ | $OCHCO_2CH_3$<br>\|<br>$CH_3$ | |
| $CH_3$ | H | $CH_3$ | $OCH_2CH_2CO_2C_2H_5$ | |
| $OCH_3$ | H | $CH_3$ | $OCH_2CH_2OC_2H_5$ | |
| $OCH_3$ | H | $CH_3$ | $OCH_2CF_3$ | |
| $OCH_3$ | H | $CH_3$ | $OCH_2CO_2CH_3$ | |
| $OCH_3$ | H | $CH_3$ | $OCH_2CH_2CO_2CH_3$ | |
| $CF_3$ | H | $CH_3$ | $OCH_2CH_2OCH_3$ | |
| $CF_3$ | H | $CH_3$ | $OCH_2CF_3$ | |
| $CF_3$ | H | $CH_3$ | $OCHCO_2C_2H_5$<br>\|<br>$CH_3$ | |
| $CF_3$ | H | $CH_3$ | $OCH_2CH_2CO_2CH_3$ | |
| $SCH_3$ | H | $CH_3$ | $OCH_2CH_2OC_2H_5$ | |

TABLE 1-A

| $R_4$ | $R_5$ | X | Y | m.p. °C |
|---|---|---|---|---|
| $SCH_3$ | H | $CH_3$ | $OCH_2CO_2C_2H_5$ | |
| O<br>↑<br>$SCH_3$ | H | $CH_3$ | $OCH_2CF_3$ | |
| O<br>↑<br>$SCH_3$ | H | $CH_3$ | $OCHCO_2CH_3$<br>\|<br>$CH_3$ | |
| O<br>↑<br>$SCH_3$ | H | $CH_3$ | $OCH_2CH_2CO_2C_2H_5$ | |
| O<br>↑<br>$SCH_3$ | H | $CH_3$ | $OCH_2CH_2OCH_3$ | |
| O<br>↑<br>$SCH_3$<br>↓<br>O | H | $CH_3$ | $OCH_2CF_3$ | |
| O<br>↑<br>S—$CH_3$<br>↓<br>O | H | $CH_3$ | $OCH_2CO_2C_2H_5$ | |
| Cl | 5-Cl | $CH_3$ | $OCH_2CH_2OCH_3$ | 151—155 |
| Cl | 5-Cl | $CH_3$ | $OCH_2CF_3$ | 162.5—165 |
| Cl | 5-Cl | $CH_3$ | $OCH_2CO_2CH_3$ | 182—186 |
| Cl | 5-Cl | $CH_3$ | $OCHCO_2CH_3$<br>\|<br>$CH_3$ | |
| Cl | 5-Cl | $CH_3$ | $OCH_2CH_2CH_2OCH_3$ | |
| Cl | 3-Cl | $CH_3$ | $OCH_2CH_2OCH_3$ | |
| Cl | 5-Br | $CH_3$ | $OCH_2CH_2OC_2H_5$ | |
| Cl | 5-F | $CH_3$ | $OCH_2CF_3$ | |
| Cl | 5-$CH_3$ | $CH_3$ | $OCH_2CO_2CH_3$ | |
| Cl | 5-$OCH_3$ | $CH_3$ | $OCH_2CF_3$ | |
| Cl | 6-Cl | $CH_3$ | $OCH_2CO_2C_2H_5$ | |
| Br | 5-Br | $CH_3$ | $OCHCO_2CH_3$<br>\|<br>$CH_3$ | |
| Cl | 5-Cl | $OCH_3$ | $OCH_2CO_2C_2H_5$ | |

TABLE 1-A

| $R_4$ | $R_5$ | X | Y | m.p. °C |
|---|---|---|---|---|
| Cl | 5-Cl | $OCH_3$ | $OCH_2CH_2OC_2H_5$ | |
| F | 5-F | $CH_3$ | $OCH_2CH_2CO_2C_2H_5$ | |
| $NO_2$ | 5-Cl | $CH_3$ | $OCH_2CH_2OCH_3$ | |
| $NO_2$ | 6-Cl | $CH_3$ | $OCH_2CO_2C_2H_5$ | |
| $NO_2$ | 3-$CH_3$ | $CH_3$ | $OCH_2CH_2OCH_3$ | |
| $NO_2$ | 5-$OCH_3$ | $CH_3$ | $OCH_2CF_3$ | |
| $CH_3$ | 5-$CH_3$ | $CH_3$ | $OCH_2CH_2OCH_3$ | |
| $CH_3$ | 5-Cl | $CH_3$ | $OCHCO_2CH_3$<br>\|<br>$CH_3$ | |
| $OCH_3$ | 5-$OCH_3$ | $CH_3$ | $OCH_2CH_2OC_2H_5$ | |
| $CF_3$ | 5-Cl | $CH_3$ | $OCH_2CF_3$ | |
| $CF_3$ | 5-Cl | $CH_3$ | $OCH_2CO_2CH_3$ | |
| $SCH_3$ | 5-Cl | $CH_3$ | $OCH_2CH_2OCH_3$ | |
| O<br>↑<br>$SCH_3$ | 5-Cl | $CH_3$ | $OCH_2CH_2CO_2CH_3$ | |
| O<br>↑<br>$SCH_3$<br>↓<br>O | 5-$OCH_3$ | $CH_3$ | $OCH_2CF_3$ | |
| Cl | H | $CH_3$ | $SCH_2CH_2OC_2H_5$ | 127—130 |
| Cl | H | $CH_3$ | $SCH_2CO_2CH_3$ | |
| Cl | 5-Cl | $CH_3$ | $SCH_2CH_2OC_2H_5$ | 131—150 |
| Cl | 5-Cl | $CH_3$ | $SCHCO_2CH_3$<br>\|<br>$CH_3$ | |
| Br | H | $CH_3$ | $SCH_2CH_2OCH_3$ | |
| F | H | $CH_3$ | $SCH_2CH_2CO_2C_2H_5$ | |
| $NO_2$ | H | $CH_3$ | $SCH_2CH_2OC_2H_5$ | |
| $NO_2$ | H | $CH_3$ | $SCH_2CO_2C_2H_5$ | |
| $CH_3$ | H | $CH_3$ | $SCH_2CH_2OCH_3$ | |
| $OCH_3$ | H | $CH_3$ | $SCH_2CH_2OC_2H_5$ | |
| $CF_3$ | H | $CH_3$ | $SCHCO_2C_2H_5$<br>\|<br>$CH_3$ | |

TABLE 1-A

| $R_4$ | $R_5$ | X | Y | m.p. °C |
|---|---|---|---|---|
| $CF_3$ | 5-Cl | $CH_3$ | S—$CH_2CH_2OCH_3$ | |
| $SCH_3$<br>↓<br>O | H | $CH_3$ | S—$CH_2CH_2OC_2H_5$ | |
| Cl | H | $OCH_3$ | $OCH_2CH_2OCH_3$ | |
| Cl | 5-Cl | $OCH_3$ | $OCH_2CF_3$ | |
| $NO_2$ | H | $OCH_3$ | $OCH_2CO_2CH_3$ | |
| $CF_3$ | H | $OCH_3$ | $OCH_2CH_2OC_2H_5$ | |
| $CH_3$ | H | $OCH_3$ | S—$CH_2CH_2OC_2H_5$ | |
| $OCH_3$ | H | $OCH_3$ | $OCH_2CF_3$ | |
| O<br>↑<br>$SCH_3$<br>↓<br>O | H | $OCH_3$ | $OCH_2CH_2CO_2CH_3$ | |
| Cl | H | $CH_3$ | $OCH_2CF_3$ | 160—163° |
| Cl | Cl | $CH_3$ | $OCH_2CF_3$ | 162.5—165° |
| Br | H | $CH_3$ | $OCH_2CF_3$ | |
| F | H | $CH_3$ | $OCH_2CF_3$ | |
| $NO_2$ | H | $CH_3$ | $OCH_2CF_3$ | 192—194° |
| $CH_3$ | H | $CH_3$ | $OCH_2CF_3$ | |
| $OCH_3$ | H | $CH_3$ | $OCH_2CF_3$ | |
| $CF_3$ | H | $CH_3$ | $OCH_2CF_3$ | |
| $CH_3S$ | H | $CH_3$ | $OCH_2CF_3$ | |
| $CH_3SO$ | H | $CH_3$ | $OCH_2CF_3$ | |
| $CH_3SO_2$ | H | $CH_3$ | $OCH_2CF_3$ | |
| $CH_3CH_2SO_2$ | H | $CH_3$ | $OCH_2CF_3$ | |
| $CH_3CH_2CH_2SO_2$ | H | $CH_3$ | $OCH_2CF_3$ | |
| $CH_3CH_2CH_2CH_2SO_2$ | H | $CH_3$ | $OCH_2CF_3$ | |
| $CH_3CH_2CH$—$CH_2S$<br>\|<br>$CH_3$ | H | $CH_3$ | $OCH_2CF_3$ | |
| $CH_3(CH_2)_4CH_2SO_2$ | H | $CH_3$ | $OCH_2CF_3$ | |
| $CH_2$=CH—$CH_2SO_2$ | H | $CH_3$ | $OCH_2CF_3$ | |
| $CH_3CH$=$CHCH_2SO_2$ | H | $CH_3$ | $OCH_2CF_3$ | |

TABLE 1-A

| $R_4$ | $R_5$ | X | Y | m.p. °C |
|---|---|---|---|---|
| Cl | Cl | $CH_3$ | $OCH_2CF_3$ | |
| $NO_2$ | Br | $CH_3$ | $OCH_2CF_3$ | |
| F | F | $CH_3$ | $OCH_2CF_3$ | |
| $CH_3$ | $CH_3$ | $CH_3$ | $OCH_2CF_3$ | |
| $NO_2$ | $OCH_3$ | $CH_3$ | $OCH_2CF_3$ | |
| Cl | H | $OCH_3$ | $OCH_2CF_3$ | |
| $NO_2$ | H | $OCH_3$ | $OCH_2CF_3$ | |
| Cl | H | $CH_3$ | $CH_2—CH_2—OCH_3$ | |
| $NO_2$ | H | $CH_3O$ | $CH_2CH_2CH_2OCH_3$ | |
| Cl | H | $CH_3$ | $CH_2CH_2CH_2CH_2OCH_3$ | |
| Cl | H | $CH_3$ | $CH_2OCH_2CH_3$ | |
| $NO_2$ | H | $CH_3$ | $CH_2F$ | |
| Cl | H | $CH_3$ | $CH_2Cl$ | |
| $NO_2$ | H | $CH_3$ | $CH_2Br$ | |
| $R_4$ | $R_5$ | X | Y | m.p. (°C) |
| $NO_2$ | H | $CH_3$ | SCN | |
| Cl | H | $CH_3$ | $N_3$ | 195° |
| $NO_2$ | H | $CH_3$ | $OCH_2CH_2OCH_3$ | 95—98° |
| Cl | H | $CH_3$ | $O—CH_2CH_2—OCH_3$ | 103—115° |
| Cl | H | $CH_3$ | $O—CH_2CH_2—OCH_2CH_3$ | |
| $NO_2$ | H | $CH_3$ | $O—CH_2CH_2CH_2OCH_3$ | 119—125° |
| $NO_2$ | H | $CH_3$ | $O—CH_2CH_2—OCH_2CH_2CH_3$ | |
| Cl | H | $CH_3$ | $S—CH_2CH_2OCH_2CH_3$ | 127—130° |
| Cl | H | $CH_3$ | $S—CH_2CH_2—SCH_3$ | |
| $NO_2$ | H | $CH_3$ | $S—CH_2CH_2OCH_2CH_3$ | 131—150° |
| $NO_2$ | H | $CH_3$ | $O—CH_2CH_2—SOCH_3$ | |
| Cl | H | $CH_3$ | $OCH_2CH_2—SO_2CH_3$ | |
| Cl | Cl | $CH_3$ | $OCH_2CH_2OCH_3$ | 151—155° |
| Cl | Cl | $CH_3$ | $O—CH_2CH_2CH_2—OCH_3$ | 103—115° |

TABLE 1-A

| $R_4$ | $R_5$ | X | Y | m.p. (°C) |
|---|---|---|---|---|
| Cl | Cl | $CH_3$ | 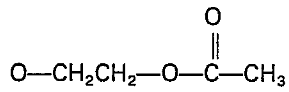 | |
| $NO_2$ | Cl | $CH_3$ | 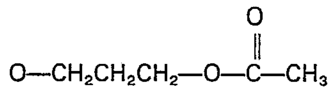 | |
| Cl | Cl | $CH_3$ | 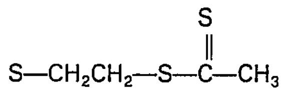 | |
| $NO_2$ | Cl | $CH_3$ | 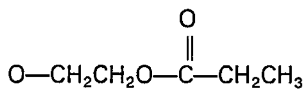 | |
| Cl | Cl | $CH_3$ | 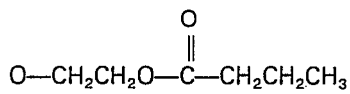 | |
| $NO_2$ | H | $CH_3$ | 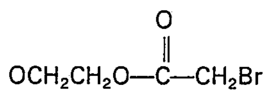 | |
| Cl | H | $CH_3$ | 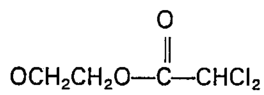 | |
| $NO_2$ | H | $CH_3$ | 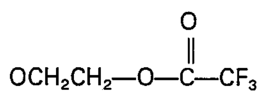 | |
| Cl | H | $CH_3$ | 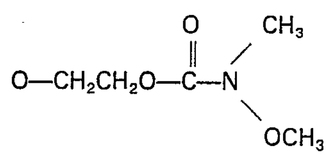 | |
| $NO_2$ | H | $CH_3$ | 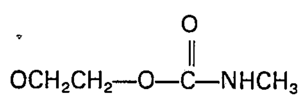 | |
| Cl | H | $CH_3$ | 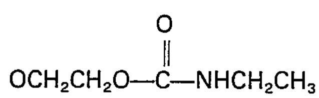 | |
| $NO_2$ | H | $CH_3$ | 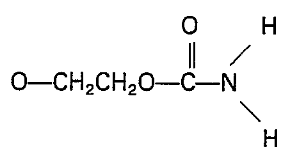 | |
| Cl | H | $CH_3$ | 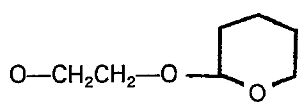 | |
| $NO_2$ | H | $CH_3$ | $OCH_2CO_2CH_3$ | 155—165° |
| Cl | H | $CH_3$ | $OCH_2CO_2CH_3$ | 157—160° |
| Cl | Cl | $CH_3$ | $OCH_2CO_2CH_3$ | 182—188° |

TABLE 1-A

| $R_4$ | $R_5$ | X | Y | m.p. (°C) |
|---|---|---|---|---|
| $NO_2$ | Cl | $CH_3$ | 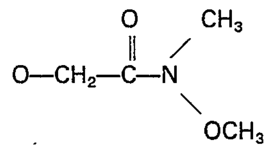 | |
| Cl | Cl | $CH_3$ | 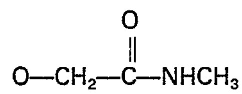 | |
| $NO_2$ | Cl | $CH_3$ | 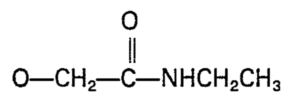 | |
| Cl | H | $CH_3$ | 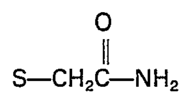 | |
| $NO_2$ | H | $CH_3$ | 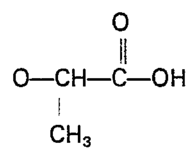 | |
| Cl | H | $CH_3$ | 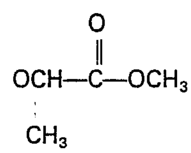 | 178—191° |
| $NO_2$ | H | $CH_3$ | 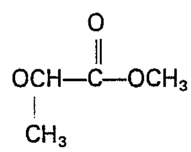 | 208—210° |
| $NO_2$ | H | $CH_3$ | 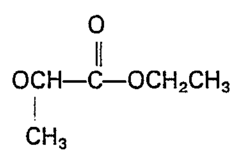 | |
| Cl | H | $CH_3$ | 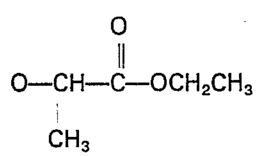 | 114—123° |
| Cl | H | $CH_3$ | 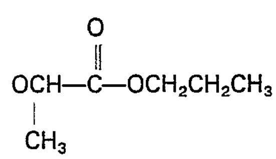 | |
| $NO_2$ | H | $CH_3$ | 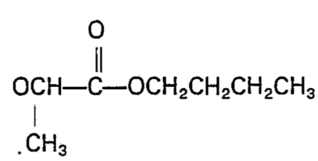 | |

TABLE 1-A

| $R_4$ | $R_5$ | X | Y | m.p. (°C) |
|---|---|---|---|---|
| Cl | H | $CH_3$ | O<br>‖<br>$—S—CH_2CH_2—C—OCH_3$ | |
| $NO_2$ | H | $CH_3$ | $NHCH_3$ | |
| Cl | H | $CH_3$ | 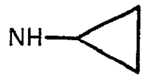 | |
| Cl | H | $CH_3$ | $CH_3$<br>/<br>N<br>\ <br>$CH_2CH_2CH$ | |
| $NO_2$ | H | $CH_3$ | $CH_3$<br>/<br>N<br>\ <br>$CH_2CH_2—CO_2CH_3$ | |
| Cl | H | $CH_3$ | $CH_3$<br>/<br>N<br>\ <br>$CH_2CH_2CO_2CH_2CH_3$ | |
| $NO_2$ | H | $CH_3$ | $CH_3$<br>/<br>N<br>\ <br>$CH_2—CH{=}CH_2$ | |
| Cl | H | $CH_3$ | $CH_3$<br>/<br>N<br>\ <br>$CH_2—CH{=}CH—CH_3$ | |
| $NO_2$ | H | $CH_3$ | $CH_3$<br>/<br>N<br>\ <br>$CH_2CH_2OCH_3$ | |
| Cl | H | $CH_3$ | $CH_3$<br>/<br>N<br>\ <br>$CH_2CH_2OCH_2CH_3$ | |
| $NO_2$ | H | $CH_3$ | $O(CH_2)_3CH_3$ | |
| Cl | H | $CH_3$ | $O(CH_2)_4CH_3$ | |
| $NO_2$ | H | $CH_3$ | $O(CH_2)_5CH_3$ | |
| Cl | H | $CH_3$ | $OCH_2CH_2Br$ | |
| Cl | H | $CH_3$ | $O—CH_2CH—CH—CH_3$<br>        \|     \|<br>       Cl   Cl | |

TABLE 1-A

| $R_4$ | $R_5$ | X | Y | m.p. (°C) |
|---|---|---|---|---|
| $NO_2$ | H | $CH_3$ | $OCH_2CH_2CH_2CN$ | |
| Cl | H | $CH_3$ | $OCH_2CH_2CH_2CH_2CN$ | |
| $NO_2$ | H | $CH_3$ | $OCH_2—CH{=}CH_2$ | |
| Cl | H | $CH_3$ | $OCH_2—CH{=}CH—CH_3$ | |
| $NO_2$ | H | $CH_3$ | $O—CH_2—$ 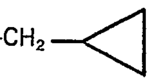 | |
| $NO_2$ | H | $CH_3$ | $OCH_2—C{\equiv}CH$ | |
| Cl | H | $CH_3$ | $OCH_2—C{\equiv}C—CH_3$ | |

TABLE 1-B

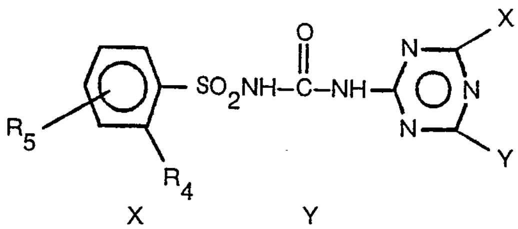

| $R_4$ | $R_5$ | X | Y | m.p. (°C) |
|---|---|---|---|---|
| Cl | H | $CH_3$ | $OCH_2CH_2OCH_3$ | 140—146° |
| Cl | 5-Cl | $CH_3$ | $OCH_2CF_3$ | |
| Cl | H | $CH_3O$ | $OCH_2CO_2C_2H_5$ | glass |
| $NO_2$ | H | $CH_3$ | $OCH_2CH_2CO_2CH_3$ | |
| $CH_3$ | H | $CH_3$ | $OCH_2CH_2CH_2OCH_3$ | |
| $OCH_3$ | H | $CH_3$ | $OCH_2CH_2OC_2H_5$ | |
| $SCH_3$ | H | $CH_3$ | $O\underset{CH_3}{\underset{\vert}{C}}HCO_2CH_3$ | |
| $\overset{O}{\overset{\uparrow}{S}}CH_3$ | H | $CH_3$ | $S—CH_2CO_2CH_3$ | |
| $CF_3$ | H | $CH_3$ | $S—CH_2CH_2OCH_3$ | |
| F | H | $OCH_3$ | $OCH_2CF_3$ | |
| Br | H | $OCH_3$ | $OCH_2CH_2OCH_3$ | |
| $\overset{O}{\overset{\uparrow}{\underset{O}{\underset{\downarrow}{S}}}}—CH_3$ | H | $OCH_3$ | $S—\underset{CH_3}{\underset{\vert}{C}}HCO_2C_2H_5$ | |
| Cl | H | $CH_3$ | $OCH_2CF_3$ | 158—166° |

TABLE 1-B

| $R_4$ | $R_5$ | X | Y | m.p. (°)C |
|---|---|---|---|---|
| Cl | H | $OCH_3$ | $OCH_2CF_3$ | 160—163° |
| Br | H | $CH_3$ | $OCH_2CF_3$ | |
| F | H | $CH_3$ | $OCH_2CF_3$ | |
| $NO_2$ | H | $CH_3O$ | $OCH_2CF_3$ | 160—168° |
| $CH_3$ | H | $CH_3$ | $OCH_2CF_3$ | |
| $OCH_3$ | H | $CH_3$ | $OCH_2CF_3$ | |
| $CF_3$ | H | $CH_3$ | $OCH_2CF_3$ | |
| $CH_3S$ | H | $CH_3$ | $OCH_2CF_3$ | |
| $CH_3SO$ | H | $CH_3$ | $OCH_2CF_3$ | |
| $CH_3SO_2$ | H | $CH_3$ | $OCH_2CF_3$ | |
| $CH_3CH_2SO_2$ | H | $CH_3$ | $OCH_2CF_3$ | |
| $CH_3CH_2CH_2SO_2$ | H | $CH_3$ | $OCH_2CF_3$ | |
| $CH_3CH_2CH_2CH_2SO_2$ | H | $CH_3$ | $OCH_2CF_3$ | |
| $CH_3CH_2CH(CH_3)—CH_2S$ | H | $CH_3$ | $OCH_2CF_3$ | |
| $CH_3(CH_2)_4CH_2SO_2$ | H | $CH_3$ | $OCH_2CF_3$ | |
| $CH_2=CH—CH_2SO_2$ | H | $CH_3$ | $OCH_2CF_3$ | |
| $CH_3CH=CHCH_2SO_2$ | H | $CH_3$ | $OCH_2CF_3$ | |
| Cl | Cl | $CH_3$ | $OCH_2CF_3$ | |
| $NO_2$ | Br | $CH_3$ | $OCH_2CF_3$ | |
| F | F | $CH_3$ | $OCH_2CF_3$ | |
| $CH_3$ | $CH_3$ | $CH_3$ | $OCH_2CF_3$ | |
| $NO_2$ | $OCH_3$ | $CH_3$ | $OCH_2CF_3$ | |
| Cl | H | $OCH_3$ | $OCH_2CH_2OCH_3$ | glass |
| Cl | H | $CH_3$ | $CH_2—CH_2—OCH_3$ | 120—130° |
| Cl | H | $CH_3O$ | $OCH_2CH_2CH_2OCH_3$ | glass |
| Cl | H | $CH_3$ | $CH_2CH_2CH_2CH_2OCH_3$ | |
| Cl | H | $CH_3$ | $CH_2OCH_2CH_3$ | |
| $NO_2$ | H | $CH_3$ | $CH_2F$ | |
| Cl | H | $CH_3$ | $CH_2Cl$ | |

TABLE 1-B

| $R_4$ | $R_5$ | X | Y | m.p. (°C) |
|---|---|---|---|---|
| $NO_2$ | H | $CH_3$ | $CH_2Br$ | |
| $NO_2$ | H | $CH_3$ | SCN | |
| Cl | H | $CH_3$ | $N_3$ | |
| Cl | H | $CH_3$ | $O-CH_2CH_2-OCH_3$ | 140—146° |
| Cl | H | $CH_3$ | $O-CH_2CH_2-OCH_2CH_3$ | oil |
| $NO_2$ | H | $CH_3$ | $O-CH_2CH_2-OCH_2CH_2CH_3$ | |
| Cl | H | $CH_3$ | $S-CH_2CH_2-SCH_3$ | |
| $NO_2$ | H | $CH_3$ | $O-CH_2CH_2-SOCH_3$ | |
| Cl | H | $CH_3$ | $O-CH_2CH_2SO_2CH_3$ | |
| Cl | H | $CH_3$ | $O-CH_2CH_2CH_2OCH_2CH_2$ | 132—135° |
| Cl | H | $CH_3$ | $O-CH_2CH_2CH_2-OCH_3$ | 54—57° |
| Cl | H | $CH_3$ | $O-CH_2CH_2-O-\overset{\overset{O}{\parallel}}{C}-CH_3$ | |
| $NO_2$ | H | $CH_3$ | $O-CH_2CH_2CH_2-O-\overset{\overset{O}{\parallel}}{C}-CH_3$ | |
| Cl | H | $CH_3$ | $S-CH_2CH_2-O-\overset{\overset{S}{\parallel}}{C}-CH_3$ | |
| $NO_2$ | H | $CH_3$ | $O-CH_2CH_2O-\overset{\overset{O}{\parallel}}{C}-CH_2CH_3$ | |
| Cl | H | $CH_3$ | $O-CH_2CH_2O-\overset{\overset{O}{\parallel}}{C}-CH_2CH_2CH_3$ | |
| $NO_2$ | H | $CH_3$ | $OCH_2CH_2O-\overset{\overset{O}{\parallel}}{C}-CH_2Br$ | |
| Cl | H | $CH_3$ | $OCH_2CH_2O-\overset{\overset{O}{\parallel}}{C}-CHCl_2$ | |
| $NO_2$ | H | $CH_3$ | $OCH_2CH_2-O-\overset{\overset{O}{\parallel}}{C}-CF_3$ | |

TABLE 1-B

| $R_4$ | $R_5$ | X | Y | m.p. (°C) |
|---|---|---|---|---|
| Cl | H | $CH_3$ | 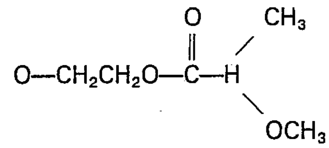 | |
| $NO_2$ | H | $CH_3$ | 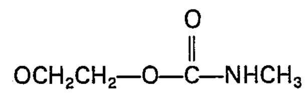 | |
| Cl | H | $CH_3$ | 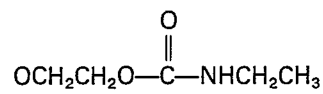 | |
| $NO_2$ | H | $CH_3$ | 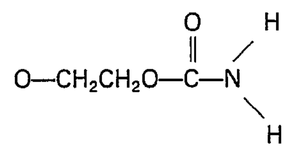 | |
| Cl | H | $CH_3$ | 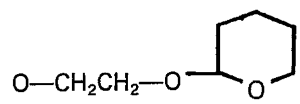 | 129—136° |
| Cl | H | $CH_3$ | $OCH_2CO_2CH_3$ | 144—147° |
| $NO_2$ | H | $CH_3$ | 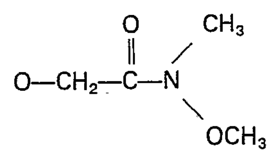 | |
| Cl | H | $CH_3$ | 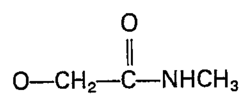 | |
| $NO_2$ | H | $CH_3$ | 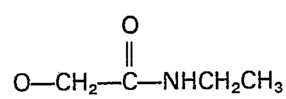 | |
| Cl | H | $CH_3$ | 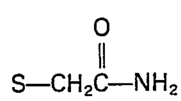 | |
| $NO_2$ | H | $CH_3O$ | 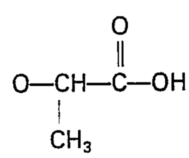 | |
| Cl | H | $CH_3O$ | 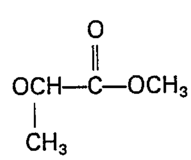 | 130—135° |
| Cl | H | $CH_3O$ | 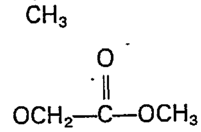 | 158—170° |

TABLE 1-B

| $R_4$ | $R_5$ | X | Y | m.p. (°C) |
|---|---|---|---|---|
| Cl | H | $CH_3$ | 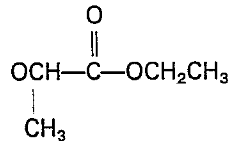 | 76—80° |
| Cl | H | $CH_3O$ | 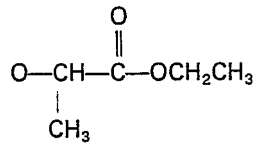 | 92° |
| Cl | H | $CH_3O$ | 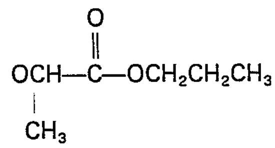 | |
| $NO_2$ | H | $CH_3O$ | 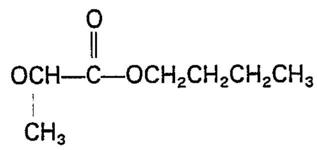 | |
| Cl | H | $CH_3O$ | 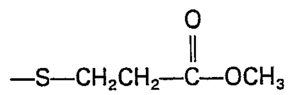 | |
| $NO_2$ | H | $CH_3O$ | $NHCH_3$ | |
| Cl | H | $CH_3O$ | NH 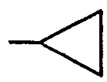 | |
| Cl | H | $CH_3$ | 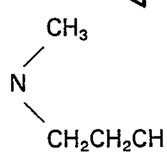 | |
| $NO_2$ | H | $CH_3$ | 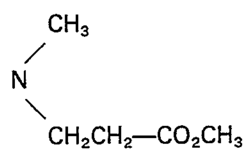 | |
| Cl | H | $CH_3$ | 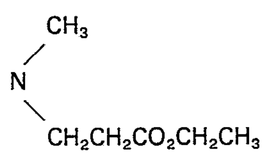 | |
| $NO_2$ | H | $CH_3$ | 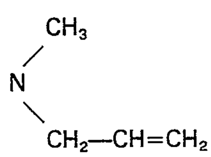 | |
| Cl | H | $OCH_3$ | $OCH_2CH_2OCH_2CH_3$ | glass |

TABLE 1-B

| $R_4$ | $R_5$ | X | Y | m.p. (°C) |
|---|---|---|---|---|
| Cl | H | $CH_3$ | 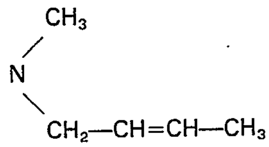 | |
| $NO_2$ | H | $CH_3$ | 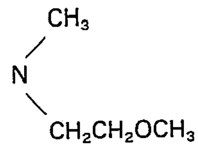 | |
| Cl | H | $CH_3$ | 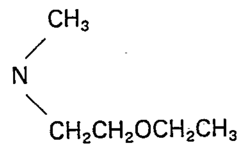 | |
| $NO_2$ | H | $CH_3$ | $O(CH_2)_3CH_3$ | |
| Cl | H | $CH_3$ | $O(CH_2)_4CH_3$ | |
| $NO_2$ | H | $CH_3$ | $O(CH_2)_5CH_3$ | |
| Cl | H | $CH_3$ | $OCH_2CH_2Br$ | |
| Cl | H | $CH_3$ | 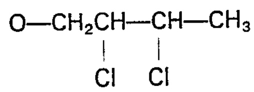 | |
| $NO_2$ | H | $CH_3$ | $OCH_2CH_2CH_2CN$ | |
| Cl | H | $CH_3$ | $OCH_2CH_2CH_2CH_2CN$ | |
| $NO_2$ | H | $CH_3$ | $OCH_2—CH=CH_2$ | |
| Cl | H | $CH_3$ | $OCH_2—CH=CH—CH_3$ | |
| $NO_2$ | H | $CH_3$ | 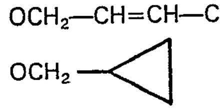 | |
| $NO_2$ | H | $CH_3$ | $OCH_2—C{\equiv}CH$ | |
| Cl | H | $CH_3$ | $OCH_2—C{\equiv}C—CH_3$ | |
| Cl | H | $CH_3O$ | $CH_2CH_2OCH_3$ | 100—107° |
| $CF_3$ | H | $CH_3$ | $CH_2CH_2OCH_3$ | 130—133° |
| Cl | H | $CH_3$ | $S—CH_2CO_2CH_3$ | 148—151° |
| Cl | H | $CH_3O$ | $SCH_2CO_2CH_3$ | 78—98° |
| Cl | H | $CH_3O$ | 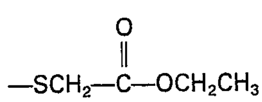 | 150—155° |
| Cl | H | $CH_3$ | 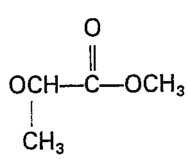 | oil |

TABLE 1-B

| $R_4$ | $R_5$ | X | Y | m.p. (°C) |
|---|---|---|---|---|
| Cl | H | $CH_3O$ | $O\underset{CH_3}{CH}-\overset{O}{\overset{\|}{C}}-OCH_3$ | 130—135° |
| Cl | H | $CH_3$ | $OCH_2CH_2Cl$ | 131—142° |

TABLE 1-C

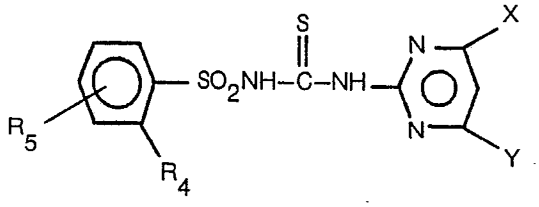

| $R_4$ | $R_5$ | X | Y |
|---|---|---|---|
| Cl | H | $CH_3$ | $OCH_2CH_2OC_2H_5$ |
| Cl | H | $CH_3$ | $OCH_2CF_3$ |
| Cl | 5-Cl | $CH_3$ | $OCH_2CO_2C_2H_5$ |
| Cl | 5-Cl | $CH_3$ | $O\underset{CH_3}{CH}CO_2CH_3$ |
| $NO_2$ | H | $CH_3$ | $OCH_2CH_2OCH_3$ |
| $NO_2$ | H | $CH_3$ | $OCH_2CF_3$ |
| Br | H | $OCH_3$ | $S—CH_2CO_2CH_3$ |
| $CH_3$ | H | $OCH_3$ | $O—CH_2CH_2CH_2OC_2H_5$ |

TABLE 1-D

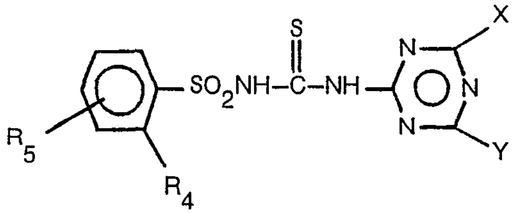

| $R_4$ | $R_5$ | X | Y |
|---|---|---|---|
| Cl | H | $CH_3$ | $OCH_2CH_2OCH_3$ |
| Cl | H | $CH_3$ | $OCH_2CF_3$ |
| Cl | 5-Cl | $CH_3$ | $OCH_2CO_2CH_3$ |
| $NO_2$ | H | $CH_3$ | $OCHCO_2C_2H_5$<br>\|<br>$CH_3$ |
| $NO_2$ | H | $CH_3$ | $S—CH_2CH_2OC_2H_5$ |
| $NO_2$ | H | $CH_3$ | $OCH_2CH_2OC_2H_5$ |
| $NO_2$ | H | $OCH_3$ | $OCH_2CF_3$ |
| Br | H | $CH_3$ | $OCH_2CH_2OCH_3$ |
| $S—CH_3$ | H | $OCH_3$ | $OCHCO_2CH_3$<br>\|<br>$CH_3$ |
| O<br>↑<br>$S—CH_3$ | H | $OCH_3$ | $S—CH_2CO_2C_2H_5$ |

Example 2

Methyl [6-methyl-2-[(2-thienylsulfonyl)amino-carbonylamino]pyrimidin-4-yloxy]acetate

To a dry stirred solution of 18 parts of methyl (2-amino-6-methylpyrimidin-4-yloxy)acetate in 300 parts of methylene chloride at ambient temperature was added 20 parts of 2-thiophenesulfonylisocyanate. The solution was allowed to stand for 3 hours and was then poured onto ice. The pH of the aqueous layer was adjusted to 11 with sodium hydroxide solution and the methylene chloride layer was separated therefrom. The aqueous layer was neutralized with aqueous hydrochloric acid and then extracted with methylene chloride. The extract was dried and stripped of solvent to yield 8 parts of methyl [6-methyl-2-[(2-thienylsulfonyl)aminocarbonylamino]pyrimidin-4-yloxy]acetate, m.p. 114—120°.

By using the procedure of Example 2 with equivalent amounts of appropriate 2-aminopyrimidines or 2-amino-1,3,5-triazines and 2-thiophenesulfonylisocyanates or isothiocyanates, the compounds of Table 2 can be prepared.

TABLE 2-A

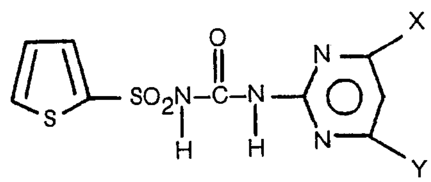

| X | Y |
|---|---|
| $CH_3$ | $OCH_2CH_2OCH_3$ |
| $CH_3$ | $OCH_2CF_3$ |
| $CH_3$ | $OCH_2CO_2CH_3$<br>\|<br>$CH_3$ |
| $CH_3$ | $O—CH_2CH_2CO_2C_2H_5$ |
| $CH_3$ | $OCH_2CH_2OCH_3$ |
| $CH_3$ | $S—CH_2CH_2OCH_2CH_3$ |
| $CH_3$ | $S—CH_2CO_2CH_3$ |
| $OCH_3$ | $O—CH_2CH_2OC_2H_5$ |
| $OCH_3$ | $O—CH_2CH_2CH_2OCH_3$ |

TABLE 2-B

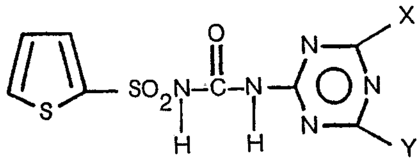

| X | Y |
|---|---|
| $CH_3$ | $OCH_2CH_2OCH_3$ |
| $CH_3$ | $OCH_2CF_3$ |
| $CH_3$ | $OCH_2CO_2CH_3$ |
| $CH_3$ | $OCHCO_2C_2H_5$<br>\|<br>$CH_3$ |
| $OCH_3$ | $OCH_2CH_2CO_2CH_3$ |
| $OCH_3$ | $OCH_2CH_2OC_2H_5$ |
| $OCH_3$ | $OCH_2CF_3$ |
| $CH_3$ | $OCH_2CO_2C_2H_5$ |
| $CH_3$ | $SCH_2CH_2OCH_3$ |
| $CH_3$ | $SCH_2CH_2CO_2CH_3$ |

TABLE 2-C

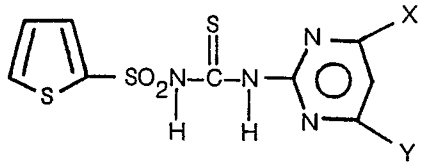

| X | Y |
|---|---|
| $CH_3$ | $OCH_2CH_2OCH_3$ |
| $CH_3$ | $OCH_2CF_3$ |
| $CH_3$ | $OCH_2CO_2C_2H5$ |
| $CH_3$ | $OCHCO_2CH_3$<br>\|<br>$CH_3$ |
| $CH_3$ | $OCH_2CH_2CO_2C_2H_5$ |
| $CH_3$ | $OCH_2CH_2OC_2H_5$ |
| $OCH_3$ | $OCH_2CO_2CH_3$ |
| $OCH_3$ | $OCH_2CF_3$ |
| $OCH_3$ | $OCH_2CH_2OCH_3$ |
| $OCH_3$ | $SCH_2CH_2OC_2H_5$ |
| $CH_3$ | $SCH_2CO_2CH_3$ |

TABLE 2-D

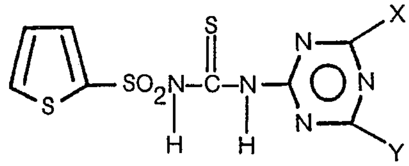

| X | Y |
|---|---|
| $CH_3$ | $OCH_2CH_2OC_2H_5$ |
| $CH_3$ | $OCH_2CF_3$ |
| $CH_3$ | $OCHCO_2CH_3$<br>\|<br>$CH_3$ |
| $OCH_3$ | $OCH_2CF_3$ |
| $OCH_3$ | $OCH_2CO_2CH_3$ |
| $OCH_3$ | $S—CH_2CH_2OC_2H_5$ |
| $OCH_3$ | $S—CH_2CO_2C_2H_5$ |
| $CH_3$ | $S—CHCO_2CH_3$<br>\|<br>$CH_3$ |
| $CH_3$ | $S—CH_2CH_2CO_2CH_3$ |

By using the procedure of Equation 2 with an equivalent amount of appropriate 2-methylamino-pyrimidines or 2-methylamino-1,3,5-triazines and appropriately substituted benzenesulfonylisocyanates or isothiocyanates, the compounds of Table 3 can be prepared. For example, to a dry stirred solution of 19.7 parts of 2-methylamino-4-(2-methyoxyethylthio)-6-methylpyrimidine in 300 parts of methylene chloride at ambient temperature is added 22.8 parts of 2-nitro-benzenesulfonylisocyanate. That mixture is stirred and refluxed for 4 hours, and then the methylene chloride is removed under reduced pressure. The resulting solid is triturated with 1-chlorobutane to yield N-[N-[4-(2-methoxyethylthio)-6-methylpyrimidin-2-yl]-N-methylaminocarbonyl]-2-nitrobenzenesulfonamide.

TABLE 3-A

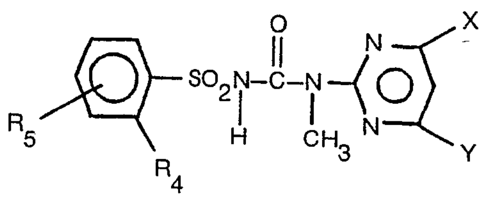

| $R_4$ | $R_5$ | X | Y |
|---|---|---|---|
| Cl | H | $CH_3$ | $SCH_2CH_2OCH_3$ |
| Cl | H | $CH_3$ | $OCH_2CF_3$ |
| Cl | 5-Cl | $CH_3$ | $OCH_2CO_2CH_3$ |
| $NO_2$ | H | $CH_3$ | $OCH_2CH_2CO_2C_2H_5$ |
| $NO_2$ | H | $OCH_3$ | $S—CH_2CH_2OC_2H_5$ |
| Br | H | $CH_3$ | $S—CH_2CO_2CH_3$ |
| $CH_3$ | H | $CH_3$ | $O—CH—CO_2CH_3$<br>\|<br>$CH_3$ |

TABLE 3-B

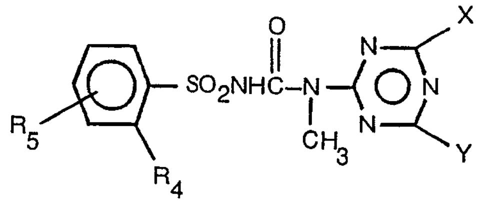

| $R_4$ | $R_5$ | X | Y |
|---|---|---|---|
| Cl | H | $CH_3$ | $SCH_2CH_2OC_2H_5$ |
| Cl | H | $CH_3$ | $OCH_2CF_3$ |
| Cl | 5-Cl | $CH_3$ | $S—CH_2CH_2OC_2H_5$ |
| $NO_2$ | H | $CH_3$ | $OCH_2CO_2C_2H_5$ |
| Br | H | $CH_3$ | $OCH_2CH_2CO_2CH_3$ |
| $OCH_3$ | H | $OCH_3$ | $OCH_2CF_3$ |

TABLE 3-C

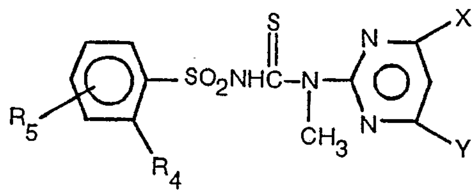

| $R_4$ | $R_5$ | X | Y |
|---|---|---|---|
| Cl | H | $CH_3$ | $SCH_2CH_2OCH_3$ |
| Cl | H | $CH_3$ | $OCH_2CO_2C_2H_5$ |
| Cl | 5-Cl | $CH_3$ | $OCH_2CH_2CH_2OCH_3$ |
| $NO_2$ | H | $CH_3$ | $OCH_2CF_3$ |
| $NO_2$ | H | $OCH_3$ | $OCH_2CF_3$ |

TABLE 3-D

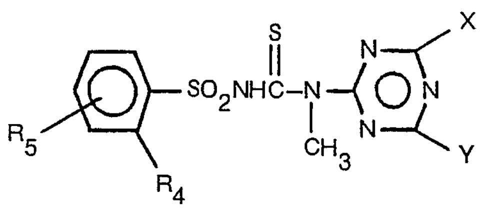

| $R_4$ | $R_5$ | X | Y |
|---|---|---|---|
| Cl | H | $CH_3$ | $OCH_2CH_2OC_2H_5$ |
| Cl | H | $CH_3$ | $OCHCO_2CH_3$ (with $CH_3$ on the CH) |
| Cl | 5-Cl | $OCH_3$ | $OCH_2CF_3$ |
| $NO_2$ | H | $CH_3$ | $S—CH_2CH_2OC_2H_5$ |
| $NO_2$ | H | $OCH_3$ | $O—CH_2CH_2CO_2C_2H_5$ |
| Br | H | $CH_3$ | $OCH_2CF_3$ |

### Example 3

N-[N-[4-(2,2,2-Trifluoroethoxy)-6-methylpyrimidin-2-yl]-N-methylaminocarbonyl]-2-thiophenesulfonamide

To a dry stirred solution of 22.1 parts of 2-methylamino-4-(2,2,2-trifluorethoxy)-6-methylpyrimidine in 250 parts of methylene chloride at ambient temperature was added 18.9 parts of 2-thiophenesulfonyl

isocyanate. That mixture was stirred and refluxed for 2 hours. The methylene chloride was removed under reduced pressure, and the resulting solid was triturated with toluene to yield 19 parts of N-[N-[4-(2,2,2-trifluoroethoxy)-6-methylpyrimidin-2-yl]-N-methylaminocarbonyl]-2-thiophenesulfonamide.

By using the procedure of Example 3 with equivalent amounts of appropriate 2-methylaminopyrimidines or 2-methylamino-1,3,5-triazines and 2-thiophenesulfonyl isocyanates or isothiocyanates, the compounds of Table 4 can be prepared.

Table 4-A

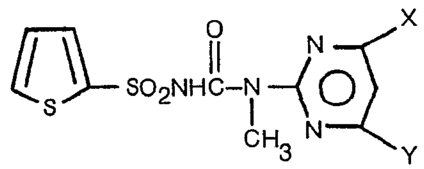

| X | Y |
|---|---|
| $CH_3$ | $SCH_2CH_2OCH_3$ |
| $CH_3$ | $OCH_2CO_2CH_3$ |
| $CH_3$ | $OCH_2CH_2CO_2CH_3$ |
| $CH_3$ | $-S-CH_2CH_2OC_2H_5$ |
| $OCH_3$ | $OCH_2CH_2OC_2H_5$ |
| $OCH_3$ | $OCH_2CF_3$ |
| $OCH_3$ | $OCH(CH_3)-CO_2CH_3$ |

TABLE 4-B

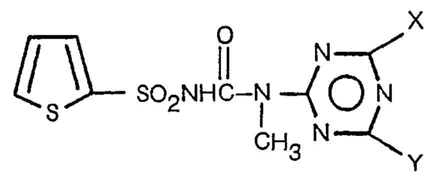

| X | Y |
|---|---|
| $CH_3$ | $OCH_2CH_2OC_2H_5$ |
| $CH_3$ | $OCH_2CF_3$ |
| $CH_3$ | $OCH_2CH_2CH_2OCH_3$ |
| $OCH_3$ | $S-CH_2CH_2CH_2OC_2H_5$ |
| $OCH_3$ | $S-CH_2CO_2C_2H_5$ |

TABLE 4-C

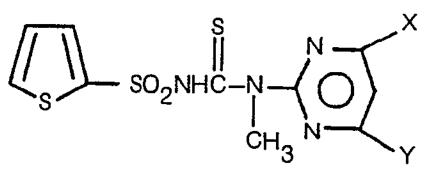

| X | Y |
|---|---|
| $CH_3$ | $SCH_2CH_2OCH_3$ |
| $CH_3$ | $OCH_2CF_3$ |
| $CH_3$ | $OCH_2CH_2CO_2CH_3$ |
| $OCH_3$ | $S-CH_2CH_2OC_2H_5$ |

TABLE 4-D

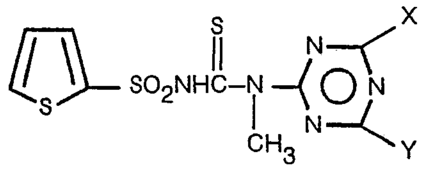

| X | Y |
|---|---|
| $CH_3$ | $OCHCO_2C_2H_5$<br>\|<br>$CH_3$ |
| $CH_3$ | $S{-}CH_2CO_2CH_3$ |
| $CH_3$ | $SCH_2CH_2OCH_3$ |
| $OCH_3$ | $OCH_2CF_3$ |

N-methyl compounds of this invention can be prepared in accordance with the procedures described above. For example, the methylation procedure of Equation 3 can be used as follows:

An equivalent amount of sodium hydride (50% mineral oil dispersion) is added to a solution of N-[[4-(2-methoxyethylthio)-6-methylpyrimidin-2-yl]amino-carbonyl]-2,5-dichlorobenzenesulfonamide in dimethylformamide under a nitrogen atmosphere. After hydrogen evolution ceases, an equivalent amount of dimethylsulfate is added. After stirring for 12 hours the reaction mixture is poured into a large volume of water. The resulting precipitate is filtered to yield N-[[4-(2-methoxyethylthio)-6-methylpyrimidin-2-yl]aminocarbonyl]-2,5-dichloro-N-methylbenzenesulfonamide.

Likewise, compounds of this invention wherein both of the urea nitrogens are methylated can be prepared by the procedure of Equation 4 as follows:

To 18 parts of N-[(2-chlorophenyl)sulfonyl]-N-methylcarbamyl chloride in 300 parts of tetrahydrofuran containing 10 parts of triethylamine is added 22 parts of 2-methylamino-4-(2,2,2-trifluoroethoxy)-6-methylpyrimidine. After stirring at reflux for 10 hours, the precipitated salts are filtered off and the filtrate is concentrated to yield N-[N-[4-(2,2,2,-trifluoroethoxy)-6-methylpyrimidin-2-yl]-N-methylaminocarbonyl]-2-chloro-N-methylbenzenesulfonamide.

By using an appropriate N-heterocyclic-N-aminocarbonylbenzenesulfonamide in the foregoing methylation procedure, the compounds of Table 5 can be prepared. Alternatively, by using an appropriate carbamylchloride and an appropriate methylaminoheterocycle with the above-described procedure, the compounds of Table 5 can be prepared.

TABLE 5-A

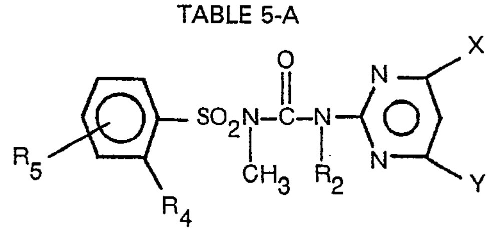

| $R_2$ | $R_4$ | $R_5$ | X | Y |
|---|---|---|---|---|
| H | Cl | H | $CH_3$ | $SCH_2CH_2OCH_3$ |
| H | Cl | H | $CH_3$ | $OCH_2CF_3$ |
| H | Cl | H | $CH_3$ | $OCH_2CO_2CH_3$ |
| H | Cl | 5-Cl | $CH_3$ | $OCH_2CH_2CO_2C_2H_5$ |
| H | $NO_2$ | H | $CH_3$ | $OCH_2CH_2OC_2H_5$ |
| H | $NO_2$ | H | $CH_3$ | $SCH_2CH_2OC_2H_5$ |
| H | $NO_2$ | H | $CH_3$ | $OCHCO_2CH_3$<br>\|<br>$CH_3$ |
| H | $NO_2$ | H | $OCH_3$ | $S{-}CH_2CO_2CH_3$ |
| H | Br | 5-Cl | $OCH_3$ | $OCH_2CF_3$ |
| $CH_3$ | $CH_3$ | H | $CH_3$ | $SCH_2CH_2OCH_3$ |
| $CH_3$ | Cl | H | $CH_3$ | $OCH_2CO_2CH_3$ |
| $CH_3$ | Cl | 5-Cl | $OCH_3$ | $OCH_2CH_2OC_2H_5$ |
| $CH_3$ | $NO_2$ | H | $CH_3$ | $OCH_2CF_3$ |
| $CH_3$ | $NO_2$ | H | $CH_3$ | $SCHCO_2C_2H_5$<br>\|<br>$CH_3$ |

TABLE 5-B

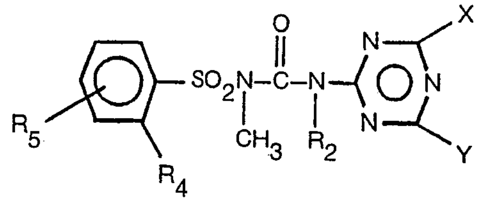

| $R_2$ | $R_4$ | $R_5$ | X | Y |
|---|---|---|---|---|
| H | Cl | H | $CH_3$ | $SCH_2CH_2OCH_3$ |
| H | Cl | 5-Cl | $CH_3$ | $OCH_2CF_3$ |
| H | $NO_2$ | H | $CH_3$ | $OCH_2CO_2CH_3$ |
| H | $NO_2$ | H | $CH_3$ | $S—CH_2CO_2CH_3$ |
| H | $NO_2$ | H | $OCH_3$ | $OCHCO_2CH_3$<br>\|<br>$CH_3$ |
| $CH_3$ | $OCH_3$ | H | $CH_3$ | $SCH_2CH_2OCH_3$ |
| $CH_3$ | $S—CH_3$ | H | $CH_3$ | $S—CH_2CH_2OC_2H_5$ |
| $CH_3$ | O<br>↑<br>$SCH_3$<br>↓<br>O | H | $OCH_3$ | $OCH_2CH_2CH_2OCH_3$ |
| $CH_3$ | $NO_2$ | H | $CH_3$ | $OCH_2CF_3$ |

By using an appropriately substituted n-methyl-N-phenylsulfonylthiocarbamyl chloride and an appropriately substituted 2-aminopyrimidine or 2-amino-1,3,5-triazine the compunds of Table 6 can be made in accordance with the procedure exemplified above.

TABLE 6-A

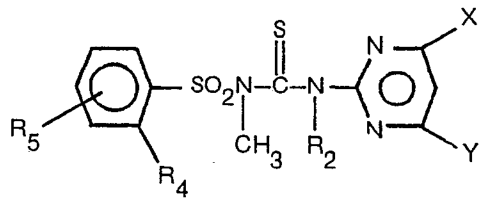

| $R_2$ | $R_4$ | $R_5$ | X | Y |
|---|---|---|---|---|
| H | Cl | H | $CH_3$ | $SCH_2CH_2OCH_3$ |
| H | Cl | 5-Cl | $CH_3$ | $OCH_2CF_3$ |
| H | $NO_2$ | H | $CH_3$ | $OCH_2CO_2CH_3$ |
| H | $NO_2$ | H | $CH_3$ | $S—CH_2CH_2OCH_3$ |
| H | $CH_3$ | H | $CH_3$ | $S—CH_2CO_2C_2H_5$ |
| H | $OCH_3$ | H | $OCH_3$ | $OCHCO_2C_2H_5$<br>\|<br>$CH_3$ |
| $CH_3$ | Br | H | $CH_3$ | $OCH_2CH_2OC_2H_5$ |
| $CH_3$ | Cl | 5-Cl | $CH_3$ | $SCHCO_2CH_3$<br>\|<br>$CH_3$ |
| $CH_3$ | $NO_2$ | H | $CH_3$ | $OCH_2CF_3$ |
| $CH_3$ | $NO_2$ | H | $OCH_3$ | $OCH_2CH_2CH_2OCH_3$ |

TABLE 6-B

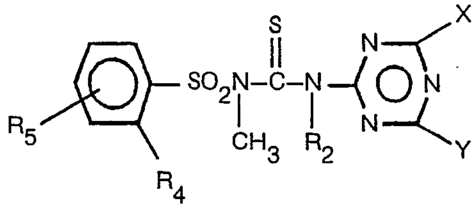

| $R_2$ | $R_4$ | $R_5$ | X | Y |
|---|---|---|---|---|
| $CH_3$ | Cl | H | $CH_3$ | $SCH_2CH_2OCH_3$ |
| $CH_3$ | Cl | 5-Cl | $CH_3$ | $OCH_2CO_2CH_3$ |
| $CH_3$ | $NO_2$ | H | $CH_3$ | $S—CH_2CH_2OCH_3$ |
| $CH_3$ | $NO_2$ | H | $CH_3$ | $S—CH_2CH_2CO_2CH_3$ |
| $CH_3$ | $CH_3$ | H | $OCH_3$ | $OCH(CH_3)CO_2CH_3$ |
| H | Br | H | $CH_3$ | $OCH_2CH_2OC_2H_5$ |
| H | Cl | 5-Cl | $CH_3$ | $OCH_2CF_3$ |
| H | $NO_2$ | H | $CH_3$ | $OCH_2CH_2CH_2OCH_3$ |
| H | $SCH_3$ | H | $OCH_3$ | $SCH_2CO_2C_2H_5$ |

The compounds of Table 7 can be prepared by the aforesaid methylation reaction by using an appropriate N-[(1,3,5-triazin-2-yl)aminocarbonyl]-2-thiophenesulfonamide; N-[(pyrimidin-2-yl)amino-carbonyl)2-thiophenesulfonamide; N-[(1,3,5-triazin-2-yl)-aminothioxomethyl]-2-thiophenesulfonamide or N-[(pyrimidin-2-yl)aminothioxomethyl]-2-thiophenesulfonamide. Alternatively, the compounds of Table 7 can be prepared in accordance with the above-exemplified procedure, using an appropriately substituted N-methyl-N-(2-thienylsulfonyl)carbamyl chloride or thiocarbamyl chloride and an appropriately substituted 2-aminopyrimidine or 2-amino-1,3,5-triazine.

TABLE 7-A

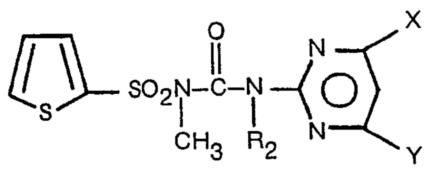

| $R_2$ | X | Y |
|---|---|---|
| H | $CH_3$ | $SCH_2CH_2OCH_3$ |
| H | $CH_3$ | $OCH_2CF_3$ |
| H | $OCH_3$ | $OCH_2CO_2CH_3$ |
| H | $OCH_3$ | $S—CH_2CH_2OC_2H_5$ |
| H | $CH_3$ | $O—CH_2CH_2CH_2OCH_3$ |
| $CH_3$ | $CH_3$ | $S—CH(CH_3)—CO_2C_2H_5$ |
| $CH_3$ | $CH_3$ | $O—CH_2CH_2CO_2CH_3$ |
| $CH_3$ | $CH_3$ | $OCH_2CH_2OC_2H_5$ |
| $CH_3$ | $CH_3$ | $OCH_2CF_3$ |

TABLE 7-B

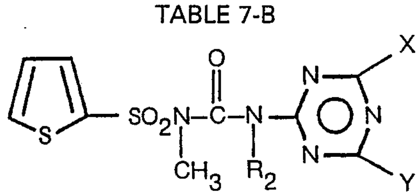

| $R_2$ | X | Y |
|---|---|---|
| H | $CH_3$ | $SCH_2CH_2OCH_3$ |
| H | $CH_3$ | $OCH_2CF_3$ |
| H | $CH_3$ | $SCH_2CH_2CH_2OCH_2$ |
| H | $OCH_3$ | $OCH_2CO_2CH_3$ |
| H | $OCH_3$ | $SCH_2CO_2CH_3$ |
| $CH_3$ | $OCH_3$ | $OCHCO_2C_2H_5$<br>$CH_3$ |
| $CH_3$ | $CH_3$ | $OCH_2CH_2CO_2CH_3$ |
| $CH_3$ | $CH_3$ | $SCH_2CH_2OC_2H_5$ |
| $CH_3$ | $CH_3$ | $SCHCO_2CH_3$<br>$CH_3$ |
| $CH_3$ | $CH_3$ | $OCH_2CF_3$ |

TABLE 7-C

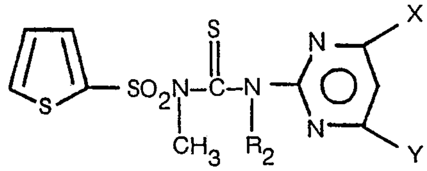

| $R_2$ | X | Y |
|---|---|---|
| H | $CH_3$ | $OCH_2CH_2OC_2H_5$ |
| H | $CH_3$ | $OCH_2CF_3$ |
| H | $CH_3$ | $OCHCO_2CH_3$<br>$CH_3$ |
| H | $O—CH_3$ | $OCH_2CF_3$ |
| H | $CH_3$ | $OCH_2CH_2CO_2CH_3$ |
| H | $CH_3$ | $SCH_2CH_2CH_2OCH_3$ |
| $CH_3$ | $CH_3$ | $S—CH_2CH_2OC_2H_5$ |
| $CH_3$ | $CH_3$ | $S—CH_2CH_2CO_2CH_3$ |
| $CH_3$ | $O—CH_3$ | $SCH_2CH_2OCH_3$ |
| $CH_3$ | $O—CH_3$ | $OCH_2CF_3$ |

TABLE 7-D

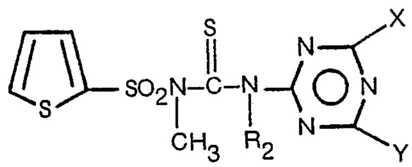

| $R_2$ | X | Y |
|---|---|---|
| H | $CH_3$ | $SCH_2CH_2OCH_3$ |
| H | $OCH_3$ | $OCH_2CF_3$ |
| H | $CH_3$ | $OCH_2CO_2CH_3$ |
| $CH_3$ | $CH_3$ | $S—CH_2CH_2OC_2H_5$ |
| $CH_3$ | $OCH_3$ | $S—CH_2CO_2CH_3$ |
| $CH_3$ | $CH_3$ | $OCH_2CH_2CH_3OCH_3$ |
| $CH_3$ | $CH_3$ | $OCHCO_2C_2H_5$<br>$CH_3$ |

Formulations

Useful formulations of the compounds of Formula I can be prepared in conventional ways. They include dusts, granules, pellets, suspensions, emulsions, wettable powders, emulsifiable concentrates and the like. Many of them can be applied directly. Sprayable formulations can be extended in suitable media and used at spray volumes of from a few liters to several hundred liters per hectare. High strength compositions are used primarily as concentrates which are to be diluted prior to ultimate use. The formulations, broadly, contain about 0.1% to 99% by weight of active ingredient(s) and at least one of a) about 0.1% to 20% surfactant(s) and b) about 1% to 99.9% solid or liquid diluent(s). More specifically, they will contain these ingredients in the approximate proportions set forth in Table 8.

TABLE 8

| | Active Ingredient | Weight Percent* Diluent(s) | Surfactant(s) |
|---|---|---|---|
| Wettable Powders | 20—90 | 0—74 | 1—10 |
| Oil Suspensions, Emulsions (including Emulsifiable Concentrates) | 5—50 | 40—95 | 0—15 |
| Aqueous Suspensions | 10—50 | 40—84 | 1—20 |
| Dusts | 1—25 | 70—99 | 0—5 |
| Granules and Pellets | 0.1—95 | 5—99.9 | 0—15 |
| High Strength Compositions | 90—99 | 0—10 | 0—2 |

* Active Ingredient plus at least one of a Surfactant or a Diluent equals 100 weight percent.

Lower or higher levels of active ingredient can be present, depending on the intended use and the physical properties of the compound. Higher ratios of surfactant to active ingredient are sometimes desirable, and are achieved by incorporation into the formulation, or by tank mixing.

Some typical solid diluents are described in Watkins, et al., "Handbook of Insecticide Dust Diluents and Carriers", 2nd Ed., Dorland Books, Caldwell, New Jersey, but other solids, either mined or manufactured, may be used. The more absorptive diluents are preferred for wettable powders and the denser ones for dusts. Typical liquid diluents and solvents are described in Marsden, "Solvents Guide", 2nd Ed., Interscience, New York, 1950. Solubility under 0.1% is preferred for suspension concentrates; solution concentrates are preferably stable against phase separation at 0°C. "McCutcheon's Detergents and Emulsifiers Annual", MC Publishing Corp., Ridgewood, New Jersey, as well as Sisely and Wood, "Encyclopedia of Surface Active Agents", Chemical Publishing Co., Inc., New York, 1964, list surfactants and recommended uses. All formulations can contain minor amounts of additives to reduce foaming, caking, corrosion, microbiological growth, etc.

The methods of making such compositions are well known. Solutions are prepared by simply mixing the ingredients. Fine solid compositions are made by blending, and usually grinding, as in a hammer or fluid energy mill. Suspensions are prepared by wet milling (see, for example, Littler, U.S. Patent 3,060,084). Granules and pellets can be made by spraying the active material on preformed granular carriers or by agglomeration techniques. See J. E. Browning, "Agglomeration", *Chemical Engineering,* Dec. 4, 1967, pp. 147ff. and "Perry's Chemical Engineer's Handbook", 4th ed., McGraw-Hill, New York, 1963, pp, 8—59ff.

For further information regarding the art of formulation, see for example:

H. M. Loux, U.S. Patent 3,235,361, Col. 6, line 16 through Col. 7, line 19 and Examples 10 through 41.

R. W. Luckenbaugh, U.S. Patent 3,309,192, Col. 5, line 43 through Col 7, line 62 and Examples 8, 12, 15, 39, 41, 52, 53, 132, 138—140, 162—164, 166, 167, 169—182.

H. Gysin and E. Knusli, U.S. Patent 2,891,855, Col. 3, line 66 through Col. 5, line 17 and Examples 1—4.

G. C. Klingman, "Weed Control as a Science", John Wiley & Sons, Inc., New York, 1961, pp. 81—96.

J. D. Fryer and S. A. Evans, "Weed Control Handbook", 5th Ed., Blackwell Scientific Publications, Oxford, 1968, pp. 101—103.

Unless indicated otherwise, all parts are by weight in the following examples.

Example 4

*Wettable Powder:*

| | |
|---|---|
| N-[[4-(2,2,2-Trifluoroethoxy)-6-methylpyrimidin-2-yl]aminocarbonyl]-2-nitrobenzenesulfonamide | 90% |
| dioctyl sodium sulfosuccinate | 0.1% |
| sodium ligninsulfonate | 1% |
| synthetic fine silica | 8.9% |

The ingredients are blended and ground in a hammer-mill to produce particles almost all of which are below 100 microns in size. That material is sifted through a U.S.S. No. 50 screen and packaged.

Example 5

*Granule*

| | |
|---|---|
| wettable powder of Example 4 | 10% |
| attapulgite granules (U.S.S. #20—40; 0.84—0.42 mm) | 90% |

A slurry of wettable powder containing 50% solids is sprayed onto the surface of attapulgite granules in a double-cone blender. The granules are dried and packaged.

EXAMPLE 6

*Wettable Powder*

| | |
|---|---|
| N-[[4-(2-methoxyethoxy)-6-methylpyrimidin-2-yl]-aminocarbonyl]-2-nitrobenzenesulfonamide | 40% |
| dioctyl sodium sulfosuccinate | 1.5% |
| sodium lignimsulfonate | 3% |
| low viscosity methyl cellulose | 1.5% |
| attapulgite | 54% |

The ingredients are thoroughly blended and passed through an air mill to produce an average particle size under 15 microns, reblended, and sifted through a U.S.S. No. 50 sieve (0.3 mm opening) before packaging.

Example 7

*Granule*

| | |
|---|---|
| Wettable powder of Example 6 | 25% |
| gypsum | 64% |
| potassium sulfate | 11% |

The ingredients are blended in a rotating mixer, and water is sprayed onto that blend so as to effect granulation. When most of the granules have reached 1.0 to 0.42 mm (U.S.S. #18 to 40 sieves) in size, they are removed, dried, and screened. Oversize material is crushed to produce additional material in the desired range. The resulting granules contain 10% of the active ingredient.

Example 8

*Wettable powder*

| | |
|---|---|
| N-[[4-(2,2,2-trifluoroethoxy)-6-methylpyrimidin-2-yl]aminocarbonyl]-2-nitrobenzenesulfonamide | 80% |
| sodium alkylnaphthalenesulfonate | 2% |
| sodium ligninsulfonate | 2% |
| synethetic amorphorus silica | 3% |
| kaolinite | 13% |

The ingredients are thoroughly blended after grinding in a hammer mill to produce particles essentially all of which are under 100 microns in size; the material is reblended, sifted through a U.S.S. No. 50 sieve and packaged.

Example 9

*Wettable Powder*

| | |
|---|---|
| N-[[4-(2-methoxyethoxy)-6-methylpyrimidin-2-yl]-aminocarbonyl]-2-nitrobenzenesulfonamide | 65% |
| dodecylphenol polyethylene glycol ether | 2% |
| sodium ligninsulfonate | 4% |
| sodium silicoaluminate | 6% |
| montmorillonite (calcined) | 23% |

The ingredients are thoroughly blended. The liquid surfactant is added by spraying on the solid ingredients in a blender. After grinding in a hammer mill to produce particles almost all of which are below 100 microns in size, the material is reblended, sifted through a U.S.S. #50 sieve (0.3 mm opening) and packaged.

Example 10

*Oil Suspension*

| | |
|---|---|
| N-[[4-(2,2,2-trifluoroethoxy)-6-methylpyrimidin-2-yl]aminocarbonyl]-2-nitrobenzenesulfonamide | 25% |
| polyoxyethylene sorbitol hexaoleate | 5% |
| highly aliphatic hydrocarbon oil | 70% |

The ingredients are ground together in a sand mill until the solid particles have been reduced to under about 5 microns. The resulting suspension may be applied directly, but preferably after being extended further with oils or emulsified in water.

Example 11

*Aqueous Suspension*

| | |
|---|---|
| N-[[4-(2-methoxyethoxy)-6-methylpyrimidin-2-yl]-aminocarbonyl]-2-nitrobenzenesulfonamide | 25% |
| hydrated attapulgite | 3% |
| crude calcium ligninsulfonate | 10% |
| sodium dihydrogen phosphate | 0.5% |
| water | 61.5% |

The ingredients are ground together in a ball or roller mill until the solid particles have been reduced to sizes under 10 microns, and then packaged.

Example 12

*Extruded Pellet*

| | |
|---|---|
| N-[[4-(2,2,2-trifluoroethoxy)-6-methylpyrimidin-2-yl]aminocarbonyl]-2-nitrobenzenesulfonamide | 25% |
| anhydrous sodium sulfate | 10% |
| crude calcium ligninsulfonate | 5% |
| sodium alkylnaphthalenesulfonate | 1% |
| calcium/magnesium bentonite | 59% |

The ingredients are blended, hammer milled and then moistened with about 12% water. The mixture is extruded in the form of cylinders about 3 mm in diameter which are cut to produce pellets about 3 mm long. The pellets may be used directly, after drying, or dried pellets may be crushed to pass a U.S.S. No. 20 sieve (0.84 mm openings). The granules held on a U.S.S. No. 40 sieve (0.42 mm openings) may be packaged for use and the fines recycled.

Example 13

*Solution*

| | |
|---|---|
| N-[[4-(2,2,2-trifluoroethoxy)-6-methylpyrimidin-2-yl]aminocarbonyl]-2-nitrobenzenesulfonamide | 5% |
| dimethylformamide | 95% |

The ingredients are combined and stirred to produce a solution, which can be used for low volume applications.

*Utility*

The compounds of the present invention are highly active herbicides. They have utility for broad spectrum pre- and/or post-emergence weed control in areas where complete control of all vegetation is desired, such as around fuel storage tanks, ammunition depots, industrial storage areas, oil well sites, drive-in theatres, parking lots, billboards, highway and railroad structures. By properly selecting rate and time of application, compounds of this invention may be used also to modify plant growth beneficially.

The precise amount of the compound of Formula I to be used in any given situation will vary according to the particular end result desired, the amount of foliage present, the weeds to be controlled, the soil type, the formulation and mode of application, weather conditions, etc. Since so many variables play a role, it is not possible to state a rate of application suitable for all situations. Broadly speaking, the compounds of this invention are used at levels of about 0.005 to 20 kg/ha with a preferred range of 0.125 to 10 kg/ha. In general, the higher rates of application from within this range will be selected for adverse conditions or where extended persistence in soil is desired.

The compounds of Formula I may be combined with other herbicides and are particularly useful in combination with ureas, such as 3-(3,4-dichlorophenyl)-1,1-dimethylurea, 3-(3,4-dichlorophenyl)-1-methoxy-1-methylurea, and 1,1-dimethyl-3-(α,α,α-trifluoro-*m*-tolyl)urea; the triazines such as 2-chloro-4-(ethylamino)-6-(isopropylamino)-*s*-triazine; the uracils such as 5-bromo-3-*sec*-butyl-6-methyluracil; N-(phosphono-methyl)-glycine; 3-cyclohexyl-1-methyl-6-dimethylamino-*s*-triazine-2,4(1H,3H)-dione, N,N-dimethyl-2,2-diphenylacetamide; 2,4-dichlorophenoxyacetic acid (and closely related compounds); 4-chloro-2-butynyl-3-chlorophenylcarbamate; diisopropylthiolcarbamic acid, ester with 2,3-dichloroallyl alcohol; diisopropylthiolcarbamic acid, S-(2,3,3-trichloroallyl)ester; ethyl-N-benzoyl-N-(3,4-dichlorophenyl)-2-aminopropionate, 1,2-dimethyl-3,5-diphenylpyrazolium methylsulfate; methyl 2-[4-(2,4-dichlorophenoxy)phenoxy]propanoate; 4-amino-6-*tert*-butyl-3-(methylthio)-1,2,4-triazin-5(4H)-one; 3-isopropyl-1H-2,1,3-benzothiodiazin(4)-3H-one 2,2-dioxide; α,α,α-trifluoro-2,6-dinitro-N,N-dipropyl-*p*-toluidine; 1,1'-dimethyl-4,4'-bipyridinium ion; monosodium methanearsonate; and 2-chloro-2',6'-diethyl(methoxymethyl)acetanilide.

The activity of the compounds of this invention was discovered in greenhouse tests. The tests are described and the data resulting from them are shown below.

0 = no effect
10 = maximum effect
B = Burn
C = chlorosis or necrosis
D = defoliation
E = emergence inhibition
G = growth retardation
H = formative effects
S = albinism
U = unusual pigmentation
6Y = abscised buds or flowers.

Test Procedure A

Seeds of crabgrass (*Digitaria* spp.), barnyard-grass (*Echinochloa crusgalli)*, wild oats (*Avena fatua*), cassia *(Cassia tora*), morningglory (*Ipomoea* spp.), cocklebur (*Xanthium* spp.), sorghum, corn, soybean, rice, and wheat, as well as nutsedge tubers (*Cyperus rotundus*), were planted in a growth medium and treated preemergence with a nonphytotoxic solvent solution of the compounds of Table 9. Other batches of seeds and tubers for all of the foregoing weed and crop plants were planted at the same time as controls. The control plantings were untreated; i.e., neither any compound nor any solvent was applied. At the same time, cotton having five leaves (including cotyledonary ones), bush beans with the third trifoliate leaf expanding, crabgrass with two leaves, barnyardgrass with two leaves, wild oats with two leaves, cassia with three leaves (including cotyledonary ones), morningglory with four leaves (including the coytledonary ones), cocklebur with four leaves (including the cotyledonary ones), sorghum with four leaves, corn with four leaves, soybean with two cotyledonary leaves, rice with three leaves, wheat with one leaf, and nutsedge with three-five leaves were sprayed with a nonphytotoxic solvent solution of the compounds of Table 9. Other groups of all the same weed and crop plants were sprayed with the same nonphytotoxic solvent so as to provide control plants. Premergence and postemergence treated plants and controls were maintained in a greenhouse for sixteen days, then all treated plants were compared with their respective controls and rated visually for response to treatment. The data in Table 9 indicate that certain of the compounds of this invention have utility for selective post-emergence weed control in wheat.

TABLE 9

| | 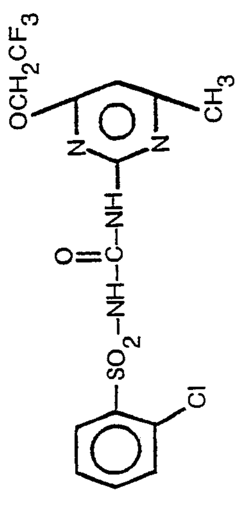 | 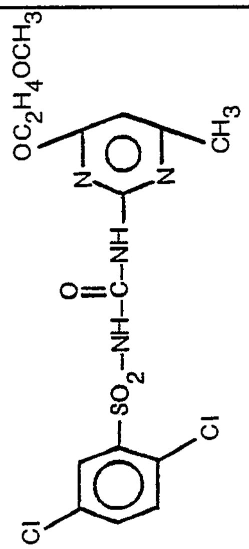 | 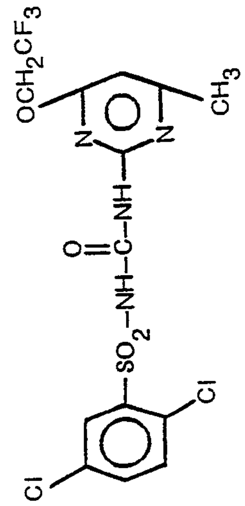 |
|---|---|---|---|
| Rate, kg/ha | 2 | 2 | 2 |
| POST-EMERGENCE | | | |
| Bushbean | 7D, 8G, 6Y | 3G, 4C, 6Y | 3C, 4G, 6Y |
| Cotton | 4C, 9G | 2C, 3H, 6G | 2C, 5G |
| Morningglory | 10C | 10C | 7C |
| Cocklebur | 10C | 2C, 7G | 3C, 7G |
| Cassia | 2C, 7G | 2C, 6G | 2C, 7G |
| Nutsedge | 8G | 2C, 7G | 2G |
| Crabgrass | 1C, 4G | 6G | 4G |
| Barnyardgrass | 2C, 8H | 3C, 7H | 2H |
| Wild Oats | 4G | 2G | 0 |
| Wheat | 3G | 2G | 0 |
| Corn | 1C, 5G | 3C, 6G | 2C |
| Soybean | 5H, 7G | 9C | 2H, 6G |
| Rice | 5G | 2C, 5G | 1C |
| Sorghum | 7G | 3C, 7G | 0 |
| PRE-EMERGENCE | | | |
| Morningglory | 9G | 9G | 8G |
| Cocklebur | 8G | 8G | 7G |
| Cassia | 9G | 9G | 4G |
| Nutsedge | 10E | 10E | 9G |
| Crabgrass | 7G | 8G | 3G |
| Barnyardgrass | 8G | 3C, 9H | 1C |
| Wild Oats | 4G | 6G | 0 |
| Wheat | 5G | 8G | 3G |
| Corn | 9H | 9G | 2C, 4G |
| Soybean | 8H | 9H | 3H |
| Rice | 10E | 9H | 6G |
| Sorghum | 9H | 2C, 9G | 3G |

TABLE 9 (Continued)

| | 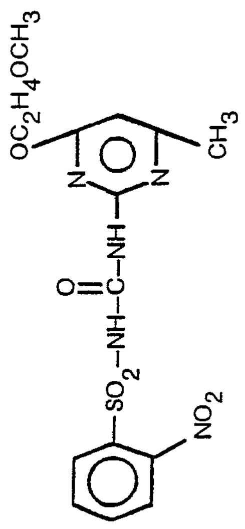 | 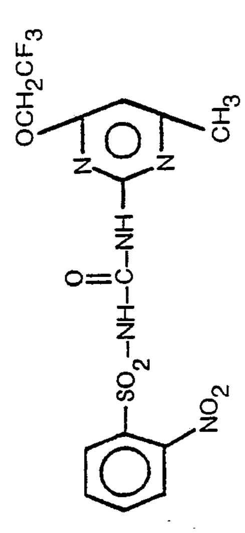 | 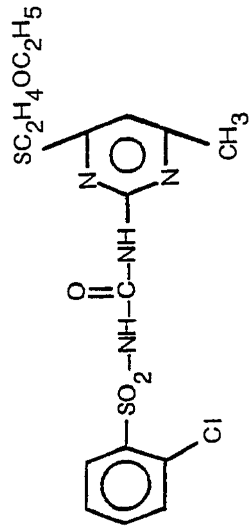 |
|---|---|---|---|
| Rate, kg/ha | 2 | 2 | 2 |
| POST-EMERGENCE | | | |
| Bushbean | - | - | 3C, 8G, 6Y |
| Cotton | 9C | 5C, 9C | - |
| Morningglory | 9C | 10C | 1C, 9G |
| Cocklebur | 9C | 10C | 1C, 7G |
| Cassia | 9C | 10C | 8H |
| Nutsedge | 5C | 8C | 9G |
| Crabgrass | 9C | 8C | 2C, 8G |
| Barnyardgrass | 9C | 9C | 9H |
| Wild Oats | 9C | 9C | 0 |
| Wheat | 9C | 9C | 0 |
| Corn | 10C | 10C | 2H, 8G |
| Soybean | 9C | 3C, 8G | 2H, 9G |
| Rice | 5C, 8G | 5C, 7G | 2C, 8G |
| Sorghum | 7C | 5C, 9G | 9H |
| PRE-EMERGENCE | | | |
| Morningglory | 10E | 9G | 9G |
| Cocklebur | 8G | 9C | 9G |
| Cassia | 9G | 9C | 8G |
| Nutsedge | 10E | 10E | 10E |
| Crabgrass | 10E | 10E | 3G |
| Barnyardgrass | 9H | 9H | 9H |
| Wild Oats | 9C | 3C, 8G | 6G |
| Wheat | 9H | 9H | 6G |
| Corn | 10E | 9H | 2U, 4G |
| Soybean | 9H | 9H | 6H |
| Rice | 10E | 10E | 10E |
| Sorghum | 9H | 9E | 9H |

TABLE 9 (Continued)

| | 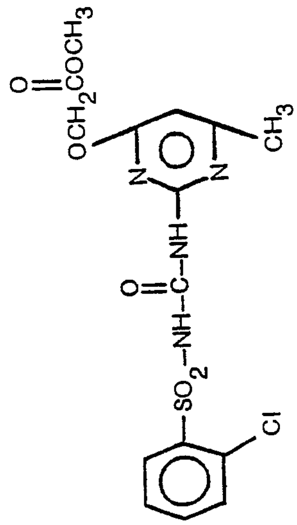 | 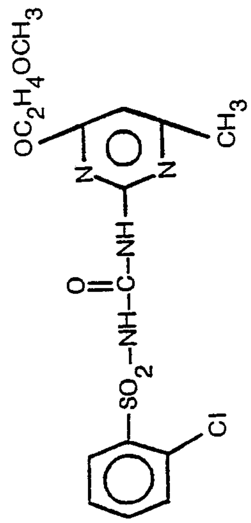 |
|---|---|---|
| Rate, kg/ha | 2 | 2 |
| POST-EMERGENCE | | |
| Bushbean | 8C, 9G | 9C |
| Cotton | – | 2C, 2H, 9G |
| Morningglory | 9C | 2H, 7G |
| Cocklebur | 5C, 8G | 1C, 5G |
| Cassia | 8C | 5C, 9G |
| Nutsedge | 7G | 1C, 8G |
| Crabgrass | 6G | 10C |
| Barnyardgrass | 5C, 9H | 10C |
| Wild Oats | 2C, 8G | 2C, 9G |
| Wheat | 3C, 7G | 2C, 8G |
| Corn | 7U, 9G | 2C, 9G |
| Soybean | 9C | 1C, 9G |
| Rice | 3C, 8G | 5C, 9G |
| Sorghum | 1U, 8G | 2U, 9G |
| PRE-EMERGENCE | | |
| Morningglory | 9H | 9G |
| Cocklebur | 8G | 10E |
| Cassia | 8G | 10E |
| Nutsedge | 9G | 10E |
| Crabgrass | 6G | 10E |
| Barnyardgrass | 9H | 3C, 9H |
| Wild Oats | 2C, 8G | 2C, 9H |
| Wheat | 9H | 9H |
| Corn | 10E | 10E |
| Soybean | 9H | 9H |
| Rice | 10E | 10E |
| Sorghum | 9H | 10C |

TABLE 9 (Continued)

| | 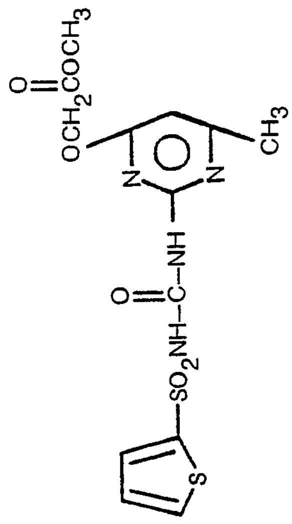 | |
|---|---|---|
| Rate, kg/ha | 2 | 0.4 |
| POST-EMERGENCE | | |
| Bushbean | 4S, 3H, 8G | 3S, 3H, 8G |
| Cotton | 3C, 9G | 2C, 8G |
| Morningglory | 10C | 10C |
| Cocklebur | 8G | 2C, 7G |
| Cassia | 2C, 9G | 3C |
| Nutsedge | 1C, 8G | 8G |
| Crabgrass | 5G | 4G |
| Barnyardgrass | 2C, 9H | 1C, 4H |
| Wild Oats | 4G | 0 |
| Wheat | 6G | 0 |
| Corn | 1C, 9G | 2H, 9G |
| Soybean | 5C, 9G | 9C |
| Rice | 5C, 9G | 3C, 9G |
| Sorghum | 2C, 9G | 1C, 9G |
| PRE-EMERGENCE | | |
| Morningglory | 9G | 7G |
| Cocklebur | 7G | 6G |
| Cassia | 7G | 8G |
| Nutsedge | 10E | 9G |
| Crabgrass | 6G | 3G |
| Barnyardgrass | 2C, 9H | 1C, 8G |
| Wild Oats | 5G | 3G |
| Wheat | 9G | 7G |
| Corn | 10E | 9G |
| Soybean | 9G | 9G |
| Rice | 10E | 10E |
| Sorghum | 9G | 9G |

TABLE 9 (Continued)

| | 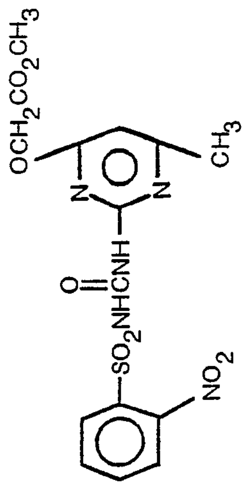 | | 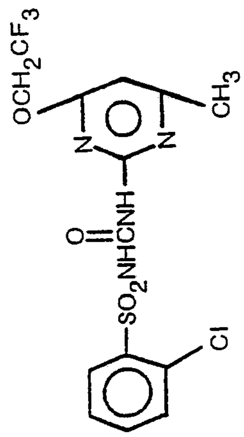 | 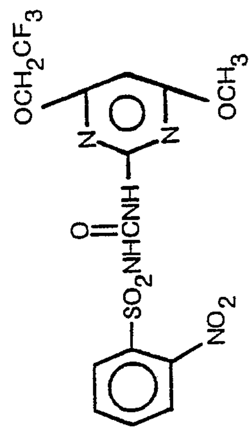 |
|---|---|---|---|---|
| Rate, kg/ha | 2 | 0.4 | 0.4 | 0.4 |
| POST-EMERGENCE | | | | |
| Bushbean | 9C | 9C, 9D | 9C | 9C |
| Cotton | 9C | 9C, 9G | 9C | 6C, 9G |
| Morningglory | 10C | 9C | 10C | 9C |
| Cocklebur | 9C | 10C | 9C | 9C |
| Cassia | 9C | 9C | 9C | 7C |
| Nutsedge | 9C | 9C | 10C | 10C |
| Crabgrass | 9C | 2C, 8G | 9C | 8C |
| Barnyardgrass | 10C | 4C, 9H | 9C | 10C |
| Wild Oats | 9C | 2C, 7G | 5C, 7G | 5C, 5G |
| Wheat | 8C | 2C,7G | 8C | 5C, 5G |
| Corn | 9C | 9C | 10C | 10C |
| Soybean | 10C | 10C | 9C | 2C, 2H |
| Rice | 9C | 9C | 9C | 6C, 9G |
| Sorghum | 9C | 9C | 9C | 9C |
| PRE-EMERGENCE | | | | |
| Morningglory | 10E | 9G | 9C | 9G |
| Cocklebur | 9G | 9G | 9G | 9G |
| Cassia | 9G | 9G | 9G | 5C, 9G |
| Nutsedge | 10E | 10E | 5C, 9G | 10E |
| Crabgrass | 9H | 9G | 7G | 2C, 5G |
| Barnyardgrass | 9H | 9H | 5C, 9H | 5C, 9H |
| Wild Oats | 9G | 9G | 1C, 5G | 2C, 6G |
| Wheat | 9H | 9G | 7G | 9H |
| Corn | 9G | 9G | 9G | 10H |
| Soybean | 9H | 9H | 9H | 9H |
| Rice | 10E | 10E | 10E | 10E |
| Sorghum | 9H | 10H | 4C, 9H | 10H |

TABLE 9 (Continued)

| | 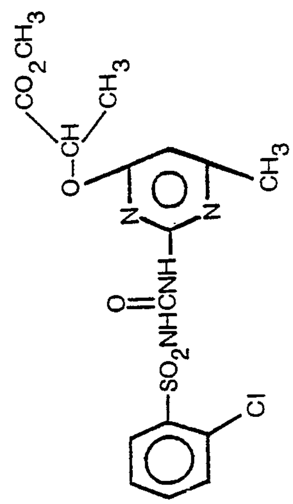 | | 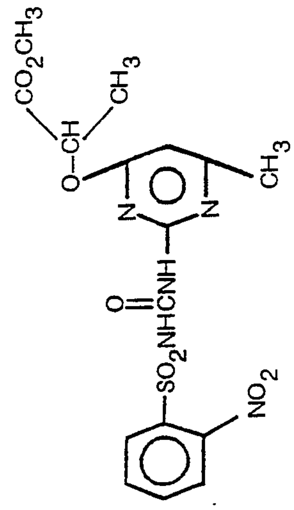 |
|---|---|---|---|
| Rate, kg/ha | 2 | 0.4 | 0.4 |
| POST-EMERGENCE | | | |
| Bushbean | 8C | 8C | 9D, 9G |
| Cotton | 9C | 9C | 9C |
| Morningglory | 10C | 9C | 10C |
| Cocklebur | 10C | 10C | 10C |
| Cassia | 9C | 9C | 9C |
| Nutsedge | 9C | 8C | 9C |
| Crabgrass | 9C | 3C, 8G | 10C |
| Barnyardgrass | 9C | 9C | 10C |
| Wild Oats | 9C | 9C | 9C |
| Wheat | 9C | 9C | 4C, 8G |
| Corn | 9C | 9C | 10C |
| Soybean | 9C | 9C | 5C, 9G |
| Rice | 9C | 9C | 9C |
| Sorghum | 9C | 9C | 10C |
| PRE-EMERGENCE | | | |
| Morningglory | 10E | 10E | 9G |
| Cocklebur | 9G | 9G | 9G |
| Cassia | 9G | 9G | 9G |
| Nutsedge | 10E | 10E | 10E |
| Crabgrass | 9G | 10E | 9E, 9G |
| Barnyardgrass | 10H | 10H | 10H |
| Wild Oats | 9H | 9G | 2C, 9G |
| Wheat | 9H | 9H | 10H |
| Corn | 10E | 10E | 10E |
| Soybean | 9H | 9H | 9H |
| Rice | 10E | 10E | 10E |
| Sorghum | 10E | 9H | 10H |

TABLE 9 (Continued)

| | 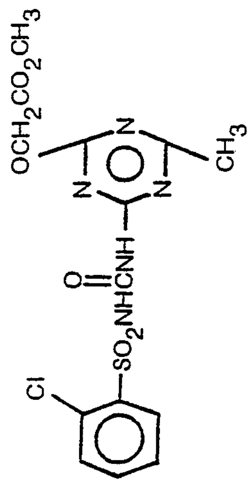 | 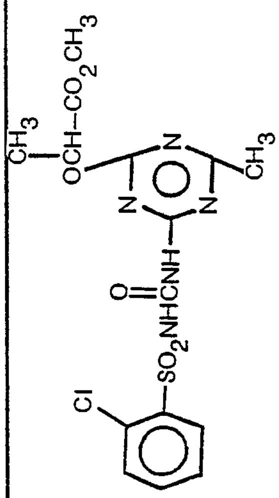 | 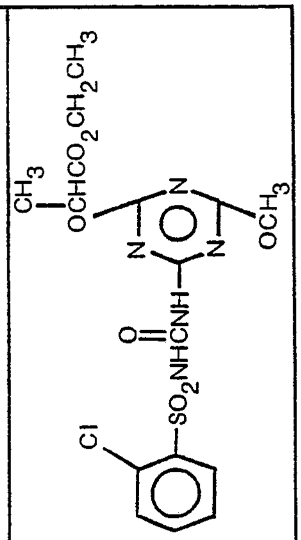 |
|---|---|---|---|
| Rate, kg/ha | 2/5 | 2/5 | 2/5 |
| POST-EMERGENCE | | | |
| Bushbean | 9C | 6C, 9G, 6Y | 9D, 9G, 6C, 1 |
| Cotton | 7C, 9G | 5C, 9G | 6C, 9G |
| Morningglory | 5C, 9G | 6C, 9G | 6C, 9G |
| Cocklebur | 1H | 3C, 8G | 1C |
| Cassia | 6C, 9G | 5C, 8G | 1C, 5G |
| Nutsedge | 2G | 3U | 2G |
| Crabgrass | 3G | 3G | 1C, 2G |
| Barnyardgrass | 1C, 5H | 4C, 8H | 3C, 8H |
| Wild Oats | 0 | 0 | 1C |
| Wheat | 1C | 0 | 1C |
| Corn | 5U, 9G | 3U, 9G | 2U, 9H |
| Soybean | 7C, 9G | 4C, 9G | 9C |
| Rice | 1C, 6G | 7G | - |
| Sorghum | 1C, 7G | 2C, 8G | 1C, 7G |
| PRE-EMERGENCE | | | |
| Morningglory | 9G | 9G | 9H |
| Cocklebur | 2C, 9G | 9G | 9G |
| Cassia | 3C, 9G | 9G | 9G |
| Nutsedge | 4G | 3G | 2G |
| Crabgrass | 0 | 5G | 2G |
| Barnyardgrass | 2C, 7H | 2C, 9H | 4C, 8H |
| Wild Oats | 0 | 0 | 0 |
| Wheat | 0 | 0 | 0 |
| Corn | 1C, 9G | 9G | 2C, 8G |
| Soybean | 1C, 4H | 9H | 9H |
| Rice | 1C, 5G | 5H | 9H |
| Sorghum | 1C, 8G | 1C, 8G | 1C, 8G |

TABLE 9 (Continued)

| | 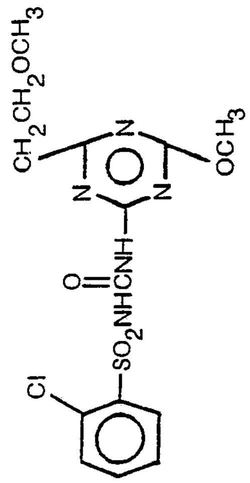 | 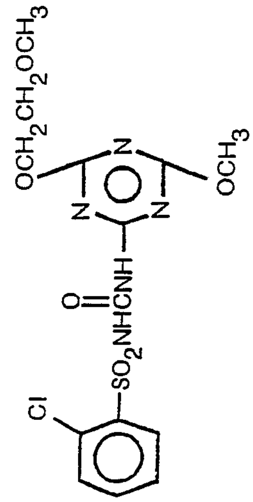 | 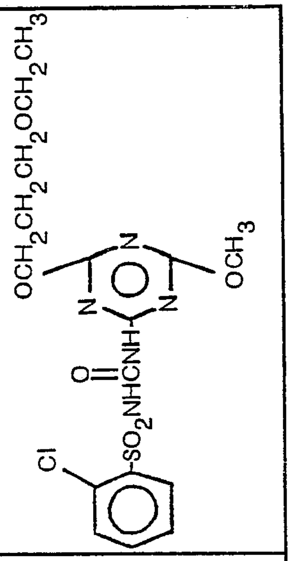 |
|---|---|---|---|
| Rate, kg/ha | 2/5 | 2/5 | 2/5 |
| POST-EMERGENCE | | | |
| Bushbean | 9C | 9C | 5C, 9G, 6Y |
| Cotton | 2H, 5C | 5C, 8G | 9C – |
| Morningglory | 1C, 6G | 10C – | 10C – |
| Cocklebur | 1C, 5G | 5G | 2C, 8G |
| Cassia | 5C, 8G | 9C | 2C, 9G |
| Nutsedge | 1C, 5G | 4G | 2C, 8G |
| Crabgrass | 1C, 8H | 1C, 5G | 2G – |
| Barnyardgrass | 9C | – | 2C, 9H |
| Wild Oats | 1C | 1C, 4G | 0 – |
| Wheat | 1C | 1C, 2G | 0 – |
| Corn | 9C | 9C | 8H – |
| Soybean | 9G | 9C | 5C, 8G |
| Rice | 4C, 7G | – | 4C, 8G |
| Sorghum | 2C, 9G | 2C, 8G | 6G – |
| PRE-EMERGENCE | | | |
| Morningglory | 9G | 9G | 9G |
| Cocklebur | 8G | 8G | 9G |
| Cassia | 8G | 9G | 9G |
| Nutsedge | 5G | 9G | 2G |
| Crabgrass | 1C | 1C, 5G | – |
| Barnyardgrass | 5C, 9H | 1C, 9H | 8H |
| Wild Oats | 1C | 5G | 0 |
| Wheat | 5G | 1C, 8G | 0 |
| Corn | 9G | 9G | 8G |
| Soybean | 9H | 9H | 9H |
| Rice | 5C, 9H | 10E | 8H |
| Sorghum | 2C, 9H | 1C, 9G | 6G |

TABLE 9 (Continued)

| | 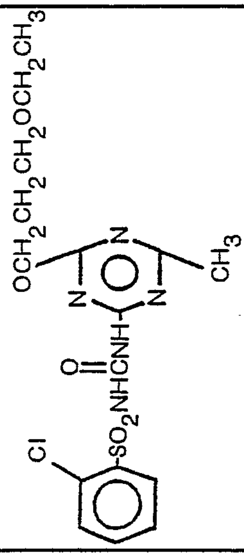 | 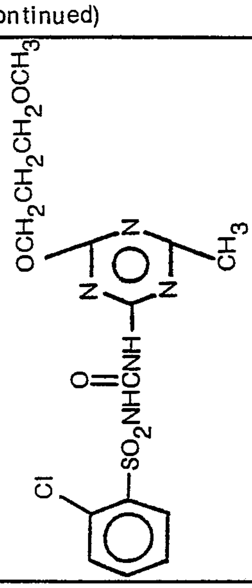 | 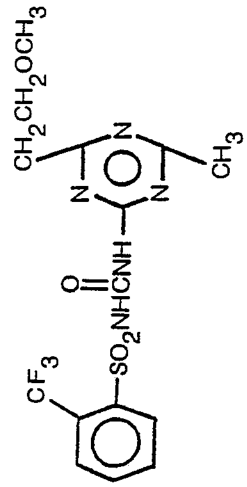 |
|---|---|---|---|
| Rate, kg/ha | 2/5 | 2/5 | 2/5 |
| POST-EMERGENCE | | | |
| Bushbean | 9D, 9G | 3C, 9G, 6Y | 9C |
| Cotton | 6C, 9G | 5C, 9G | 5C, 8G |
| Morningglory | 10C | 9C | 5C, 9G |
| Cocklebur | 3H, 8G | 5C, 9G | 1C, 3H, 6F |
| Cassia | 5C, 8G | 4C, 8G | 5C, 6G |
| Nutsedge | 0 | 2G | 6G |
| Crabgrass | 1C | 2C, 4G | 1C, 6G |
| Barnyardgrass | 3C, 9H | 5C, 8H | 9C |
| Wild Oats | 2C | 2C | 1C, 3G |
| Wheat | 0 | 1C | 4C, 8H |
| Corn | 4C, 9G | 9U, 9C | 10C |
| Soybean | 5C, 9G | 9C | 4C, 8G |
| Rice | – | 6C, 9G | 5C, 8G |
| Sorghum | – | 2C, 8G | 4U, 8G |
| PRE-EMERGENCE | | | |
| Morningglory | 2C, 9G | 9G | 9G |
| Cocklebur | 9C | 9G | 9G |
| Cassia | 5C, 9G | 5C, 9G | 5C, 9G |
| Nutsedge | 0 | 4G | 2C, 9G |
| Crabgrass | 2G | 3G | 2C, 6G |
| Barnyardgrass | 9H | 6H | 5C, 9G |
| Wild Oats | 5G | 7G | 2C, 7G |
| Wheat | 5G | 9H | 1C, 9H |
| Corn | 9G | 5C, 9G | 10H |
| Soybean | 7H | 5H, 9G | 3C, 8H |
| Rice | 9H | 10E | 10E |
| Sorghum | 2C, 8H | 3C, 9G | 2C, 9H |

TABLE 9 (Continued)

| | 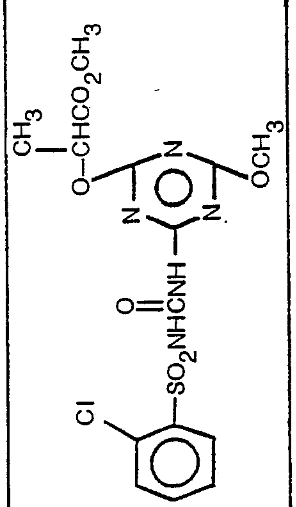 | 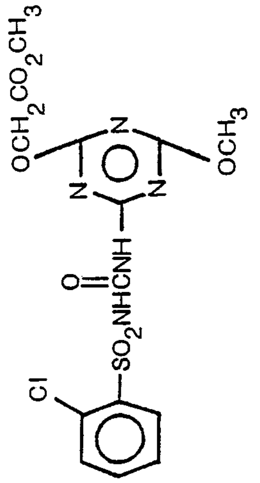 | 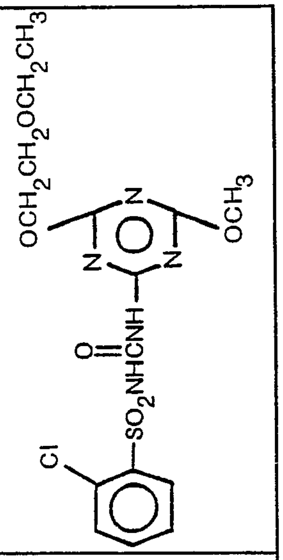 |
|---|---|---|---|
| Rate, kg/ha | 2/5 | 2/5 | 2/5 |
| POST-EMERGENCE | | | |
| Bushbean | 2S, 8G, 6Y | 2C, 6Y | 5C, 9G |
| Cotton | 5C, 8G | 3C, 5G | 5C, 8G |
| Morningglory | 10C | 3C, 8G | 10C |
| Cocklebur | 1C | 0 | 2C, 8G |
| Cassia | – | 3C | 9C |
| Nutsedge | 0 | 2C | 0 |
| Crabgrass | 2G | 2G | 1C, 3G |
| Barnyardgrass | 5G | 2G | 10C |
| Wild Oats | 0 | 0 | 1C, 3G |
| Wheat | 1C | 0 | 1C, 3G |
| Corn | 1C, 5G | 1C | 5U, 8G |
| Soybean | 9C | 5C, 8G | 6C, 8G |
| Rice | – | – | – |
| Sorghum | 4G | 0 | 1C, 8G |
| PRE-EMERGENCE | | | |
| Morningglory | 9G | 7G | 9G |
| Cocklebur | 8G | 3G | 7G |
| Cassia | 9G | 5G | 8G |
| Nutsedge | 0 | 0 | 4G |
| Crabgrass | 2G | 2G | 0 |
| Barnyardgrass | 6G | 0 | 2C, 9H |
| Wild Oats | 0 | 0 | 2G |
| Wheat | 2G | 0 | 2G |
| Corn | 2C, 8G | 3G | 1U, 9G |
| Soybean | 9H | 1C, 1H | 9H |
| Rice | 1C, 4G | 1C | 10E |
| Sorghum | 4G | 1C, 3G | 1C, 9G |

TABLE 9 (Continued)

| | 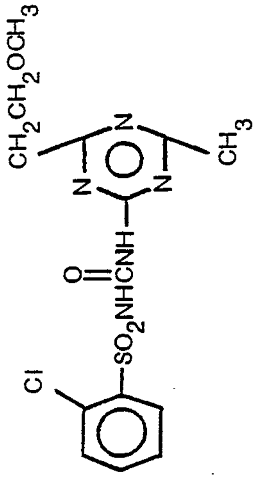 | 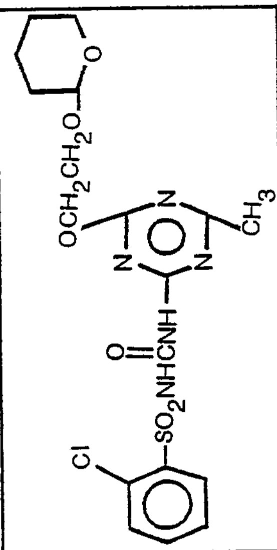 | 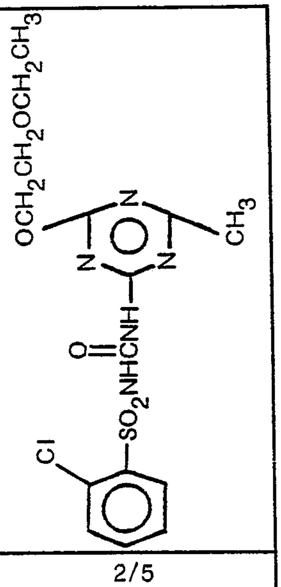 |
|---|---|---|---|
| Rate, kg/ha | 2/5 | 2/5 | 2/5 |
| POST-EMERGENCE | | | |
| Bushbean | 9C | 8C | 8C |
| Cotton | 6C, 9G | 4G, 2H, 5G | 6C, 9G |
| Morningglory | 10C | 2C, 6G | 10C |
| Cocklebur | 1C, 6F | 4G, 6F | 10C |
| Cassia | 6C, 8G | 3C, 4H | 5C, 7G |
| Nutsedge | 1C, 6G | 0 | 5G |
| Crabgrass | 6C, 9G | 1C | 4G |
| Barnyardgrass | 9C | 2C, 7H | 4C, 9H |
| Wild Oats | 6C, 8H | 2C, 4G | 5C, 8H |
| Wheat | 2U, 4C, 7G | 2C, 6G | 9C |
| Corn | 10C | 9C | 10C |
| Soybean | 9G | 2C, 3H | 6C, 9G |
| Rice | 6C, 9G | 5C, 9G | 9C |
| Sorghum | 4U, 8G | 1C, 9G | 2C, 9G |
| PRE-EMERGENCE | | | |
| Morningglory | 9G | 5C | 9H |
| Cocklebur | 9G | 2H | 9G |
| Cassia | 3C, 9G | 2C, 8G | 2C, 9G |
| Nutsedge | 1C, 7G | 0 | 3C, 8G |
| Crabgrass | 3C, 7G | 2G | 3G |
| Barnyardgrass | 5C, 9H | 2C, 8H | 3C, 9H |
| Wild Oats | 2C, 8G | 6G | 2C, 9G |
| Wheat | 1C, 9G | 9H | 9G |
| Corn | 9G | 9G | 9H |
| Soybean | 9H | 1C, 3H | 8H |
| Rice | 10E | 10E | 10E |
| Sorghum | 5C, 9H | 3C, 9H | 5C, 9H |

TABLE 9 (Continued)

| | 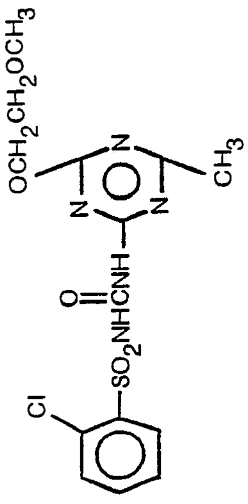 | 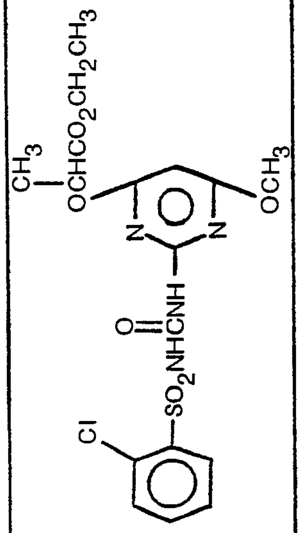 | 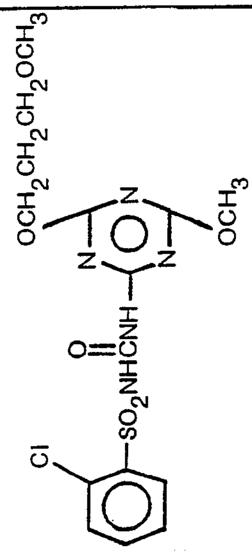 |
|---|---|---|---|
| Rate, kg/ha | 2/5 | 2/5 | 2/5 |
| POST-EMERGENCE | | | |
| Bushbean | 9C | 4C, 9G, 6Y | 5C, 9G, 6Y |
| Cotton | 9C | 4C, 7G | 5C, 8G |
| Morningglory | 10C | 9C | 9C |
| Cocklebur | 10C | 2H | 5G |
| Cassia | 9C | 2C, 8G | 2C, 9G |
| Nutsedge | 5G | 0 | 3G |
| Crabgrass | 3C, 7G | 2G | 2G |
| Barnyardgrass | 9C | 2H | 2C, 7H |
| Wild Oats | 9C | 0 | 1C |
| Wheat | 10C | 0 | 1C |
| Corn | 10C | 1C, 4H | 8H |
| Soybean | 9C | 9C | 9C |
| Rice | 10C | 1C, 5G | 2C, 6G |
| Sorghum | 10C | 2H | 3G |
| PRE-EMERGENCE | | | |
| Morningglory | 9C | 8G | 9G |
| Cocklebur | 9G | 8G | 8G |
| Cassia | 9C | 9G | 9G |
| Nutsedge | 7G | 3G | 3G |
| Crabgrass | 2C, 8G | 3G | 3G |
| Barnyardgrass | 3C, 9H | 3G | 7G |
| Wild Oats | 3C, 9G | 0 | 2G |
| Wheat | 1C, 9G | 0 | 2G |
| Corn | 3C, 9G | 4G | 7G |
| Soybean | 9H | 8H | 9H |
| Rice | 10E | 6G | 9H |
| Sorghum | 10H | 3G | 6G |

TABLE 9 (Continued)

| | 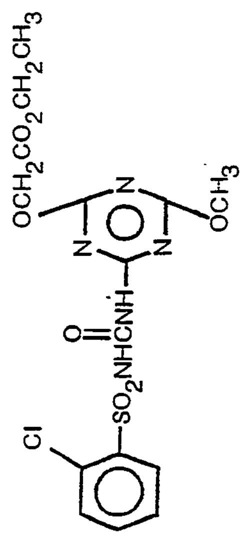 | 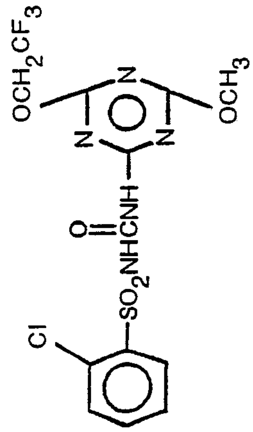 | 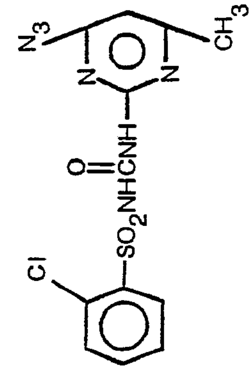 |
|---|---|---|---|
| Rate, kg/ha | 2/5 | 2/5 | 2 |
| POST-EMERGENCE | | | |
| Bushbean | 2C, 8D, 9G | 8C | 2C, 4H |
| Cotton | 3C, 7G | 6C, 9G | 2H |
| Morningglory | 10C | 10C | 5C, 9G |
| Cocklebur | 8G | 6C, 9G | 2C, 8G |
| Cassia | - | 5C, 9G | 1C, 1H |
| Nutsedge | 4G | 2C, 9G | 3G |
| Crabgrass | 1C | 0 | 0 |
| Barnyardgrass | 2C | 10C | 2G |
| Wild Oats | 0 | 1C | 1C |
| Wheat | 0 | 2C, 6G | 1C |
| Corn | 8H | 9C | 7H |
| Soybean | 9C | 9C | 5H |
| Rice | - | - | 2G |
| Sorghum | 3G | 2C, 9G | 6G |
| PRE-EMERGENCE | | | |
| Morningglory | 9C | 9G | 10E |
| Cocklebur | 7G | 8G | 9H |
| Cassia | 8G | 9G | 9G |
| Nutsedge | 7G | 10E | 10E |
| Crabgrass | 0 | 0 | 5G |
| Barnyardgrass | 7H | 2C, 9H | 2C, 8G |
| Wild Oats | 0 | 2G | 1C, 6G |
| Wheat | 2G | 1C, 9G | 6G |
| Corn | 1C, 8G | 2C, 9G | 2C, 9G |
| Soybean | 9H | 9H | 9H |
| Rice | 9H | 10E | 10E |
| Sorghum | 8G | 1C, 9G | 2H, 9G |

TABLE 9 (Continued)

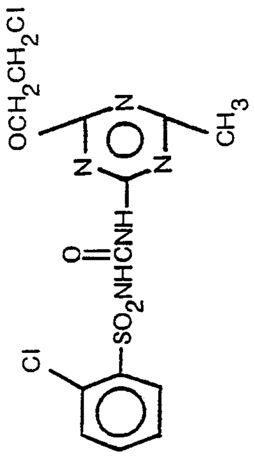

| | |
|---|---|
| Rate, kg/ha | 0.4 |
| POST-EMERGENCE | |
| Bushbean | 9C |
| Cotton | 6C, 9G |
| Morningglory | 10C |
| Cocklebur | 4G |
| Cassia | 2C, 7G |
| Nutsedge | 5G |
| Crabgrass | 2G |
| Barnyardgrass | 1C, 8H |
| Wild Oats | 1C |
| Wheat | 1C |
| Corn | 10C |
| Soybean | 1C, 9G |
| Rice | 1C, 9G |
| Sorghum | 1C, 9G |
| PRE-EMERGENCE | |
| Morningglory | 9G |
| Cocklebur | 8G |
| Cassia | 8G |
| Nutsedge | 0 |
| Crabgrass | 0 |
| Barnyardgrass | 0 |
| Wild Oats | 0 |
| Wheat | 0 |
| Corn | 8H |
| Soybean | 5H |
| Rice | 3G |
| Sorghum | 3G |

TABLE 9 (Continued)

| | 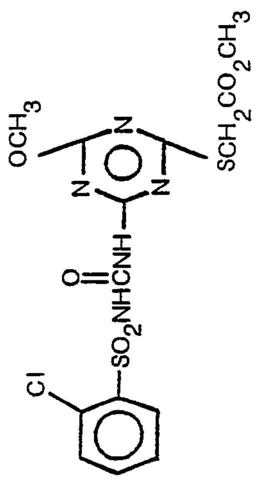 | 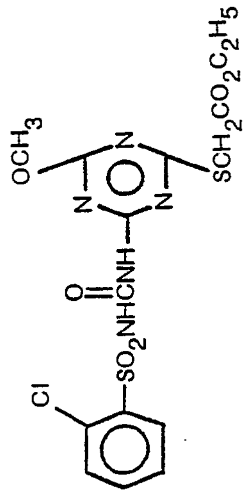 | 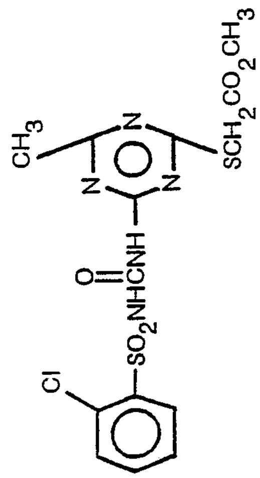 |
|---|---|---|---|
| Rate, kg/ha | 0.4 | 0.4 | 0.4 |
| POST-EMERGENCE | | | |
| Bushbean | 5C, 6Y | 2C | 4C, 8G, 6Y |
| Cotton | 3C, 5G | 2C, 7G | 4C, 9G |
| Morningglory | 4C, 8G | 2C, 5G | 5C, 9G |
| Cocklebur | 2G | 1C | 3G |
| Cassia | - | 5C | 1C, 5G |
| Nutsedge | 2G | 0 | 0 |
| Crabgrass | 2G | 0 | 1C |
| Barnyardgrass | 2G | 1C | 1C, 3G |
| Wild Oats | 0 | 0 | 0 |
| Wheat | 0 | 0 | 0 |
| Corn | 0 | 1C | 6H |
| Soybean | 9C | 2C, 6G | 2C, 8G |
| Rice | - | 5G | 0 |
| Sorghum | 0 | 1C | 1C, 5G |
| PRE-EMERGENCE | | | |
| Morningglory | 8G | 0 | 9G |
| Cocklebur | 2G | 0 | 8G |
| Cassia | 5G | 0 | 1C, 9G |
| Nutsedge | 0 | 0 | 0 |
| Crabgrass | 0 | 0 | 0 |
| Barnyardgrass | 0 | 0 | 1H |
| Wild Oats | 0 | 0 | - |
| Wheat | 0 | 0 | 0 |
| Corn | 1C, 5G | 0 | 1C, 8G |
| Soybean | 2C, 5H | 0 | 1C |
| Rice | 1C | 0 | 1C |
| Sorghum | 1C | 0 | 2G |

Test Procedure B

Two bulb pans were filled with fertilized and limed Fallsington silt loam soil. One pan was planted with seeds of corn, sorghum, Kentucky bluegrass and several grassy weeds. The other pan was planted with seeds of cotton and soybeans, purple nutsedge tubers (*Cyperus rotundus*), and seeds of several broadleaf weeds. Seeds of the following grassy and broadleaf weeds were planted: crabgrass (*Digitaria sanaguinalis*), barnyardgrass (*Echinochloa crusgalli*), wild oats (*Avena* fatua), johnsongrass (*Sorghum halepense*), giant foxtail (*Setaria faberii*), dallisgrass (*Paspalum dilatatum*), cheatgrass (*Bromus secalinu*), mustard (*Brassica arvensis*), cocklebur (*Xanthium pennsylvanicum)*, pigweed (*Amaranthus retroflexus*), morningglory (*Ipomoea hederacea*), cassia (*Cassia tora*), teaweed (*Sida spinosa*), velvetleaf (*Abutilon theophrasti*), and jimsonweed (*Datura stramonium*). A smaller pot was also filled with prepared soil and planted with rice and wheat seeds. Another small pot was planted with seeds of sugarbeets. The above four containers were treated preemergence with nonphytotoxic solvent solutions of the compund of Table 10 (i.e., solutions of compounds were sprayed on the soil surface before seed germination). Duplicates of the above-described seeded containers were prepared without treatment and used as controls.

Twenty-eight days after treatment, the treated and control plants were evaluated and the data recorded as set forth in Table 10. Note that the data indicate that certain compounds of this invention are useful for weed control in crops such as soybeans and wheat.

TABLE 10

| PRE-EMERGENCE ON FALLSINGTON SILT LOAM | | |
|---|---|---|
| | 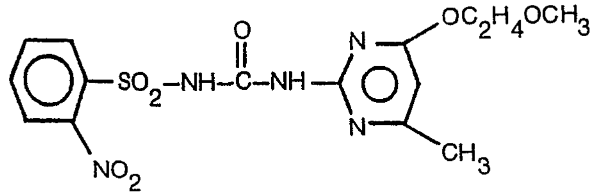 | |
| Rate, kg/ha | 1/16 | 1/4 |
| | | |
| Crabgrass | 10C | 10C |
| Barnyardgrass | 8G, 8C | 10C |
| Sorghum | 9G, 9C | 10C |
| Wild Oats | 4G, 3C | 8G, 5C |
| Johnsongrass | 8G, 5C | 10C |
| Dallisgrass | 8G, 5C | 10C |
| Giant Foxtail | 8G, 6C | 10C |
| Ky. bluegrass | 10C | 10C |
| Cheatgrass | 10C | 10C |
| Sugarbeets | 10C | 10C |
| Corn | 8G, 8H | 10C |
| Mustard | 10C | 10C |
| Cocklebur | 2G | 7G |
| Pigweed | 10E | 10E |
| Nutsedge | 4G | 9G |
| Cotton | 5G, 3H | 7G, 5H |
| Morningglory | 5G | 8G |
| Cassia | 5G | 8G, 9C |
| Teaweed | 5G, 3C | 9G, 8C |
| Velvetleaf | 5G | 9G, 9C |
| Jimsonweed | 5G | 9G, 9C |
| Soybean | 2G | 8G, 5H |
| Rice | 9G, 9C | 10C |
| Wheat | 7G, 4C | 10C |
| | | |
| | | |
| | | |

TABLE 10 (Continued)

| PRE-EMERGENCE ON FALLSINGTON SILT LOAM | | |
|---|---|---|
| | 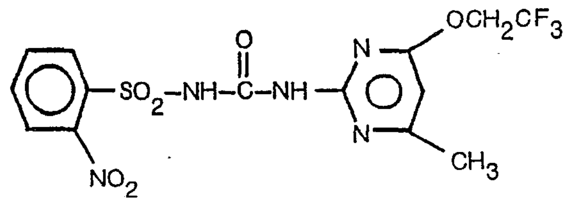 | |
| Rate, kg/ha | 1/16 | 1/4 |
| | | |
| Crabgrass | 5G | 7G |
| Barnyardgrass | 8G, 9C | 10C |
| Sorghum | 7G, 8C | 10C |
| Wild Oats | 4G | 8G, 5C |
| Johnsongrass | 8G, 5C | 9G, 5C |
| Dallisgrass | 7G | 8G, 5C |
| Giant Foxtail | 7G, 4C | 9G, 8C |
| Ky. Bluegrass | 6G, 3C | 8G, 8C |
| Cheatgrass | 8G, 3C | 8G, 3C |
| Sugarbeets | 10C | 10C |
| Corn | 8G, 8H | 9G, 9H |
| Mustard | 10C | 10C |
| Cocklebur | 4G | 8G, 5H |
| Pigweed | 10E | 10E |
| Nutsedge | 10E | 10E |
| Cotton | 7G | 7G |
| Morningglory | 7G | 8G, 3H |
| Cassia | 6G | 8G, 5C |
| Teaweed | 6G, 3C | 8G, 8C |
| Velvetleaf | 6G | 10C |
| Jimsonweed | 6G | 10C |
| Soybean | 7G, 5H | 8G, 5H |
| Rice | 9G, 9C | 10C |
| Wheat | 4G | 7G, 3C |
| | | |
| | | |
| | | |

TABLE 10 (Continued)

| PRE-EMERGENCE ON FALLSINGTON SILT LOAM | | |
|---|---|---|
| | 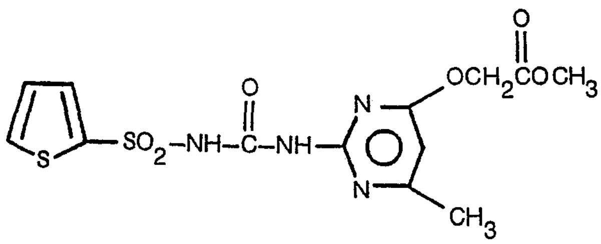 | |
| Rate, kg/ha | 1/16 | 1/4 |
| | | |
| Crabgrass | 0 | 3G |
| Barnyardgrass | 4G | 6G |
| Sorghum | 3G | 7G |
| Wild Oats | 2G | 4G |
| Johnsongrass | 0 | 7G |
| Dallisgrass | 0 | 0 |
| Giant Foxtail | 0 | 4G |
| Ky. Bluegrass | 0 | 5G |
| Cheatgrass | 0 | 5G |
| Sugarbeets | 8G, 5C | 9G, 8C |
| Corn | 3G | 7G |
| Mustard | – | – |
| Cocklebur | 6G | 5G |
| Pigweed | – | – |
| Nutsedge | 7G | 5G |
| Cotton | – | – |
| Morningglory | 5G | 5G |
| Cassia | – | – |
| Teaweed | – | – |
| Velvetleaf | – | – |
| Jimsonweed | 3G | 5G |
| Soybean | 5G | 7G, 5H |
| Rice | 7G | 10C |
| Wheat | 0 | 0 |
| | | |
| | | |
| | | |

TABLE 10 (Continued)

| PRE-EMERGENCE ON FALLSINGTON SILT LOAM | | |
|---|---|---|
| | 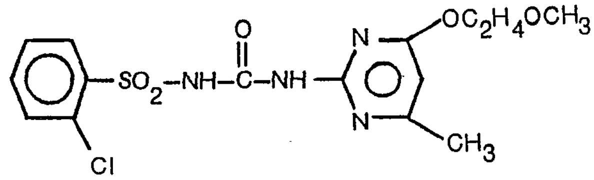 | |
| Rate, kg/ha | 1/16 | 1/4 |
| | | |
| Crabgrass | 0 | 5G |
| Barnyardgrass | 8G, 8H | 10C |
| Sorghum | 8G, 8H | 10C |
| Wild Oats | 5G, 3H | 5G |
| Johnsongrass | 7G, 5H | 10C |
| Dallisgrass | 4G | 10E |
| Giant Foxtail | 6G | 10C |
| Ky. bluegrass | 8G | 10E |
| Cheatgrass | 8C, 5H | 10C |
| Sugarbeets | 4G | 8G, 5H |
| Corn | 7G, 5H | 10C |
| Mustard | 8G | 10C |
| Cocklebur | 0 | 0 |
| Pigweed | 10C | 10C |
| Nutsedge | 0 | 0 |
| Cotton | - | - |
| Morningglory | 0 | 3G |
| Cassia | 0 | 8G |
| Teaweed | 0 | 3G |
| Velvetleaf | - | - |
| Jimsonweed | 0 | 4G |
| Soybean | 0 | 2G |
| Rice | 7G, 5H | 10C |
| Wheat | 5G | 7G |
| | | |
| | | |
| | | |

Test Procedure C

Pots filled with Fallsington silt loam were planted to soybeans, cotton, alfalfa, corn, rice, wheat, sorghum, velvetleaf (*Abutilon theophrasti*), sesbania (*Sesbania exaltata*), cassia (*Cassia tora*), morningglory (*Ipomoea* spp.), jimsonweed (*Datura stramonium*), cocklebur (*Xanthium pennsylvanicum*), crabgrass (*Digitaria* spp.), nutsedge (*Cyperus rotunda*), barnyardgrass (*Echinochloa crusgalli*), giant foxtail (*Setaria faberii*), and wild oats (*Avena fatua*). Approximately 2—1/2 weeks after planting, the young plants and the soil around them were sprayed overall with the compounds of Table II dissolved in a nonphytotoxic solvent. Other groups of all the same weed and crop plants were sprayed with the same nonphytotoxic solvent so as to provide control plants. Fourteen days after treatment, all treated plants were compared with the nonphytotoxic solvent controls and visually rated for response to treatment to give the data presented in Table 11. The data indicate that several of the compounds tested by this procedure are useful for the post-emergence control of weeds in wheat.

TABLE 11

| OVER THE TOP SOIL/FOLIAGE TREATMENT | | |
|---|---|---|
| | 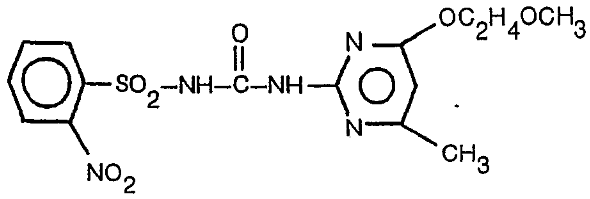 | |
| Rate, kg/ha | 1/16 | 1/4 |
| | | |
| Soybeans | 10C | 10C |
| Velvetleaf | 10G, 7C | 10G, 9C |
| Sesbania | 10C | 10C |
| Cassia | 5G, 3C | 10G, 7C |
| Cotton | 8G, 5C | 10G, 7C |
| Morningglory | 5G, 5C | 10C |
| Alfalfa | 10G, 7C | 10G, 9C |
| Jimsonweed | 5G, 3C | 10C |
| Cocklebur | 3G | 5G, 3C |
| Corn | 10G, 9C | 10G, 9C |
| Crabgrass | 10G, 7C | 10G, 5C |
| Rice | 10G, 4C | 10G, 6C |
| Nutsedge | 4G | 7G |
| Barnyardgrass | 10C | 10G, 6C |
| Wheat | 10G, 4C | 10G, 2C |
| Giant Foxtail | 10G, 3C | 10G, 6C |
| Wild Oats | 10G, 3C | 10G, 6C |
| Sorghum | 10G, 8C | 10G, 6C |

TABLE 11 (Continued)

| OVER THE TOP SOIL/FOLIAGE TREATMENT | | |
|---|---|---|
| | 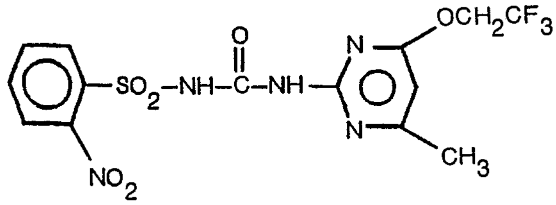 | |
| Rate, kg/ha | 1/16 | 1/4 |
| | | |
| Soybeans | 10C | 10C |
| Velvetleaf | 10C | 10C |
| Sesbania | 10C | 10C |
| Cassia | 10G, 5C | 10G, 6C |
| Cotton | 10G, 3C | 8G, 5C |
| Morningglory | 9G, 3C | 10C |
| Alfalfa | 10G, 9C | 10G, 9C |
| Jimsonweed | 10C | 10C |
| Cocklebur | 10G, 7C | 10G, 7C |
| Corn | 10G, 7C | 10G, 9C |
| Crabgrass | 10G, 6C | 10G, 9C |
| Rice | 10G, 7C | 7G, 4C |
| Nutsedge | 7G | 7G |
| Barnyardgrass | 10C | 10G, 7C |
| Wheat | 6G | 7G, 2C |
| Giant Foxtail | 10G, 5C | 10G, 3C |
| Wild Oats | 10G, 3C | 8G, 2C |
| Sorghum | 10G, 5C | 10G, 9C |

TABLE 11 (Continued)

| OVER THE TOP SOIL/FOLIAGE TREATMENT | | |
|---|---|---|
| | 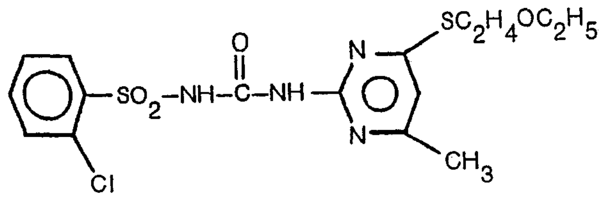 | |
| Rate, kg/ha | 1/16 | 1/4 |
| | | |
| Soybeans | 8G, 2C | 10G, 4C |
| Velvetleaf | 0 | 5G |
| Sesbania | 0 | 5G, 2C |
| Cassia | 3H | 3G, 5H |
| Cotton | 3G | 3G |
| Morningglory | 0 | 6G |
| Alfalfa | 0 | 7G |
| Jimsonweed | 0 | 7G, 4C |
| Cocklebur | 0 | 3G, 2C |
| Corn | 3G, 2H | 8G, 5H |
| Crabgrass | 0 | 3G |
| Rice | 0 | 0 |
| Nutsedge | 0 | 0 |
| Barnyardgrass | 5G | 8G |
| Wheat | 0 | 0 |
| Giant Foxtail | 0 | 3G |
| Wild Oats | 0 | 0 |
| Sorghum | 5G, 3H | 7G, 3H |

TABLE 11 (Continued)

| OVER THE TOP SOIL/FOLIAGE TREATMENT | | |
|---|---|---|
| | 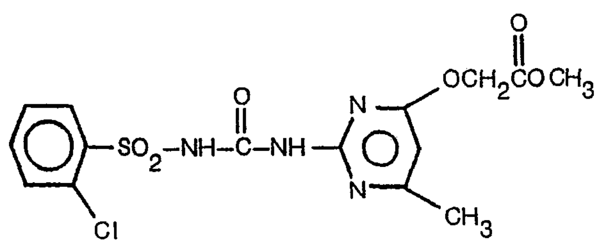 | |
| Rate, kg/ha | 1/16 | 1/4 |
| | | |
| Soybeans | 10G, 6C | 10G, 7C |
| Velvetleaf | 5G, 2C | 10G, 5H |
| Sesbania | 10G, 5C | 10G, 8C |
| Cassia | 10G, 4C | 10G, 6C |
| Cotton | 8G, 2C | 10C |
| Morningglory | 6G | 8G, 2C |
| Alfalfa | 4G | 10G, 4C |
| Jimsonweed | 7G, 2C | 9G, 3H |
| Cocklebur | 3G | 5G |
| Corn | 7G | 8G |
| Crabgrass | 0 | 5G |
| Rice | 10G | 10G, 2C |
| Nutsedge | 0 | 0 |
| Barnyardgrass | 10G | 10G, 4C |
| Wheat | 0 | 7G |
| Giant Foxtail | 5G | 10G, 3C |
| Wild Oats | 0 | 8G, 3C |
| Sorghum | 10G | 10G, 3C |
| | | |
| | | |
| | | |
| | | |
| | | |
| | | |
| | | |
| | | |
| | | |

TABLE 11 (Continued)

| OVER THE TOP SOIL/FOLIAGE TREATMENT | | |
|---|---|---|
| | 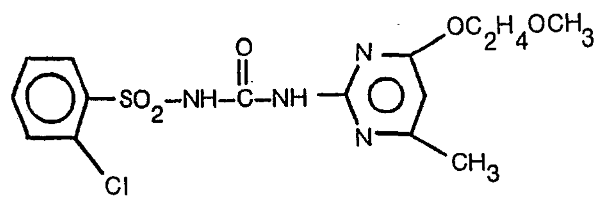 | |
| Rate, kg/ha | 1/16 | 1/4 |
| | | |
| Soybeans | 2G, 3B | 5G, 3B |
| Velvetleaf | 2G | 5G |
| Sesbania | 10G, 2C | 10G, 5C |
| Cassia | 0 | 5G |
| Cotton | 4G | 6G |
| Morningglory | 0 | 0 |
| Alfalfa | 5G | 5G |
| Jimsonweed | 2G | 4G |
| Cocklebur | 0 | 0 |
| Corn | 10G, 3C | 10G, 4C |
| Crabgrass | 3G | 4G |
| Rice | 7G | 5G, 2C |
| Nutsedge | 0 | 3G |
| Barnyardgrass | 8G, 3C | 10G, 4C |
| Wheat | 8G, 3C | 10G, 3C |
| Giant Foxtail | 6G | 10G |
| Wild Oats | 6G | 10G, 3C |
| Sorghum | 8G, 2C | 10G, 3C |

**Claims for the Contracting States: BE CH DE FR GB IT LI LU NL SE**

1. A compound, or an agriculturally suitable salt thereof, which has the formula:

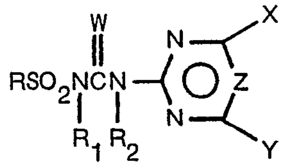 (I)

wherein
R is

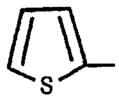 or 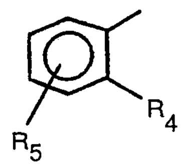

$R_1$ and $R_2$ are independently H or $CH_3$;
$R_4$ is Cl, Br, F, $NO_2$, $CH_3$, $OCH_3$, $CF_3$ or $R_3S(O)_n$, where $R_3$ is $C_1$—$C_6$ alkyl or $C_3$—$C_4$ alkenyl;
$R_5$ is H, Cl, Br, F, $CH_3$ or —$OCH_3$;
n is 0, 1 or 2; W is O or S;
X is $CH_3$ or —$OCH_3$;
Y is $C_2$—$C_4$ alkyl substituted with $CH_3O$; $C_1$—$C_4$ alkyl substituted with $C_2H_5O$; $CH_2F$; $CH_2Cl$; $CH_2Br$; SCN; $N_3$; $A(CH_2)_mA_1R_7$, where m is 2 or 3;
$R_7$ is $C_1$—$C_3$ alkyl; A is O or S; and $A_1$ is O or $S(O)_n$;

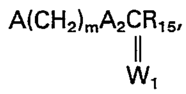

where $A_2$ and $W_1$ are independently O or S, and $R_{15}$ is $C_1$—$C_3$ alkyl, optionally substituted with 1—3 F, Cl or Br, or $R_{15}$ is $N(CH_3)OCH_3$ or $NR_8R_9$;

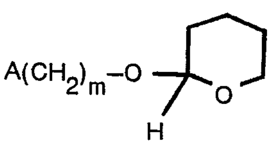 ; 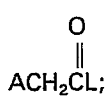; 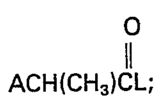; or 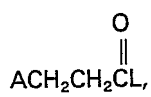,

where L is $N(CH_3)OCH_3$ or $NR_8R_9$, where $R_8$ and $R_9$ are independently H, $CH_3$ or $C_2H_5$, or L is $OR_{10}$ where $R_{10}$ is H or $C_1$—$C_4$alkyl; $NR_{11}R_{12}$ where $R_{11}$ is H or $CH_3$ and $R_{12}$ is $C_3$—$C_4$ alkenyl, $C_2$—$C_3$ alkyl substituted with $OCH_3$ or $OC_2H_5$, cyclopropyl or $C_1$—$C_2$ alkyl substituted with CN, $CO_2CH_3$ or $CO_2C_2H_5$; $OR_{13}$ where $R_{13}$ is $C_4$—$C_6$ alkyl, $C_3$—$C_4$ cyanoalkyl, $C_3$—$C_4$ alkenyl, $C_3$—$C_4$ alkynyl,

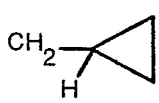 ,

$C_2$ alkyl substituted with an atom of F, Cl or Br, $C_4$ alkyl substituted with 1—3 atoms of F, Cl or Br, or $C_4$ alkyl substituted with 3 atoms of F; and Z is CH or N; provided that when either or both of $R_1$ and $R_2$ is $CH_3$, Y cannot be $OCH_2CH_2OCH_3$.

2. A compound of Claim 1 wherein Y is SCN; $N_3$; $A(CH_2)_mA_1R_7$, where m is 2 or 3, $R_7$ is $C_1$—$C_3$ alkyl and $A_1$ is O or $S(O)_n$;

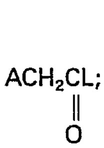; 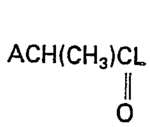 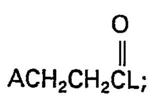;

where A is O or S, and L is $N(CH_3)OCH_3$ or $NR_8R_9$, where $R_8$ and $R_9$ are independently H, $CH_3$ or $C_2H_5$, or L is $OR_{10}$ where $R_{10}$ is $C_1$—$C_4$ alkyl; $NR_{11}R_{12}$ where $R_{11}$ is H or $CH_3$ and $R_{12}$ is $C_3$—$C_4$ alkenyl, $C_2$—$C_3$ alkyl substituted with $OCH_3$ or $OC_2H_5$, or $C_1$—$C_2$ alkyl substituted with CN, $CO_2CH_3$ or $CO_2C_2H_5$; $OR_{13}$ where $R_{13}$ is $C_3$—$C_4$cyanoalkyl, $C_3$—$C_4$ alkenyl, $C_3$—$C_4$ alkynyl,

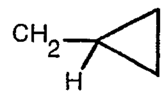 ,

$C_2$ alkyl substituted with an atom of F, Cl or Br, $C_4$ alkyl substituted with 1—3 atoms of F, Cl or Br or $C_2$—$C_4$ alkyl substituted with 3 atoms of F.

3. A compound of Claims 2 wherein $R_1$ and $R_2$ are H and W is O.

4. A compound of Claim 3 wherein Y is $AR_{16}$ where $R_{16}$ is $CH_2CF_3$, $CH_2CH_2OR_{17}$, $CH(CH_3)CO_2R_{17}$, $(CH_2)_3OR_{17}$ or $CH_2CH_2CO_2R_{17}$, where A is O or S and $R_{17}$ is $CH_3$, $—C_2H_5$ or $i$-$C_3H_7$;

5. A compound of Claim 4 wherein A is O and $R_{16}$ is other than $CH_2CH_2CO_2R_{17}$.

6. A compound of claim 5 wherein
R is

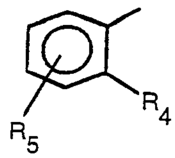

7. A compound of Claim 6 wherein $R_5$ is H or Cl.

8. A compound of Claim 7 wherein $R_4$ is Cl or $NO_2$.

9. A compound of Claim 8 wherein $R_5$ is H.

10. A compound of claim 1 wherein Y is $OCH_2CF_3$, $ACH_2CH_2OR_7'$, $ACH_2CO_2R_7'$, $ACH(CH_3)CO_2R_7'$, $ACH_2CH_2CH_2OR_7'$ or $ACH_2CH_2CO_2R_7'$ where $R_7'$ is $CH_3$ or $CH_2CH_3$ and A is O or S; and $R_4$ is Cl, Br, F, $NO_2$, $CH_3$, $OCH_3$, $CF_3$ or $CH_3S(O)_n$.

11. A compound of claim 1 which is 2-[((6-Methyl-2-)[2-nitrophenylsulfonylamino)carbonylamino]pyrimidin-4-yloxy]acetic acid, methyl ester, and its agriculturally suitable salts.

12. A compound of claim 1 which is 2-chloro-[6-methyl-4-(2,2,2-trifluoroethoxy)-1,3,5-triazin-2-yl]amino-carbonyl]benzenesulfonamide, and its agriculturally suitable salts.

13. A compound of claim 1 which is N-[[4-methoxy-6-(2,2,2-trifluoroethoxy)-1,3,5-triazin-2-yl)aminocarbonyl]-2-nitrobenzenesulfonamide, and its agriculturally suitable salts.

14. A compound of claim 1 which is 2-[(2-[(2-chloro-phenylsulfonylamino)carbonylamino]-6-methylpyrimidin-4-yloxy)]propanoic acid, methyl ester, and its agriculturally suitable salts.

15. A compound of claim 1 which is 2-[(6-methyl-2-[(2-nitrophenyl)sulfonylaminocarbonylamino]-4-pyrimidin-yloxy)]propanoic acid, methyl ester, and its agriculturally suitable salts.

16. A composition suitable for controlling the growth of undesired vegetation which comprises an effective amount of a herbicidal compound and at least one of the following: surfactant, solid or liquid diluent, characterised in that said herbicidal compound is a compound of any of claims 1—15.

17. A method for controlling the growth of undesired vegetation by applying to the locus to be protected an effective amount of a herbicidal compound characterised in that said herbicidal compound is a compound of any of claims 1—15.

18. A method for regulating the growth of plants by applying to the locus of said plants an effective amount of a plant growth regulant, characterised in that said growth regulant is a compound of any of claims 1—15.

19. A process for preparing a compound of claim 1 which comprises:

(a) reacting together compounds of the general formulae

$RSO_2NCW$ and 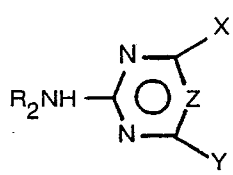

wherein R, $R_2$, W, X, Y and Z are as defined in claim 1;

(b)methylating a compound of general formulae

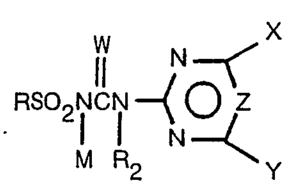

with a compound of general formula $CH_3Q$, wherein R, $R_2$, W, X, Y and Z are as defined in claim 1, M is a metal cation and Q is an anion; or

(c)reacting together compounds of the general formulae

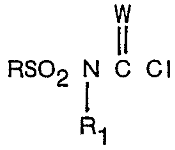 and 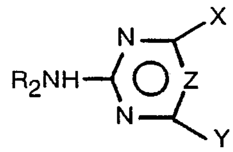

wherein R, $R_2$, W, X, Y and Z are as defined in claim 1 and $R_1$ is methyl.

**Claims for the Contracting State: AT**

1. A process for preparing a compound, or an agriculturally suitable thereof, which has the formula:

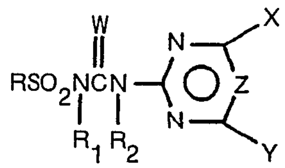 (I)

wherein

R is

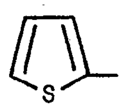 or 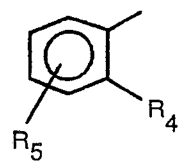

$R_1$ and $R_2$ are independently H or $CH_3$;

$R_4$ is Cl, Br, F, $NO_2$, $CH_3$, $OCH_3$, $CF_3$ or $R_3S(O)_n$, where $R_3$ is $C_1$—$C_6$ alkyl or $C_3$—$C_4$ alkenyl;

$R_5$ is H, Cl, Br, F, $CH_3$ or —$OCH_3$;

n is 0, 1 or 2; W is O or S;

X is $CH_3$ or —$OCH_3$;

Y is $C_2$—$C_4$ alkyl substituted with $CH_3O$; $C_1$—$C_4$ alkyl substituted with $C_2H_5O$; $CH_2F$; $CH_2Cl$; $CH_2Br$; SCN; $N_3$; $A(CH_2)_mA_1R_7$, where m is 2 or 3;

$R_7$ is $C_1$—$C_3$ alkyl; A is O or S; and $A_1$ is O or $S(O)_n$;

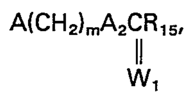

where $A_2$ and $W_1$ are independently O or S, and $R_{15}$ is $C_1$—$C_3$ alkyl, optionally substituted with 1—3 F, Cl or Br, or $R_{15}$ is $N(CH_3)OCH_3$ or $NR_8R_9$;

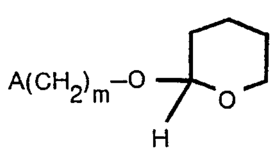 ; 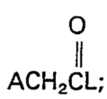 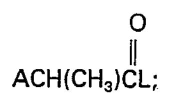 or 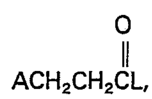

where L is $N(CH_3)OCH_3$ or $NR_8R_9$, where $R_8$ and $R_9$ are independently H, $CH_3$ or $C_2H_5$, or L is $OR_{10}$ where $R_{10}$ is H or $C_1$—$C_4$alkyl; $NR_{11}R_{12}$ where $R_{11}$ is H or $CH_3$ and $R_{12}$ is $C_3$—$C_4$ alkenyl, $C_2$—$C_3$ alkyl substituted with $OCH_3$ or $OC_2H_5$, cyclopropyl or $C_1$—$C_2$ alkyl substituted with CN, $CO_2CH_3$ or $CO_2C_2H_5$; $OR_{13}$ where $R_{13}$ is $C_4$—$C_6$ alkyl, $C_3$—$C_4$ cyanoalkyl, $C_3$—$C_4$ alkenyl, $C_3$—$C_4$ alkynyl,

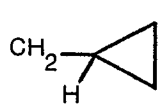 ,

$C_2$ alkyl substituted with an atom of F, Cl or Br, $C_4$ alkyl substituted with 1—3 atoms of F, Cl or Br, or $C_1$—$C_4$ alkyl substituted with 3 atoms of F; and Z is CH or N; provided that when either or both of $R_1$ and $R_2$ is $CH_3$, Y cannot be $OCH_2CH_2OCH_3$; which comprises reacting a compound of general formula

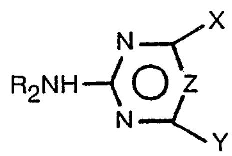

wherein $R_2$, X, Y and Z are as defined above, with a compound of general formula

$RSO_2NCW$ or 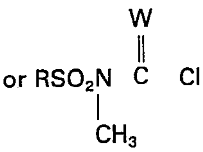

wherein R and W are as defined above, to obtain a compound of general formula (I), whereafter if desired a product wherein $R_1$ is H is converted to a metal derivative of general formula

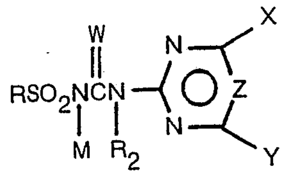

wherein M is a metal cation, and said metal derivative is reacted with a compound of general formula $CH_3Q$, wherein Q is an anion, to obtain a product of general formula (I) wherein $R_1$ is $CH_3$.

2. A process of Claim 1 wherein Y is SCN; $N_3$; $A(CH_2)_mA_1R_7$, where m is 2 or 3, $R_7$ is $C_1$—$C_3$ alkyl and $A_1$ is O or $S(O)_n$;

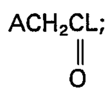; 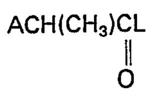 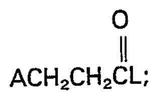;

where A is O or S, and L is $N(CH_3)OCH_3$ or $NR_8R_9$, where $R_8$ and $R_9$ are independently H, $CH_3$ or $C_2H_5$, or L is $OR_{10}$ where $R_{10}$ is $C_1$—$C_4$ alkyl; $NR_{11}R_{12}$ where $R_{11}$ is H or $CH_3$ and $R_{12}$ is $C_3$—$C_4$ alkenyl, $C_2$—$C_3$ alkyl substituted with $OCH_3$ or $OC_2H_5$, or $C_1$—$C_2$ alkyl substituted with CN, $CO_2CH_3$ or $CO_2C_2H_5$; $OR_{13}$ where $R_{13}$ is $C_3$—$C_4$cyanoalkyl, $C_3$—$C_4$ alkenyl, $C_3$—$C_4$ alkynyl,

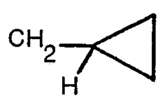,

$C_2$ alkyl substituted with an atom of F, Cl or Br, $C_4$ alkyl substituted with 1—3 atoms of F, Cl or Br or $C_2$—$C_4$ alkyl substituted with 3 atoms of F.

3. A process of Claim 2 wherein $R_1$ and $R_2$ are H and W is O.

4. A process of Claim 3 wherein Y is $AR_{16}$ where $R_{16}$ is $CH_2CF_3$, $CH_2CH_2OR_{17}$, $CH(CH_3)CO_2R_{17}$, $(CH_2)_3OR_{17}$ or $CH_2CH_2CO_2R_{17}$, where A is O or S and $R_{17}$ is $CH_3$, —$C_2H_5$ or *i*-$C_3H_7$;

5. A process of Claim 4 wherein A is O and $R_{16}$ is other than $CH_2CH_2CO_2R_{17}$.

6. A process of Claim 5 wherein
R is

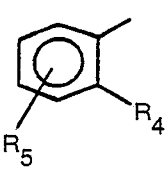;

7. A process of Claim 6 wherein $R_5$ is H or Cl.

8. A process of Claim 7 wherein $R_4$ is Cl or $NO_2$.

9. A process of Claim 8 wherein $R_5$ is H.

10. The process of claim 1 wherein a compound of general formula

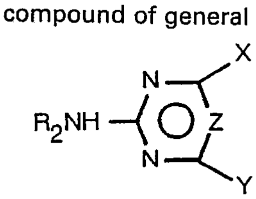

wherein $R_2$, X, Y and Z are as defined in claim 1, is reacted with a compound of general formula

$$RSO_2NCW$$

wherein R and W are as defined in claim 1.

11. The process of Claim 1 wherein the product is

2-[((6-methyl-2-)[2-nitrophenylsulfonylamino)carbonylamino]pyrimidin-4-yloxy]acetic acid, methyl ester, or an agriculturally suitable salt thereof;

2-chloro-[[6-methyl-4-(2,2,2-trifluoroethoxy)-1,3,5-triazin-2-yl]amino-carbonyl]benzenesulfonamide, or an agriculturally suitable salt thereof;

N-[[4-methoxy-6-(2,2,2-trifluoroethoxy)-1,3,5-triazin-2-yl)aminocarbonyl]-2-nitrobenzenesulfonamide, or an agriculturally suitable salt thereof;

2 - [(2 - [(2 - chloro - phenylsulfonylamino)carbonylamino] - 6 - methylpyrimidin - 4 - yloxy)]propanoic acid, methyl ester, or an agriculturally suitable salt thereof; or

2 - [(6 - methyl - 2 - [(2 - nitrophenyl)sulfonylaminocarbonylamino] - 4 - pyrimidinyloxy)]propanoic acid, methyl ester, or an agriculturally suitable salt thereof.

12. A method for controlling the growth of undesired vegetation by applying to the locus of said vegetation an effective amount of a herbicidal compound characterised in that said herbicidal compound is a compound of general formula (I) containing substituent groups as

13. A method for regulating the growth of plants by applying to the locus of said plants an effective amount of a plant growth regulant, characterised in that said plant growth regulant is a compound of general formula (I) containing substituent groups as defined in any of claims 1 to 9 or 11.

**Patentansprüche für den Vertragsstaaten: BE CH DE FR GB IT LI LU NL SE**

1. Eine Verbindung oder ein landwirtschaftlich brauchbares Salz derselben, von der Formel

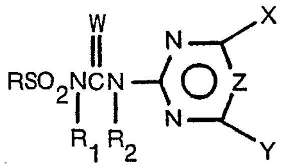

(I)

worin bedeutet

R 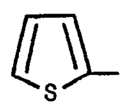 oder 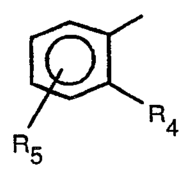

$R_1$ und $R_2$ unabhängig H oder $CH_3$;

$R_4$ Cl, Br, F, $NO_2$, $CH_3$, $OCH_3$, $CF_3$ oder $R_3S(O)_n$, wobei $R_3$ $C_1$—$C_6$-Alkyl oder $C_3$—$C_4$-Alkenyl;

$R_5$ H, Cl, Br, F, $CH_3$ oder —$OCH_3$;

n 0, 1 oder 2; W O oder S;

X $CH_3$ oder —$OCH_3$;

Y mit $CH_3O$ substituiertes $C_2$—$C_4$-Alkyl; mit $C_2H_5O$ substituiertes $C_1$—$C_4$-Alkyl; $CH_2F$; $CH_2Cl$; $CH_2Br$; SCN; $N_3$; $A(CH_2)_mA_1R_7$, wobei m 2 oder 3 ist; $R_7$ $C_1$—$C_3$-Alkyl ist; A O oder S und $A_1$ O oder $S(O)_n$ ist;

$$A(CH_2)_mA_2\underset{\underset{W_1}{\|}}{C}R_{15},$$

wobei $A_2$ und $W_1$ unabhängig O oder S und $R_{15}$ $C_1$—$C_3$-Alkyl sind, gegebenenfalls substituiert mit 1—3 F, Cl oder Br, oder $R_{15}$ $N(CH_3)OCH_3$ oder $NR_8R_9$ ist;

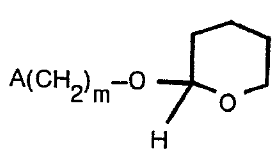 ; $ACH_2\overset{\overset{O}{\|}}{C}L$; $ACH(CH_3)\overset{\overset{O}{\|}}{C}L$ oder $ACH_2CH_2\overset{\overset{O}{\|}}{C}L$,

wobei L $N(CH_3)OCH_3$ oder $NR_8R_9$ ist, wobei $R_8$ und $R_9$ unabhängig H, $CH_3$ oder $C_2H_5$ sind oder L $OR_{10}$ ist, wobei $R_{10}$ H oder $C_1$—$C_4$-Alkyl ist; $NR_{11}R_{12}$, wobei $R_{11}$ H oder $CH_3$ ist und $R_{12}$ $C_3$—$C_4$-Alkenyl ist, mit $OCH_3$

oder $OC_2H_5$ substituiertes $C_2$—$C_3$-Alkyl, Cyclopropyl oder mit CN, $CO_2CH_3$ oder $CO_2C_2H_5$ substituiertes $C_1$—$C_2$-Alkyl; $OR_{13}$, wobei $R_{13}$ $C_4$—$C_6$-Alkyl, $C_3$—$C_4$-Cyanalkyl, $C_3$—$C_4$-Alkenyl, $C_3$—$C_4$-Alkynyl,

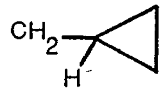 ,

mit einem Atom F, Cl oder Br substituiertes $C_2$-Alkyl, mit 1—3 Atomen F, Cl oder Br substituiertes $C_4$-Alkyl, oder mit 3 Atomen F substituiertes $C_2$—$C_4$-Alkyl ist; und

Z CH oder N;

mit der Massgabe, dass wenn eines von $R_1$ und $R_2$ oder beide $CH_3$ sind, Y nicht $OCH_2CH_2OCH_3$ sein kann.

2. Eine Verbindung von Anspruch 1, worin bedeutet: Y SCN; $N_3$; $A(CH_2)_mA_1R_7$, wobei m 2 oder 3, $R_7$, $C_1$—$C_3$-Alkyl und $A_1$ O oder $S(O)_n$ ist;

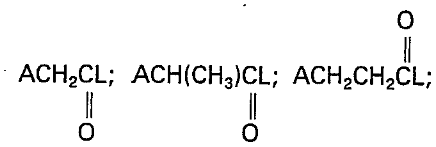

wobei A O oder S und L $N(CH_3)OCH_3$ oder $NR_8R_9$ ist, wobei $R_8$ und $R_9$ unabhängig H, $CH_3$ oder $C_2H_5$ sind, oder L $OR_{10}$ ist, wobei $R_{10}$ $C_1$—$C_4$-Alkyl ist; $NR_{11}R_{12}$, wobei $R_{11}$ H oder $CH_3$ ist und $R_{12}$ $C_3$—$C_4$-Alkenyl, mit $OCH_3$ oder $OC_2H_5$ substituiertes $C_2$—$C_3$-Alkyl, oder mit CN, $CO_2CH_3$ oder $CO_2C_2H_5$ substituiertes $C_1$—$C_2$-Alkyl; $OR_{13}$, wobei $R_{13}$ $C_3$—$C_4$-Cyanoalkyl, $C_3$—$C_4$-Alkenyl, $C_3$—$C_4$-Alkynyl,

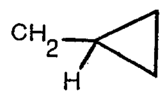 ,

mit einem Atom F, Cl oder Br substituiertes $C_2$-Alkyl, mit 1—3 Atomen F, Cl oder Br substituiertes $C_4$-Alkyl, oder mit 3 Atomen F substituiertes $C_2$—$C_4$-Alkyl ist.

3. Verbindung von Anspruch 2, worin $R_1$ und $R_2$ H sind und W O ist.

4. Verbindung von Anspruch 3, worin Y $AR_{16}$ ist, wobei $R_{16}$ $CH_2CF_3$, $CH_2CH_2OR_{17}$, $CH(CH_3)CO_2R_{17}$, $(CH_2)_3OR_{17}$ oder $CH_2CH_2CO_2R_{17}$ ist, wobei A O oder S und $R_{17}$ $CH_3$, —$C_2H_5$ oder i-$C_3H_7$ ist.

5. Verbindung von Anspruch 4, worin A O und $R_{16}$ nicht $CH_2CH_2CO_2R_{17}$ ist.

6. Verbindung von Anspruch 5, worin

R

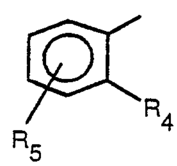

ist.

7. Verbindung von Anspruch 6, worin $R_5$ H oder Cl ist.

8. Verbindung von Anspruch 7, worin $R_4$ Cl oder $NO_2$ ist.

9. Verbindung von Anspruch 8, worin $R_5$ H ist.

10. Verbindung von Anspruch 1, worin Y $OCH_2CF_3$, $ACH_2CH_2OR_7'$, $ACH_2CO_2R_7'$, $ACH(CH_3)CO_2R_7'$, $ACH_2CH_2CH_2OR_7'$ oder $ACH_2CH_2CO_2R_7'$ ist, wobei $R_7'$ $CH_3$ oder $CH_2CH_3$ ist und A O oder S ist; und $R_4$ Cl, Br, F, $NO_2$, $CH_3$, $OCH_3$, $CF_3$ oder $CH_3S(O)_n$ ist.

11. Verbindung von Anspruch 1, nämlich 2-[((6-Methyl-2-)[2-nitrophenylsulfonylamino)carbonyl-amino]pyrimidin-4-yloxy]essigsäure-Methylester, und ihre landwirtschaftlich geeigneten Salze.

12. Verbindung von Anspruch 1, nämlich 2-Chlor-[[6-methyl4-(2,2,2-trifluorethoxy)-1,3,5-triazin-2-yl]-amino-carbonyl]sulfonamid, und ihre landwirtschaftlich geeigneten Salze.

13. Verbindung von Anspruch 1, nämlich N-[(4-Methoxy-6-(2,2,2-trifluorethoxy)-1,3,5-triazin-2-yl)-aminocarbonyl]-2-nitrobenzolsulfonamid, und ihre landwirtschaftlich geeigneten Salze.

14. Verbindung von Anspruch 1, nämlich 2-[(2-[(2-Chlor-phenylsulfonylamino)carbonylamino]-6-methylpyrimidin-4-yloxy)]propionsäure-Methylester, und ihre landwirtschaftlich geeigneten Salze.

15. Verbindung von Anspruch 1, nämlich 2-[(6-Methyl-2-[(2-nitrophenyl)sulfonylaminocarbonyl-amino]-4-pyrimidinyloxy)]propionsäure-Methylester, und ihre landwirtschaftlich geeigneten Salze.

16. Eine Zusammensetzung, geeignet für die Kontrolle des Wachstums unerwünschter Vegatation, enthaltend eine wirksame Menge einer herbiziden Verbindung und mindestens einen der nachfolgenden Zusätze: oberflächenaktives Mittel, festes oder flüssiges Verdünnungsmittel, dadurch gekennzeichnet, dass die herbizide Verbindung eine Verbindung nach einem der Ansprüche 1 bis 15 ist.

17. Verfahren zur Kontrolle des Wachstums unerwünschter Vegetation durch Aufbringung einer wirksamen Menge einer herbiziden Verbindung auf die zu schützende Stelle, dadurch gekennzeichnet, dass die herbizide Verbindung eine Verbindung nach einem der Ansprüche 1 bis 15 ist.

18. Verfahren zur Regulierung des Wachstums von Pflanzen durch Aufbringung einer wirksamen Menge eines Wachstumsregulators auf die zu schützende Stelle der Pflanzen, dadurch gekennzeichnet, dass der Wachstumsregulator eine Verbindung nach einem der Ansprüche 1 bis 15 ist.

19. Verfahren zur Herstellung einer Verbindung von Anspruch 1, wobei man

(a) Verbindungen der allgemeinen Formeln

$RSO_2NCW$ und 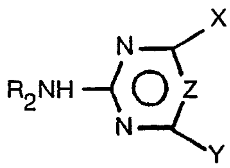

worin R, $R_2$, X, W, Y und Z die Bedeutung wie in Anspruch 1 definiert haben, miteinander zur Umsetzung bringt;

(b) eine Verbindung der allgemeinen Formel

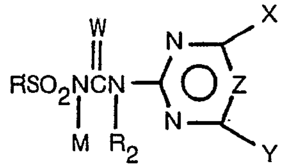

mit einer Verbindung der allgemeinen Formel $CH_3Q$, worin R, $R_2$, W, X, Y und Z wie in Anspruch 1 definiert sind, M ein Metallkation und Q ein Anion bedeutet, methyliert; oder

(c) Verbindungen der allgemeinen Formeln

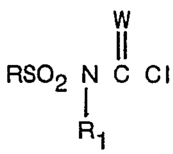 und 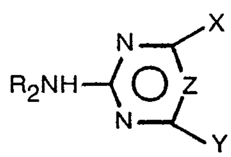

worin R, $R_2$, W, X, Y und Z wie in Anspruch 1 definiert sind und $R_1$ Methyl bedeutet, miteinander zur Umsetzung bringt.

**Patentansprüche für den Vertragsstaat: AT**

1. Verfahren zur Herstellung einer Verbindung oder eines landwirtschaftlich geeigneten Salzes derselben, von der Formel:

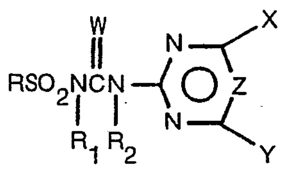 (I)

worin bedeutet

R 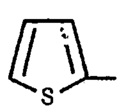 oder 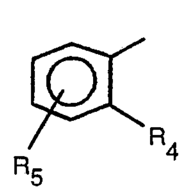

$R_1$ und $R_2$ unabhängig H oder $CH_3$;

$R_4$ Cl, Br, F, $NO_2$, $CH_3$, $OCH_3$, $CF_3$ oder $R_3S(O)_n$, wobei $R_3$ $C_1$—$C_6$-Alkyl oder $C_3$—$C_4$-Alkenyl;

$R_5$ H, Cl, Br, F, $CH_3$ oder $—OCH_3$;

n 0, 1 oder 2; W O oder S;

X $CH_3$ oder $—OCH_3$;

Y mit $CH_3O$ substituiertes $C_2$—$C_4$-Alkyl; mit $C_2H_5O$ substituiertes $C_1$—$C_4$-Alkyl; $CH_2F$; $CH_2Cl$; $CH_2Br$; SCN; $N_3$; $A(CH_2)_mA_1R_7$, wobei m 2 oder 3 ist; $R_7$ $C_1$—$C_3$-Alkyl ist; A O oder S und $A_1$ O oder $S(O)_n$ ist;

$$A(CH_2)_mA_2\underset{\underset{W_1}{\|}}{C}R_{15},$$

wobei $A_2$ und $W_1$ unabhängig O oder S und $R_{15}$ $C_1$—$C_3$-Alkyl sind, gegebenenfalls substituiert mit 1—3 F, Cl oder Br, oder $R_{15}$ $N(CH_3)OCH_3$ oder $NR_8R_9$ ist;

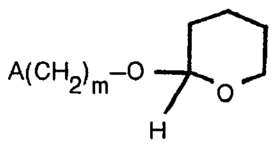 ; $ACH_2\overset{\overset{O}{\|}}{C}L$; $ACH(CH_3)\overset{\overset{O}{\|}}{C}L$ oder $ACH_2CH_2\overset{\overset{O}{\|}}{C}L$,

wobei L $N(CH_3)OCH_3$ oder $NR_8R_9$ ist, wobei $R_8$ und $R_9$ unabhängig H, $CH_3$ oder $C_2H_5$ sind oder L $OR_{10}$ ist, wobei $R_{10}$ H oder $C_1$—$C_4$-Alkyl ist; $NR_{11}R_{12}$, wobei $R_{11}$ H oder $CH_3$ ist und $R_{12}$ $C_3$—$C_4$-Alkenyl ist, mit $OCH_3$ oder $OC_2H_5$ substituiertes $C_2$—$C_3$-Alkyl, Cyclopropyl oder mit CN, $CO_2CH_3$ oder $CO_2C_2H_5$ substituiertes $C_1$—$C_2$-Alkyl; $OR_{13}$, wobei $R_{13}$ $C_4$—$C_6$-Alkyl, $C_3$—$C_4$-Cyanalkyl, $C_3$—$C_4$-Alkenyl, $C_3$—$C_4$-Alkynyl,

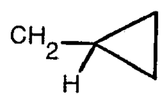 ,

mit einem Atom F, Cl oder Br substituiertes $C_2$-Alkyl, mit 1—3 Atomen F, Cl oder Br substituiertes $C_4$-Alkyl, oder mit 3 Atomen F substituiertes $C_2$—$C_4$-Alkyl ist; und

Z CH oder N;

mit der Massgabe, dass wenn eines von $R_1$ und $R_2$ oder beide $CH_3$ sind, Y nicht $OCH_2CH_2OCH_3$ sein kann, wobei man eine Verbindung der allgemeinen Formel

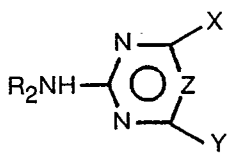

worin $R_2$, X, Y und Z wie oben definiert sind, mit einer Verbindung der allgemeinen Formel

$$RSO_2NCW \text{ or } RSO_2\underset{\underset{CH_3}{|}}{N}\ \overset{\overset{W}{\|}}{C}\ Cl,$$

worin R und W wie oben definiert sind, miteinander zur Umsetzung bringt, um eine Verbindung der allgemeinen Formel (I) zu erhalten, worauf gegebenenfalls ein Produkt, in welchem $R_1$ H ist, umgewandelt wird in ein Metallderivat der allgemeinen Formel

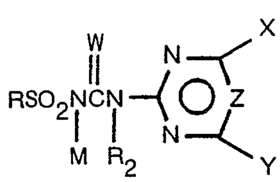

worin M ein Metallkation bedeutet, und dieses Metallderivat mit einer Verbindung der allgemeinen Formel $CH_3Q$ zur Umsetzung bringt, worin Q ein Anion ist, um ein Produkt der allgemeinen Formel I zu erhalten, in der $R_1$ $CH_3$ ist.

2. Verfahren nach Anspruch 1, worin bedeutet: Y SCN; $N_3$; $A(CH_2)_mA_1R_7$, wobei m 2 oder 3, $R_7$ $C_1$—$C_3$-Alkyl und $A_1$ O oder $S(O)_n$ ist;

$$ACH_2\underset{\underset{O}{\|}}{C}L;\quad ACH(CH_3)\underset{\underset{O}{\|}}{C}L;\quad ACH_2CH_2\overset{\overset{O}{\|}}{C}L;$$

wobei A O oder S und L $N(CH_3)OCH_3$ oder $NR_8R_9$ ist, wobei $R_8$ und $R_9$ unabhängig H, $CH_3$ oder $C_2H_5$ sind, oder L $OR_{10}$ ist, wobei $R_{10}$ $C_1$—$C_4$-Alkyl ist; $NR_{11}R_{12}$, wobei $R_{11}$ H oder $CH_3$ ist und $R_{12}$ $C_3$—$C_4$-Alkenyl, mit

$OCH_3$ oder $OC_2H_5$ substituiertes $C_2$—$C_3$-Alkyl, oder mit CN, $CO_2CH_3$ oder $CO_2C_2H_5$ substituiertes $C_1$—$C_2$-Alkyl; $OR_{13}$, wobei $R_{13}$ $C_3$—$C_4$-Cyanoalkyl, $C_3$—$C_4$-Alkenyl, $C_3$—$C_4$-Alkynyl,

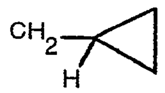,

mit einem Atom F, Cl oder Br substituiertes $C_2$-Alkyl, mit 1—3 Atomen F, Cl oder Br substituiertes $C_4$-Alkyl, oder mit 3 Atomen F substituiertes $C_2$—$C_4$-Alkyl ist.

3. Verfahren nach Anspruch 2, worin $R_1$ und $R_2$ H sind und W O ist.

4. Verfahren nach von Anspruch 3, worin Y $AR_{16}$ ist, wobei $R_{16}$ $CH_2CF_3$, $CH_2CH_2OR_{17}$, $CH(CH_3)CO_2R_{17}$, $(CH_2)_3OR_{17}$ oder $CH_2CH_2CO_2R_{17}$ ist, wobei A O oder S und $R_{17}$ $CH_3$, $—C_2H_5$ oder i-$C_3H_7$ ist.

5. Verfahren nach Anspruch 4, worin A O und $R_{16}$ nicht $CH_2CH_2CO_2R_{17}$ ist.

6. Verfahren nach Anspruch 5, bei dem

R 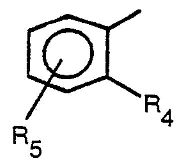

ist.

7. Verfahren nach Anspruch 6, bei dem $R_5$ H oder Cl ist.

8. Verfahren nach Anspruch 7, bei dem $R_4$ Cl oder $NO_2$ ist.

9. Verfahren nach Anspruch 8, bei dem $R_5$ H ist.

10. Verfahren nach Anspruch 1, bei dem eine Verbindung der allgemeinen Formel

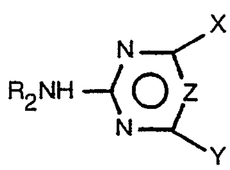

worin $R_2$, X, Y und Z wie in Anspruch 1 definiert sind, mit einer Verbindung der allgemeinen Formel

$RSO_2NCW$

zur Umsetzung gebracht wird, worin R und W wie in Anspruch 1 definiert sind.

11. Verfahren nach Anspruch 1, bei dem das Produkt

2-[((6-Methyl-2-)[2-nitrophenylsulfonylamino)carbonylamino]pyrimidin-4-yloxy]essigsäure-Methylester, oder ein landwirtschaftlich geeignetes Salz davon;

2-Chlor-[[6-methyl-4-(2,2,2-trifluorethoxy)-1,3,5-triazin-2-yl]amino-carbonyl]benzolsulfonamid, oder ein landwirtschaftlich geeignetes Salz davon;

N-[(4-Methoxy-6-(2,2,2-trifluorethoxy)-1,3,5-triazin-2-yl)aminocarbonyl]-2-nitrobenzolsulfonamid, oder ein landwirtschaftlich geeignetes Salz davon;

2-[(2-[(2-Chlor-phenylsulfonylamino)carbonylamino]-6-methylpyrimidin-4-yloxy)]propionsäure-Methylester, oder ein landwirtschaftlich geeignetes Salz davon; oder

2-[(6-Methyl-2-[(2-nitrophenyl)sulfonylaminocarbonylamino]-4-pyrimidinyloxy])propionsäure-Methylester, oder ein landwirtschaftlich geeignetes Salz davon ist.

12. Verfahren zur Kontrolle des Wachstums von unerwünschter Vegetation durch Aufbringung einer wirksamen Menge einer herbiziden Verbindung auf die zu schützende Stelle der Vegetation, dadurch gekennzeichnet, dass die herbizide Verbindung eine Verbindung der allgemeinen Formel (I) ist, welche Substituentengruppen, wie in einem der Ansprüche 1 bis 9 oder 11 definiert, enthält.

13. Verfahren zur Regulierung des Wachstums von Pflanzen durch Aufbringung einer wirksamen Menge eines Wachstumsregulators auf die Pflanzen, dadurch gekennzeichnet, dass der Wachstumsregulator eine Verbindung der allgemeinen Formel (I) ist, die Substituentengruppen wie in einem der Ansprüche 1 bis 9 oder 11 definiert enthält.

**Revendications pour les Etats contractants: BE CH DE FR GB IT LI LU NL SE**

1. Un composé, ou un sel de ce composé convenant pour l'agriculture, qui a la formule:

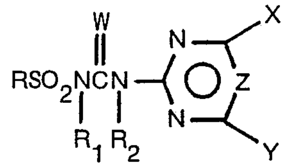

(I)

ou

R est

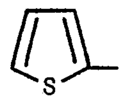

ou

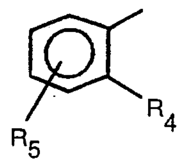

$R_1$ et $R_2$ sont indépendamment H ou $CH_3$;

$R_4$ est Cl, Br, F, $NO_2$, $CH_3$, $OCH_3$, $CF_3$ ou $R_3S(O)_n$, où $R_3$ est un radical alcoyle en $C_1$—$C_6$ ou alcényle en $C_3$—$C_4$;

$R_3$ est H, Cl, Br, F, $CH_3$ ou —$OCH_3$;

n est 0, 1 ou 2; W est O ou S;

X est $CH_3$ ou —$OCH_3$;

Y est un radical alcoyle en $C_2$—$C_4$ substitué par $CH_3O$; un radical alcoyle en $C_1$—$C_4$ substitué par $C_2H_5O$; $CH_2F$; $CH_2Cl$; $CH_2Br$; SCN; $N_3$; $A(CH_2)_mA_1R_7$, où m est 2 ou 3; $R_7$ est un radical alcoyle en $C_1$—$C_3$; A est O ou S; et $A_1$ est O ou $S(O)_n$;

$$A(CH_2)_mA_2\underset{\underset{W_1}{\|}}{C}R_{15},$$

où $A_2$ et $W_1$ sont indépendamment O ou S, et $R_{15}$ est un radical alcoyle en $C_1$—$C_3$, éventuellement substitué par 1—3 atomes de F, Cl ou Br, où $R_{15}$ est $N(CH_3)OCH_3$ ou $NR_8R_9$;

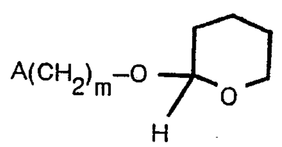 ; $ACH_2\overset{\overset{O}{\|}}{C}L$; $ACH(CH_3)\overset{\overset{O}{\|}}{C}L$; ou $ACH_2CH_2\overset{\overset{O}{\|}}{C}L$,

où L est $N(CH_3)OCH_3$ ou $NR_8R_9$, où $R_8$ et $R_9$ sont indépendamment H, $CH_3$ ou $C_2H_5$, ou L est $OR_{10}$ où $R_{10}$ est H ou un radical alcoyle en $C_1$—$C_4$; $NR_{11}R_{12}$ où $R_{11}$ est H ou $CH_3$ et $R_{12}$ est un radical alcényle en $C_3$—$C_4$, alcoyle en $C_2$—$C_3$ substitué par $OCH_3$ ou $OC_2H_5$, cyclopropyle ou alcoyle en $C_1$—$C_2$ substitué par CN, $CO_2CH_3$ ou $CO_2C_2H_5$; $OR_{13}$ où $R_{13}$ est un radical alcoyle en $C_4$—$C_6$, cyanoalcoyle en $C_3$—$C_4$, alcényle en $C_3$—$C_4$, alcynyle en $C_3$—$C_4$,

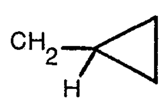 ,

alcoyle en $C_2$ substitué par un atome de F, Cl ou Br, ou alcoyle en $C_4$ substitué par 1—3 atomes de F, Cl ou Br, alcoyle en $C_4$ substitué par 3 atomes de F; et Z est H ou CN; avec la condition que, quand $R_1$ ou $R_2$ ou chacun d'eux est $CH_3$, Y ne peut pas être $OCH_2CH_2OCH_3$.

2. Un composé selon la revendication 1, dans lequel Y est SCN; $N_3$; $A(CH_2)_mA_1R_7$, où m est 2 ou 3, $R_7$ est un radical alcoyle en $C_1$—$C_3$ et $A_1$ est O ou $S(O)_n$;

$$ACH_2\underset{\underset{O}{\|}}{C}L;\ ACH(CH_3)\underset{\underset{O}{\|}}{C}L;\ ACH_2CH_2\overset{\overset{O}{\|}}{C}L;$$

où A est O ou S, et L est $N(CH_3)OCH_3$ ou $NR_8R_9$, où $R_8$ et $R_9$ sont indépendamment H, $CH_3$ ou $C_2H_5$, ou L est

$OR_{10}$ où $R_{10}$ est un radical alcoyle en $C_1$—$C_4$; $NR_{11}R_{12}$ où $R_{11}$ est H ou $CH_3$ et $R_{12}$ est un radical alcényle en $C_3$—$C_4$, alcoyle en $C_2$—$C_3$ substitué par $OCH_3$ ou $OC_2H_5$, ou alcoyle en $C_1$—$C_2$ substitué par CN, $CO_2CH_3$ ou $CO_2C_2H_5$; $OR_{13}$ est un radical cyanoalcoyle en $C_3$—$C_4$, alcényle en $C_3$—$C_4$, alcynyle en $C_3$—$C_4$,

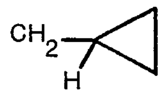 ,

alcoyle en $C_2$ substitué par un atome de F, Cl ou Br, un radical alcoyle en $C_4$ substitué par 1—3 atomes de F, Cl ou Br ou un radical alcoyle en $C_2$—$C_4$ substitué par 3 atomes de F.

3. Un composé selon la revendication 2, dans lequel $R_1$ et $R_2$ sont H et W est O.

4. Un composé selon la revendication 3, dans lequel Y est $AR_{16}$ où $R_{16}$ est $CH_2CF_3$, $CH_2CH_2OR_{17}$, $CH(CH_3)$—$CO_2R_{17}$, $(CH_2)_3\,OR_{17}$ ou $CH_2CH_2CO_2R_{17}$, où A est O ou S et $R_{17}$ est $CH_3$, —$C_2H_5$ ou $i$-$C_3H_7$.

5. Un composé selon la revendication 4, dans lequel A est O et $R_{16}$ est autre que $CH_2CH_2CO_2R_{17}$.

6. Un composé selon la revendication 5, dans lequel R est

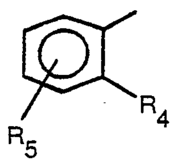

7. Un composé selon la revendication 6, dans lequel $R_5$ est H ou Cl.

8. Un composé selon la revendication 7, dans lequel $R_4$ est Cl ou $NO_2$.

9. Un composé selon la revendication 8, dans lequel $R_5$ est H.

10. Un composé selon la revendication 1, dans lequel Y est $OCH_2CF_3$, $ACH_2CH_2OR_7'$, $ACH_2CO_2R_7'$, $ACH(CH_3)$—$CO_2R_7'$, $ACH_2CH_2CH_2OR_7'$, ou $ACH_2CH_2CO_2R_7'$ où $R_7'$ est $CH_3$ ou $CH_2CH_3$ et A est O ou S; et $R_4$ est Cl, Br, F, $NO_2$, $CH_3$, $OCH_3$, $CF_3$ ou $CH_3S(O)_n$.

11. Un composé selon la revendication 1, qui est l'ester méthylique de l'acide 2-[((6-méthyl-2-)[2-nitrophénylsulfonylamino)carbonylamino]pyrimidin-4-yloxy]acétique, et ses sels convenant pour l'agriculture.

12. Un composé selon la revendication 1, qui est le 2-chloro-[6-méthyl-4-(2,2,2-trifluoroéthoxy)-1,3,5-triazin-2-yl]amino-carbonyl]benzènesulfonamide, et ses sels convenant pour l'agriculture.

13. Un composé selon la revendication 1, qui est le N-[[4-méthoxy-6-(2,2,2-trifluoroéthoxy)-1,3,5-triazin-2-yl)-aminocarbonyl]-2-nitrobenzènesulfonamide, et ses sels convenant pour l'agriculture.

14. Un composé selon la revendication 1, qui est l'ester méthylique de l'acide 2-[(2-[(2-chlorophénylsulfonylamino)carbonylamino]-6-méthylpyrimidin-4-yloxy]propanoïque et ses sels convenant pour l'agriculture.

15. Un composé selon la revendication 1, qui est l'ester méthylique de l'acide 2-[(6-méthyl-2-[(2-nitrophényl)sulfonylaminocarbonylamino]-4-pyrimidin-yloxy]propanoïque, et ses sels convenant pour l'agriculture.

16. Une composition utilisable pour lutter contre la croissance de végétation indésirable, qui comprend une quantité efficace d'un composé herbicide et au moins un des ingrédients suivants: agent tensio-actif, diluant solide ou liquide, caractérisée en ce que ledit composé herbicide est un composé selon l'une quelconque des revendications 1—15.

17. Un procédé de lutte contre la croissance de végétation indésirable en appliquant au lieu à protéger une quantité efficace d'un composé herbicide, caractérisé en ce que ledit composé herbicide est un composé selon l'une quelconque des revendications 1—15.

18. Un procédé pour régler la croissance de plantes en appliquant au lieu de ces plantes une quantité efficace d'un agent de réglage de la croissance de plantes, caractérisé en ce que ledit agent de réglage de la croissance de plantes est un composé selon l'une quelconque des revendications 1—15.

19. Un procédé de préparation d'un composé de la revendication 1, selon lequel:

(a) on fait réagir ensemble des composés des formules générales:

$RSO_2NCW$ et 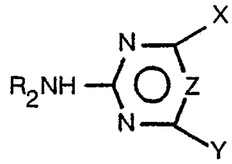

où R, $R_2$, W, X, Y et Z sont tels que définis dans la revendication 1;

(b) on méthyle un composé de formule générale:

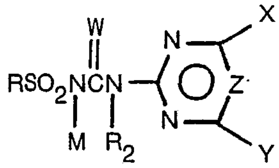

avec un composé de formule générale $CH_3Q$, où R, $R_2$, W, X, Y et Z sont tels que définis dans la revendication 1, M est un cation de métal et Q est un anion; ou

(c) on fait réagir ensemble des composés des formules générales:

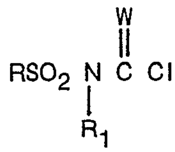 et 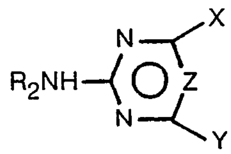

où R, $R_2$, W, X, Y et Z sont tels que définis dans la revendication 1 et $R_1$ est un radical méthyle.

**Revendications pour l'Etat contractant: AT**

1. Un procédé pour la préparation d'un composé, ou d'un de ses sels convenant pour l'agriculture, qui a la formule:

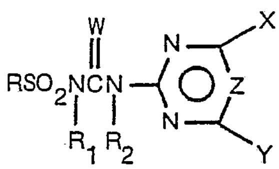 (I)

où

R est

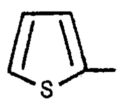 ou 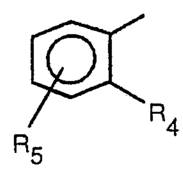

$R_1$ et $R_2$ sont indépendamment H ou $CH_3$;

$R_4$ est Cl, Br, F, $NO_2$, $CH_3$, $OCH_3$, $CF_3$ ou $R_3S(O)_n$, où $R_3$ est un radical alcoyle en $C_1$—$C_6$ ou alcényle en $C_3$—$C_4$;

$R_5$ est H, Cl, Br, F, $CH_3$ ou —$OCH_3$;

n est 0, 1 ou 2; W est O ou S;

X est $CH_3$ ou —$COH_3$;

Y est un radical alcoyle en $C_2$—$C_4$ substitué par $CH_3O$; un radical alcoyle en $C_1$—$C_4$ substitué par $C_2H_5O$; $CH_2F$; $CH_2Cl$; $CH_2Br$; SCN; $N_3$; $A(CH_2)_mA_1R_7$, où m est 2 ou 3; $R_7$ est un radical alcoyle en $C_1$—$C_3$; A est O ou S; et $A_1$ est O ou $S(O)_n$;

$$A(CH_2)_mA_2\underset{\underset{W_1}{\|}}{C}R_{15},$$

où $A_2$ et $W_1$ sont indépendamment O ou S, et $R_{15}$ est un radical alcoyle en $C_1$—$C_3$, éventuellement substitué par 1—3 substituants choisis parmi F, Cl et Br, ou $R_{15}$ est $N(CH_3)OCH_3$ ou $NR_8R_9$,

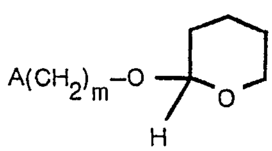 ; $ACH_2\overset{\overset{O}{\|}}{C}L$; $ACH(CH_3)\overset{\overset{O}{\|}}{C}L$; ou $ACH_3CH_3\overset{\overset{O}{\|}}{C}L$

où L est $N(CH_3)OCH_3$ ou $NR_8R_9$, où $R_8$ et $R_9$ sont indépendamment H, $CH_3$ ou $C_2H_5$, ou L est $OR_{10}$ où $R_{10}$ est H ou un radical alcoyle en $C_1$—$C_4$; $NR_{11}R_{12}$ où $R_{11}$ est H ou $CH_3$ et $R_{12}$ est un radical alcényle en $C_3$—$C_4$, alcoyle en $C_2$—$C_3$ substitué par $OCH_3$ ou $OC_2H_5$, cyclopropyle ou alcoyle en $C_1$—$C_2$ substitué par CN, $CO_2CH_3$ ou

$CO_2C_2H_5$; $OR_{13}$ où $R_{13}$ est un radical alcoyle en $C_4$—$C_6$, cyanoalcoyle en $C_3$—$C_4$, alcényle en $C_3$—$C_4$, alcynyle en $C_3$—$C_4$,

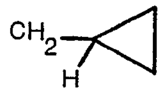

alcoyle en $C_2$ substitué par un atome de F, Cl, ou Br, alcoyle en $C_4$ substitué par 1—3 atomes de F, Cl ou Br ou alcoyle en $C_2$—$C_4$ substitué par 3 atomes de F; et Z est CH ou N; avec la condition que, quand $R_1$ ou $R_2$ ou chacun d'eux est $CH_3$, Y ne peut pas être $OCH_2CH_2OCH_3$; selon lequel on fait réagir un composé de formule générale

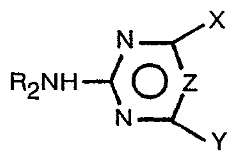

où $R_2$, X, Y et Z sont tels que définis ci-dessus, avec un composé de formule générale

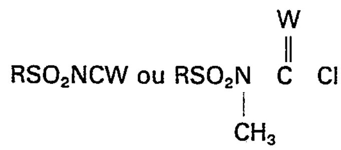

où R et W sont tels que définis ci-dessus, pour obtenir un composé de formule générale (I), après quoi, si on le désire, un produit dans lequel $R_1$ est H est transformé en un dérivé de métal de formule générale

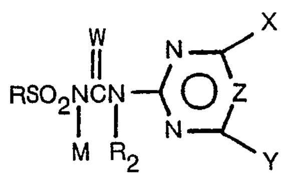

où M est un cation de métal, et ce dérivé de métal est mis à réagir avec un composé de formule générale $CH_3Q$, où Q est un anion, de façon à obtenir un produit de formule générale (I) où $R_1$ est $CH_3$.

2. Un procédé selon la revendication 1, dans lequel Y est SCN; $N_3$; $A(CH_2)_mA_1R_7$, où m est 2 ou 3, R est un radical alcoyle en $C_1$—$C_3$ et $A_1$ est O ou $S(O)_n$;

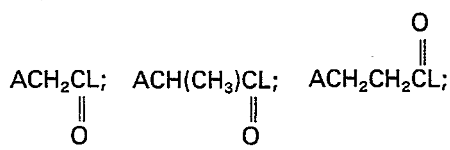

où A est O ou S, et L est $N(CH_3)OCH_3$ ou $NR_8R_9$, où $R_8R_9$, où $R_8$ et $R_9$ sont indépendamment H, $CH_3$ ou $C_2H_5$, ou L est $OR_{10}$ où $R_{10}$ est un radical alcoyle en $C_1$—$C_4$; $NR_{11}R_{12}$ où $R_{11}$ est H ou $CH_3$ et $R_{12}$ est un radical alcényle en $C_3$—$C_4$, alcoyle en $C_2$—$C_3$ substitué par $OCH_3$ ou $OC_2H_5$ ou alcoyle en $C_1$—$C_2$ substitué par CN, $CO_2CH_3$ ou $CO_2C_2H_5$; $OR_{13}$ où $R_{13}$ est un radical cyanoalcoyle en $C_3$—$C_4$, alcényle en $C_3$—$C_4$, alcynyle en $C_3$—$C_4$,

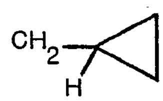

alcoyle en $C_2$ substitué par un atome de F, Cl ou Br, alcoyle en $C_4$ substitué par 1—3 atomes de F, Cl ou Br ou alcoyle en $C_2$—$C_4$ substitué par 3 atomes de F.

3. Un procédé selon la revendication 2, dans lequel $R_1$ et $R_2$ sont H et W est O.

4. Un procédé selon la revendication 3, dans lequel Y est $AR_{16}$ où $R_{16}$ est $CH_2CF_3$, $CH_2CH_2OR_{17}$, $CH(CH_3)CO_2R_{17}$, $(CH_2)_3OR_{17}$ ou $CH_2CH_2CO_2R_{17}$, où A est O ou S et $R_{17}$ est $CH_3$, —$C_2H_5$ ou $i$-$C_3H_7$.

5. Un procédé selon la revendication 4, dans lequel A est O et $R_{16}$ est autre que $CH_2CH_2CO_2R_{17}$.

6. Un procédé selon la revendication 5, dans lequel R est

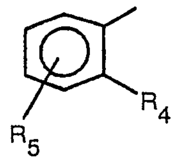

7. Un procédé selon la revendication 6, dans lequel $R_5$ est H ou Cl.
8. Un procédé selon la revendication 7, dans lequel $R_4$ est Cl ou $NO_2$.
9. Un procédé selon la revendication 8, dans lequel $R_5$ est H.
10. Un procédé selon la revendication 1, dans lequel un composé de formule générale

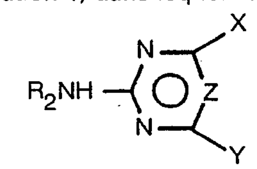

où $R_2$, X, Y et Z sont tels que définis dans la revendication 1, est mis à réagir avec un composé de formule générale

$RSO_2NCW$

où R et W sont tels que définis dans la revendication 1.

11. Un procédé selon la revendication 1, dans lequel le produit est choisi parmi les suivants:
ester méthylique de l'acide 2-[((6-méthyl-2-)[2-nitrophénylsulfonylamino)carbonylamino]pyrimidin-4-yloxy]acétique ou un de ses sels convenant pour l'agriculture;
2-chloro-[[6-méthyl-4-(2,2,2-trifluoroéthoxy)-1,3,5-triazin-2-yl]amino-carbonyl]benzènesulfonamide ou un de ses sels convenant pour l'agriculture;
N-[[4-méthoxy-6-(2,2,2-trifluoroéthoxy)-1,3,5-triazin-2-yl)aminocarbonyl]-2-nitrobenzènesulfonamide ou un de ses sels convenant pour l'agriculture;
ester méthylique de l'acide 2-[(2-[(2-chloro-phénylsulfonylamino)carbonylamino]-6-méthylpyrimidin-4-yloxy)]propanoïque ou un de ses sels convenant pour l'agriculture; et
ester méthylique de l'acide 2-[(6-méthyl-2-[(2-nitrophényl)sulfonylaminocarbonylamino]-4-pyrimidinyloxy)]propanoïque ou un de ses sels convenant pour l'agriculture.

12. Un procédé de lutte contre la croissance de végétation indésirable en appliquant au lieu de cette végétation une quantité efficace d'un composé herbicide, caractérisé en ce que le composé herbicide est un composé de formule générale (I) contenant des groupes substituants tels que définis dans l'une quelconque des revendications 1 à 9 ou 11.

13. Un procédé pour régler la croissance de plantes en appliquant au lieu de ces plantes une quantité efficace d'un agent de réglage de la croissance de plantes, caractérisé en ce que l'agent de réglage de la croissance de plantes est un composé de formule générale (I) contenant des groupes substituants tels que définis dans l'une quelconque des revendications 1 à 9 ou 11.